# EUROPEAN PATENT APPLICATION

(11) **EP 3 795 161 A1**
(43) Date of publication of application: **24.03.2021**
(21) Application number: 19198311.3
(22) Date of filing: 19.09.2019
(51) Int. Cl.: A61K 35/768, A61K 39/395, C07K 14/125, C07K 16/28, C12N 7/00, C12N 7/02, C12N 15/86

(54) **RECOMBINANT ONCOLYTIC NEWCASTLE DISEASE VIRUSES WITH INCREASED ACTIVITY AND IMPROVED VIRAL SAFETY**

(71) Applicant: Thaller, Arno, 91801 Markt Berolzheim (DE)
(72) Inventor: Thaller, Arno, 91801 Markt Berolzheim (DE)
(74) Representative: Becker & Kurig Partnerschaft Patentanwälte PartmbB

(57) **Abstract**

The invention relates to transgene expressing Newcastle Disease Viruses (NDV), which have been demonstrated to possess significant oncolytic activity against mammalian cancers and an improved safety profile. The invention provides novel oncolytic viruses through the use of genetic engineering, including the transfer of foreign genes or parts thereof, such as genes encoding Nivolumab, Ipilimumab, IL-12, NS1, Apoptin, or B18R. The present invention also provides nucleic acids encoding a reverse genetically engineered (rg-)NDV comprising one or more of these foreign genes and having a mutation in the HN gene, said mutation allowing replication of said rgNDV in a cancer cell to a higher level than replication of an otherwise identical rgNDV not having said mutation in the HN gene, as well as at least one mutation in the F gene, said mutation resulting in a reduced ICPI value as compared to an otherwise identical rgNDV not having said at least one mutation in the F gene.

SEQUENCE LISTING

<110> Dr. Arno Thaller
<120> Recombinant oncolytic Newcastle Disease Viruses with increased
activity and improved viral safety

<160> 30

<210> 1
<211> 15186
<212> DNA
<213> Newcastle disease virus

<220>
<223> STRAIN MTH-68/H

<400> 1
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gcccccaccc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgagccctc ctctataaac ccaagattca aggctccaag cgataacctt 3180

ctcccgcctc ttccatccca ctgaatgatc gcgcagctgc aattaatcta gcaacattaa 3240

agattaagaa aaaatacggg tagaatcgga gtgcaccgat tgtgccaaga tggactcatc 3300

taggacaatt gggttatact ttgattctgc ccttccttct agcaacctgt tagcattccc 3360

gattgtctta cgagatgtag gagatggaaa gaagcaaatt actccgcaat ataggatccg 3420

gcgtcttgac tcgtggacag acagtaaaga agactcggta ttcatcacta cctacggatt 3480

catcttccag gttgggaatg aagaagtcac tgtcggcatg atcaatgata atcccaagcg 3540

agagttactc tccgctgcga tgctctgcct aggaagcgtc ccgaatgttg gagatcctgt 3600

tgagctggca agggcctgcc tcactatggt ggtaacatgc aagaagagtg caactaatac 3660

tgagagaatg gtcttctcag tagtgcaggc accccgggtg ctgcaaagct gtagggtcgt 3720

ggcagacaaa tactcgtcag tgaatgcagt taagcacgtg aaagcaccag agaagatccc 3780

tgggagtgaa accctagagt acaaggtgaa ttttgtctct ttgactgtgg tgccgaagaa 3840

ggatgtctac aagatcccaa ccgcagtatt gaaggtatcc ggctcgagcc tgtataatct 3900

tgcgctcaat gtcactattg atgtggaggt agacccaaag agcccgttag tcaagtccct 3960

ttctaggtcc gacagtggat actatgctaa tctcttctta catatcggac ttatgtccac 4020

tgtagataag aggggaaaga aagtgacatt tgaccagctg gagaggaaga taaggagact 4080

tgatctatct gtcgggctca gtgatgtgct cggaccttct gtgcttgtga aggcgagagg 4140

tgcacggact aggctgctgg cacctttctt ctctaacagc gggacagcct gctaccctat 4200

agcaaatgcc tctcctcagg tagctaagat actctggagt cagaccgcgt gcctgcggag 4260

tgtaaaaatc attattcaag cgggcaccca acgcgctgtc gcagtgactg ctgaccatga 4320

ggttacctct actaagatag agaagaggca taccatcgct aaatacaatc ccttcaagaa 4380

ataggccgta tccctgagac tgcaattcac ccgccttccc aaaacaccat gacaccagat 4440

aatgatctgt cttgattact tacagttagt ttccctgtct atcaaattag aaaaaacacg 4500

ggtagaagag ttcggatccc ggccggcgca ccaaaagcgc aagatgggcc ccagatcttc 4560

taccaggatc ccagtacctc taatgctgac catacggatc acgctggcac tgagttatgt 4620

ccgtctgaca agttctcttg atggcaggcc tcttgcagcc gcagggatcg tggtaacagg 4680

ggataaagca gttaacatat acacctcatc ccagacaggg tcaatcatag tcaagttact 4740

cccaaatatg cccaaggaca aagaggcatg tgcaaaagcc ccattggagg cttacaacag 4800

gacactgact actttgctta ccccccttgg tgattctatc cgcaggatac aagagtctgt 4860

gactacatcc ggaggaagga gacagagacg ctttataggt gccattattg gcagtgtagc 4920

tctaggggtt gcaacagctg cacagataac ggcagcctcg gctctgatac aagccaacca 4980

gaatgctgct aacatcctcc ggcttaagga gagcattgct gcaaccaatg aagctgtaca 5040

cgaggtcact ggcggattgt cacagttagc agtggcagtt ggaaagatgc agcaatttgt 5100

taatgaccag tttaataaca cagctcaaga gttggactgt ataaaaatta cacagcaggt 5160

tggtgtagaa ctcaacttat acttaactga attgactaca gtgttcgggc cacaaatcac 5220

ttcccctgcc ttaactcagt tgactgttca ggctctttac aatctggctg gtggtaatgt 5280

agattacttg ttgactaagt taggtgtagg gaacaaccag ctcagctcat tgattggtag 5340

cggcttgatc accggtaacc ctatttttta cgactcacag actcaactct tgggcataca 5400

ggtgacttta ccctcagtcg ggaacctaaa taatatgcgt gccacctact tggagacctt 5460

gtctgtaagc acaacaaagg gatttgcctc agcacttgtc ccaaaagtag tgacacaggt 5520

cggttctgtg atagaagagc ttgacacctc atactgtata gaagctgatt tggatttata 5580

ttgtacaaga atagtgacat tccctatgtc ccctggtatt tattcctgtt tgagcggcaa 5640

tacatcggct tgcatgtatt caaggactga aggcgcactt actacaccat acatgactct 5700

caaaggctca gttgttgcca attgccagat gacaacatgt agatgtgcag acccccgggg 5760

tatcatatca caaaattatg gagaggctgt gtctctaata gataagcact catgcaatgt 5820

cgtatcctta gacgggataa ctttgaggct cagtggggaa tttgatgcaa cttatcaaaa 5880

gaatatctca atattagatt ctcaagtact agtgacaggc aatctcgata tctcaactga 5940

gcttgggaat gtcaaccact caataagtaa tgctttggat aagttagagg aaagcaacag 6000

caaactagac aaagtcaatg tcaaactgac cagcacatct gctctcatta cctatattgt 6060

tttaactgtc atatctcttg ttcttggtat gcttagcctg gttctagcat gctatctgat 6120

gtacaagcaa aaagcgcaac gaaagacctt gttgtggctt ggaaataata ccctagatca 6180

gatgagagct actacaaaaa tgtgaatgca gatgagaggc agaggtatcc ccaatagcaa 6240

tctgtgtgtc aattctggca gcctgttaat cagaagaatt aagaaaaaac taccggatgt 6300

aggtgaacaa aagggaatat acgggtagaa cggcctgaga ggccacccct caatcgggag 6360

ccaggcccca ctacgtccgc tctaccgcat caccaacagc agtcttcagt catggacagc 6420

gcagttagcc aagttgcgct agagaatgat agaagagaag cgaataatac atggcgcttg 6480

gttttccgga tcgcagcctt acttttactg gtaataacct tagccgtctc tgcagtcgcc 6540

ctggcatata gtatggaggc tagcacacct ggcgaccttg taagcatacc gactgcgatc 6600

tctagggcag aggaaaggat tacatctgca ctcggttcca atcaagatgt agtagatagg 6660

atatacaagc aagtggccct tgagtctcca ttggcactgc taaataccga atctataatt 6720

atgaatgcaa taacgtctct ctcttatcaa atcaatggag ccacaaataa tagcgggtgt 6780

ggggcacctg ttcatgaccc agattacatc ggggggatag gtcaagaact tattgtagat 6840

gatacgagtg acgtcacatc attttatccc tctgcattcc aagaacacct gaattttatc 6900

ccggcgccca ctacaggatc aggctgcact cggataccct cattcgacat gagtgctacc 6960

cattactgtt acactcacaa tgtgatattg tctggctgca gagatcactc acactcacat 7020

cagtatttag cacttggtgt gcttcggaca tctgcaacag ggagggtatt cttttctact 7080

ctgcgttcca tcaacttgga tgacgcccaa aatcggaagt cttgcagtgt gagtgcaact 7140

cctttaggtt gtgatatgct gtgctctaaa atcacagaga ctgaggaaga ggattataaa 7200

tcagttatcc ccacatcgat ggtacatgga aggttagggt ttgacggcca ataccatgag 7260

aaggacctag acgtcacaac actatttagg gactgggtgg caaattaccc aggagtagga 7320

ggtgggtctt ttattaacaa tcgcgtatgg ttcccggtct acggagggct aaaacccagc 7380

tcgcctagtg acactgcaca agaaggagga tacgtaatat acaagcgata caatgacaca 7440

tgcccggatg agcaagatta ccagattcgg atggctaagt catcgtataa gcctaggcgg 7500

tttggtggaa aacgcgtaca gcaggccatc ctatccatca aggtgtcaac atccttgggt 7560

gaggacccgg tgttgactgt accgcctaat acggtcgcac tcatgggggc cgaaggcaga 7620

gttctcacag tggggacatc tcatttctta tatcagcgag ggtcatcata cttctctccc 7680

gctttgttat accctatgac agtcaacaac aaaacagcca ctcttcataa tccttataca 7740

ttcaatgctt tcactcggcc aggtagtgtc ccttgccagg cttcagcaag atgccccaac 7800

tcatgtgtta ccggagtcta tactgatcca tatcccttag tcttccatag gaaccacacc 7860

ttgcgagggg tattcggaac aatgcttgat gacgaacaag caagactcaa ccctgtatct 7920

gcagtatttg ataacatatc ccgcagtcgc ataactcggg taagttcaag cagtaccagg 7980

gcagcataca cgacatcaac gtgttttaaa gttgtcaaga ccaataaaac ctattgcctc 8040

agcattgcag aaatatccaa taccctcttc ggggaattca gaattgtccc cttactagtt 8100

gagattctca aggatggtgg ggtttaagaa gctaggaatg gtcggttgag tcaaccgtga 8160

gaaggcgggg aagatgatat tgcatcatct atcttctgta acaccaagaa tcaaatcgaa 8220

taccagtgcg agctcgaatc ctacgctgcc agtcagccat aatcggctgg tgctaatgtg 8280

gttagcctga atcttgtcga tagtcacttg attaagaaaa aatgtggacg gtagtgaaat 8340

ataaggcaaa gcagctcaca gaggacagca cgggtaggac atggcgggct ccggacccga 8400

aagggcagag catcagatta tcctaccaga gtcacatctg tcctcaccat tggtcaagca 8460

taaattgctt tattactgga aattaactgg gctaccgctt cctgacgaat gtgacttcga 8520

ccatcttatt atcagccgac aatggaagaa ggtacttgaa tcggccaccc ctgacattga 8580

gagaatgata aaactagggc gggcagtaca ccagactctc aaccacaatt ccaggataac 8640

cggagtactc catccccggt gtttagagga actggctagt attgaggttc ctgattcaac 8700

caacaaattt cggaagatcg aaaaaaagat ccagattcac aacacaaggt atggagaact 8760

gttcacaaga ttatgcacgc atgtagaaaa gaaactattg gggtcatctt ggtctaacaa 8820

tgtcccacgg tcagaggaat tcaacagcat ccgtacagat ccggcatttt ggtttcactc 8880

aaaatggtcc acagccaaat ttgcatggct ccatataaaa caggtccaga ggcatctgat 8940

tgtagcagca agaacaaggt ccgcagtcaa caaattagtg acgctgaccc ataaggtagg 9000

ccaaatcttt gttactcctg agcttgtcat tgtgacacat acagatgaga acaagttcac 9060

gtgtcttacc caggaacttg tgttgatgta tgcagatatg atggagggca gagatatggt 9120

cagcataata tcatccacgg cggcacatct taggagctta tcagagaaaa ttgatgatat 9180

tctgcggtta gtagatgctc tggcaagaga tttgggcaat caagtctacg atgttgtagc 9240

actaatggag ggattcgcat acggcgctgt tcagctgctt gaaccgtcag gtacatttgc 9300

gggggatttc ttcgcattca acctgcagga gctcaaagat actctaaccg gactcctccc 9360

caaggatatc gcagaatctg tgactcacgc aatcgcaacc atattctctg gcttagaaca 9420

aaatcaagca gctgagatgt tgtgcctgtt gcgtctgtgg ggtcacccac tactcgagtc 9480

ccgtattgca gcaaaagcag ttaggagtca aatgtgcgca ccaaaaatgg tagactttga 9540

tatgatcctc caggtattat ctttctttaa aggaacaatc atcaatggat atagaaagaa 9600

gaatgcaggc gtgtggccac gtgtcaagat agatacgata tacgggaagg tcatagggca 9660

gctacacgca gattctgcag agatttcaca tgatgtcatg ttgagggaat acaagagttt 9720

atctgcactt gaattcgagc catgtataga gtatgaccct gtcaccaatc tgagcatgtt 9780

tttaaaagac aaggcaatcg cacacccgaa agacaactgg ctcgcttcgt ttaggcgaaa 9840

ccttctctct gaggaccaga agaaacatgt aagagaggca acctcaacta accgcctctt 9900

gatagagttc ttagagtcaa atgattttga tccatataaa gagatggaat atctgacgac 9960

ccttgagtac ctaagagatg ataatgtggc agtatcatac tcactcaaag aaaaagaggt 10020

gaaagttaat gggcggattt tcgcaaagct aacaaataga ttaaggaatt gtcaggtaat 10080

ggcagaaggg atcctagctg accagattgc acctttcttc cagggaaatg gggtcattca 10140

ggatagcata tctttgacta aaagtatgct agcgatgagt caactgtctt tcaacagcaa 10200

caagaaacgt attactgact gcaaagaaag agtatcctca aaccgcaatc atgatccgaa 10260

gagcaagaat cgtcggagag ttgccacttt tgtaactacc gacctgcaaa agtattgtct 10320

caattggaga tatcagacag tcaagctgtt cgctcatgcc atcaatcagc tgatgggctt 10380

acctcacttc ttcgagtgga ttcatcttag actaatggat actacgatgt ttgtagggga 10440

ccctttcaat cctccaagtg accctacgga ctgtgatcta tcaagagtcc caaatgatga 10500

catatatatt gtcagtgcta ggggaggcat tgagggatta tgccagaagc tatggacaat 10560

gatctcaatt gctgcaatcc agcttgctgc agcaagatca cattgtcgcg ttgcctgtat 10620

ggtacaaggc gacaatcaag taatagctgt aacgagagag gtaagatcag atgactcccc 10680

agagatggtg ttaacacaat tgcatcaagc cagtgataat ttcttcaagg aattgattca 10740

tgtcaatcat ttgatcggcc ataatttgaa ggatcgtgaa accatcaggt cagacacgtt 10800

cttcatatac agcaaacgaa tattcaaaga tggagcaata ctcagtcagg tcctcaaaaa 10860

ctcatctaaa ttagtgctaa tatcaggcga ccttagtgaa aacactgtaa tgtcttgtgc 10920

caacattgca tctactgtag cacggttatg cgagaacggg cttcctaagg atttctgtta 10980

ttacttaaac tacttaatga gttgcgtgca gacatacttc gattccgaat tttccatcac 11040

caacaactcg caactcgatt ctaaccagtc gtggatagag gacatttctt ttgtgcactc 11100

atatgtcctg acccctgctc aactgggggg actgagtaac cttcaatact caaggctcta 11160

cacaaggaac atcggcgacc cgggaaccac tgctttcgca gagatcaaga gattagaggc 11220

agtggggtta ctgagtccta gcattatgac taacatctta actaggccgc ctggaaatgg 11280

agattgggcc agtctgtgca acgatccata ctcctttaat tttgagactg tcgcaagccc 11340

aaatattgtc cttaagaaac atacacaaag agtcctattt gaaacttgct cgaatccctt 11400

attatctgga gtgcacacag aggataatga ggcagaagag aaggcattgg ctgaattctt 11460

gctcaatcaa gaaatgattc atccacgtgt cgcgcatgct atcatggaag caagctctgt 11520

aggtaggaga aagcaaattc aggggcttgt tgacacaaca aacaccgtga ttaagattgc 11580

actgactagg aggccactcg gcatcaagag gctgatgcgg atagtcaatt actcgagcat 11640

gcatgcaatg ctatttagag atgatgtttt ctcgtctaat agatccaacc atcccttagt 11700

ctcttctgat atgtgttctc tgacactggc agactatgca cggaacagaa gctggtcacc 11760

tttgacaggg ggtagaaaaa tactgggtgt atctaatcct gataccatag aacttgtaga 11820

gggtgagatc cttagtgtca gtggagggtg cactagatgt gacagtgggg atgaacagtt 11880

tacttggttc catcttccaa gcaatataga gctaaacgat gacaccagca agaatcctcc 11940

aatgagagtg ccatatctcg ggtcaaagac tcaagagagg agagccgcct cgcttgcgaa 12000

aatagctcat atgtcaccac acgtgaaggc ggctctaagg gcgtcatctg tgttaatctg 12060

ggcttatggg gacaacgaaa taaactggac tgctgccctt aagattgcaa ggtctcggtg 12120

caacataagc tcagagtatc ttcgactatt gtcacccttg cctacagctg ggaatctcca 12180

acatagattg gacgacggca taactcagat gacattcacc cctgcatctc tatacagggt 12240

gtcaccttac attcatatat ctaatgattc tcaaaggcta tttactgaag aaggagtcaa 12300

agaggggaat gtggtttatc agcaaatcat gctcttgggt ttgtccttaa ttgagtcact 12360

cttcccaatg acaacaacca agacatatga tgaaatcaca ttgcacctcc acagtaaatt 12420

tagctgctgt atcagggaag cacctgttgc agttcctttc gagctactcg gggtggcacc 12480

ggaactaagg gcagtaacct caaataagtt tatgtatgat cctagccctg tatcagaggg 12540

agactttgcg agacttgact tagctatctt taagagttat gaacttaatt tagagtcata 12600

ttccacaata gagctaatga acgttctttc aatatctagt gggaagttga ttggccagtc 12660

tgtggtttct tatgatgaag atacctccat aaagaatgac gctataatag tgtatgacaa 12720

cacacggaat tggatcagcg aagctcagaa ttcagatgtg gtccgcctat tcgagtatgc 12780

ggcactcgaa gtgctcctcg actgttctta tcaactctac tatctgagag taagaggcct 12840

aaacaatatc gtcctgtaca tgagtgattt atacaagaat atgccaggaa ttctactctc 12900

taatattgca gccacaatat ctcaccctgt catccattca aggttgaatg cagtaggtct 12960

ggtcaaccat gacgggtcac accaacttgc agacacagat ttcattgaaa tgtctgcaaa 13020

gctgctagtc tcttgcactc gacgcgtggt ctcaggttta catgcaggga ataagtatga 13080

cctgctgttt ccatctgtct tggatgataa cctaagtgag aagatgcttc agttgatatc 13140

ccaattatgc tgtctgtata cggtgctctt tgctacaaca agagaaatcc cgaaaataag 13200

aggcttatct gcagaagaga aatgttcagt acttactgag tacctactgt cagatgctgt 13260

gaaaccgtta cttgggtccg agcaagtgag ctctatcatg tctcccaaca tagttacgtt 13320

cccagccaat ctgtattaca tgtctaggaa gagccttaat ttgatcaggg agagagagga 13380

cagggatact atcttggcat tgttgttccc tcaagaaccg ctgctcgagt ttcctctggt 13440

acgagatatt ggtgctcgtg taaaagatcc atttacccga caacctgcgg cgtttttaca 13500

agagttagat ttgagtgctc cggcaaggta tgacgcattc acaatcagtc aggcgcattc 13560

tgaacacata ttgccaaacc cagaggaaga tcacttagta cgatacttgt tcagaggaat 13620

agggactgcg tcctcctctt ggtataaggc atcccatctt ctttctgtac ccgaggtcag 13680

atgtgcaagg catgggaact ccttatattt agcagaagga agtggagcca tcatgagtct 13740

tctcgaattg catataccac acgaaactat ctattacaat acacttttct cgaacgagat 13800

gaacccccca cagcgacatt tcggaccaac tccaacacag tttctgaatt cggttgtttt 13860

taggaattta caggcggaag taccatgcaa ggatggattt gtccaggagt tccgtccgtt 13920

atggagagag aatacagaag aaagcgatct gacctcagat aaagcagtgg gatatatcac 13980

atctgtagtg ccgtacaggt ctgtatcatt gctgcattgt gacattgaaa tccctccagg 14040

atctaatcaa agcttactag atcagctggc taccaatctg tctctgattg ccatgcattc 14100

tgtgaaggag ggcggggtcg tgattatcaa agtactgtat gcaatgggat attacttcca 14160

tctactcatg aacttgttta ctccatgttc cacgaaagga tatattctct ctaatggcta 14220

tgcctgtaga ggggatatgg agtgttacct gatatttgtc atgggctacc taggcgggcc 14280

tacatttgta catgaggtgg tgaggatggc aaaaacgcta gtacagcggc atggcacact 14340

tttgtctaag tcagatgaga ttacactgac taggttattt acttcacagc aacagcgtgt 14400

aacagacatc ctatccagcc ctttaccgag actaatgaag tacttgagag agaatattga 14460

tactgcactg attgaagccg ggggacagcc tgtccgtcca ttctgtgcag agagtttagt 14520

gagcacacta acagacatga ctcagacaac ccagatcatc gccagccaca ttgatacagt 14580

cattcgatct gtgatctata tggaagcaga gggtgatctt gctgacacag tattcttatt 14640

taccccttac aacctctcta tggacgggaa aaagagaaca tcacttaaac agtgcacaag 14700

acagatctta gaggtcacaa tactgggtct cagagtcaaa aatctcaata aagtaggtga 14760

tgtaatcagc ctagtactca gaggtatgat ttctctggag gaccttctcc cactgagaac 14820

ctacttgaag tgtagtacct gccctaagta tttgaaggct gtcctaggta ttaccaaact 14880

caaagaaatg ttcacagacg cctctttatt atacttgact cgtgctcaac aaaaattcta 14940

catgaaaact ataggcaatg cagtcaaggg atactatagt aactgtgact cttaaaggca 15000

accacatatc aataggccct ctttctagcc gatcgtattc ttgttgactt cattatacca 15060

tattagaaaa aaattgaatt ccgacccttt aagactcgta ttcggattca aataattatc 15120

tcagaaaaaa agtgcacgta gttgttcttg attatagtcc cgtcattcac caagtctttg 15180

tttggt 15186

<210> 2
<211> 15186
<212> DNA
<213> Newcastle disease virus

<220>
<223> Mut HN
(F277L)/M(G165W)/F(F117S)/F(F190L)/L(V757I)/L(F1551S)/L(R1700L)

<400> 2
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840
cacggcaggt ggaagttcca cttattatag cttggtaggg gacatagact catacatcag 900
gaacaccggg cttactgcat tcttcctgac actcaggtat ggaattaaca ccaagacatc 960
agcccttgca ctcagcagcc tcgcaggcga tatccaaaaa atgaagcagc tcatgcgtct 1020
atatcggatg aaaggagata atgcgccgta catgacactg ctcggtgaca gtgaccagat 1080
gagctttgca ccggctgagt atgcacaact ctactctttt gcaatgggta tggcatccgt 1140
cctagacaaa ggaactggca agtaccagtt cgccagagac tttatgagca catcattctg 1200
gagacttgga gtagagtatg ctcgggctca gggaagcagc atcaatgagg atatggctgc 1260
cgagctaaag ctgaccccag cggcaaggag gggcctggca gctgctgccc aacgagcgtc 1320
cgaggagacc ggcagcatgg acattcccac tcaacaagct ggggtcctca cagggctcag 1380
cgacggaggc cctcaagccc cacaaggtgg actgaacagg tcgcaagggc aaccggacgc 1440
cggagatggg gagacccaat ttttagattt gatgagagca gtggcaaata gtatgagaga 1500
agcgccaaac tctgtgcaga acaccactca gcaggagcct ccatctactc ctgggccatc 1560
tcaagacaac gacaccgact ggggatactg accgacaaca ctcagcctgc ctccatcgaa 1620
ttatctcgat tcttctgcct gtaacctaac ccctggtcca cagtctcaca cggccaaacc 1680
cgcaaacgag cccccccctc tctccccccc cctccagcca cacggctcca accgcccaaa 1740
acaacacggg cacaactcga ctcactcata atctacacag agccgaagat cttagaaaaa 1800
aatacgggta gaagagagac attcagagat caggacaaat catcagggtc tccgctctcc 1860
cctctaccca gcagaccaga gtgaagatgg ccaccttcac ggatgcagag atcgacgatc 1920
tatttgaaac cagtggaact gtcattgaca gcataattac ggcccaaggc aacccagtag 1980
agactgttgg aaggagcgca atcccacaag gcaaaactaa agcattgagt gcagcatggg 2040
agaaacatgg aagcacccaa ccgccggccg gccaagacac ccctgatcga atggacagat 2100
cagacaaaca accatcccca cccgaacagg caaccacaca caacagcccg ccagccacat 2160
ccaccgacca gcccccaccc caggccgcag gcgaagccgg tgacacacag ctcaagactg 2220
gagcaagcaa ctccctcctg tcgatgctcg acaagcttag caataaatcg tccaatgcta 2280
aaaagggccc acggtcgagt ccccaggaag ggcaccacca acctccgacc cagcagcaag 2340
ggagtcaaca gagccgcgga aacagtcagg agagatcgca aaaccaggtc aaggccgcct 2400
ctggagacca gggcacagac gcgaacacag catatcatgg acaatggaag gagtcacaac 2460
catcagctgg tgcaatccct catgctctcc ggtcagggca gagccaagcc aatactcctg 2520
catctgtgga tcatgtccag ctacctgtcg actttgtgca ggcgatgatg tctatgatgg 2580
aggcgatatc acagaaggta aataaagtcg actatcagct agaccttgtc tcaaagcaga 2640
catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgagccctc ctctataaac ccaagattca aggctccaag cgataacctt 3180

ctcccgcctc ttccatccca ctgaatgatc gcgcagctgc aattaatcta gcaacattaa 3240

agattaagaa aaaatacggg tagaatcgga gtgcaccgat tgtgccaaga tggactcatc 3300

taggacaatt gggttatact ttgattctgc ccttccttct agcaacctgt tagcattccc 3360

gattgtctta cgagatgtag gagatggaaa gaagcaaatt actccgcaat ataggatccg 3420

gcgtcttgac tcgtggacag acagtaaaga agactcggta ttcatcacta cctacggatt 3480

catcttccag gttgggaatg aagaagtcac tgtcggcatg atcaatgata atcccaagcg 3540

agagttactc tccgctgcga tgctctgcct aggaagcgtc ccgaatgttg gagatcctgt 3600

tgagctggca agggcctgcc tcactatggt ggtaacatgc aagaagagtg caactaatac 3660

tgagagaatg gtcttctcag tagtgcaggc accccgggtg ctgcaaagct gtagggtcgt 3720

ggcagacaaa tactcgtcag tgaatgcagt taagcacgtg aaagcaccag agaagatccc 3780

ttggagtgaa accctagagt acaaggtgaa ttttgtctct ttgactgtgg tgccgaagaa 3840

ggatgtctac aagatcccaa ccgcagtatt gaaggtatcc ggctcgagcc tgtataatct 3900

tgcgctcaat gtcactattg atgtggaggt agacccaaag agcccgttag tcaagtccct 3960

ttctaggtcc gacagtggat actatgctaa tctcttctta catatcggac ttatgtccac 4020

tgtagataag aggggaaaga aagtgacatt tgaccagctg gagaggaaga taaggagact 4080

tgatctatct gtcgggctca gtgatgtgct cggaccttct gtgcttgtga aggcgagagg 4140

tgcacggact aggctgctgg cacctttctt ctctaacagc gggacagcct gctaccctat 4200

agcaaatgcc tctcctcagg tagctaagat actctggagt cagaccgcgt gcctgcggag 4260

tgtaaaaatc attattcaag cgggcaccca acgcgctgtc gcagtgactg ctgaccatga 4320

ggttacctct actaagatag agaagaggca taccatcgct aaatacaatc ccttcaagaa 4380

ataggccgta tccctgagac tgcaattcac ccgccttccc aaaacaccat gacaccagat 4440

aatgatctgt cttgattact tacagttagt ttccctgtct atcaaattag aaaaaacacg 4500

ggtagaagag ttcggatccc ggccggcgca ccaaaagcgc aagatgggcc ccagatcttc 4560

taccaggatc ccagtacctc taatgctgac catacggatc acgctggcac tgagttatgt 4620

ccgtctgaca agttctcttg atggcaggcc tcttgcagcc gcagggatcg tggtaacagg 4680

ggataaagca gttaacatat acacctcatc ccagacaggg tcaatcatag tcaagttact 4740

cccaaatatg cccaaggaca aagaggcatg tgcaaaagcc ccattggagg cttacaacag 4800

gacactgact actttgctta ccccccttgg tgattctatc cgcaggatac aagagtctgt 4860

gactacatcc ggaggaagga gacagagacg ctctataggt gccattattg gcagtgtagc 4920

tctaggggtt gcaacagctg cacagataac ggcagcctcg gctctgatac aagccaacca 4980

gaatgctgct aacatcctcc ggcttaagga gagcattgct gcaaccaatg aagctgtaca 5040

cgaggtcact ggcggattgt cacagttagc agtggcagtt ggaaagatgc agcaatttgt 5100

taatgaccag cttaataaca cagctcaaga gttggactgt ataaaaatta cacagcaggt 5160

tggtgtagaa ctcaacttat acttaactga attgactaca gtgttcggcc cacaaatcac 5220

ttcccctgcc ttaactcagt tgactgttca ggctctttac aatctggctg gtggtaatgt 5280

agattacttg ttgactaagt taggtgtagg gaacaaccag ctcagctcat tgattggtag 5340

cggcttgatc accggtaacc ctatttttta cgactcacag actcaactct tgggcataca 5400

ggtgacttta ccctcagtcg ggaacctaaa taatatgcgt gccacctact tggagacctt 5460

gtctgtaagc acaacaaagg gatttgcctc agcacttgtc ccaaaagtag tgacacaggt 5520

cggttctgtg atagaagagc ttgacacctc atactgtata gaagctgatt tggatttata 5580

ttgtacaaga atagtgacat tccctatgtc ccctggtatt tattcctgtt tgagcggcaa 5640

tacatcggct tgcatgtatt caaggactga aggcgcactt actacaccat acatgactct 5700

caaaggctca gttgttgcca attgccagat gacaacatgt agatgtgcag acccccgggg 5760

tatcatatca caaaattatg gagaggctgt gtctctaata gataagcact catgcaatgt 5820

cgtatcctta gacgggataa ctttgaggct cagtggggaa tttgatgcaa cttatcaaaa 5880

gaatatctca atattagatt ctcaagtact agtgacaggc aatctcgata tctcaactga 5940

gcttgggaat gtcaaccact caataagtaa tgctttggat aagttagagg aaagcaacag 6000

caaactagac aaagtcaatg tcaaactgac cagcacatct gctctcatta cctatattgt 6060

tttaactgtc atatctcttg ttcttggtat gcttagcctg gttctagcat gctatctgat 6120

gtacaagcaa aaagcgcaac gaaagacctt gttgtggctt ggaaataata ccctagatca 6180

gatgagagct actacaaaaa tgtgaatgca gatgagaggc agaggtatcc ccaatagcaa 6240

tctgtgtgtc aattctggca gcctgttaat cagaagaatt aagaaaaaac taccggatgt 6300

aggtgaacaa aagggaatat acgggtagaa cggcctgaga ggccacccct caatcgggag 6360

ccaggcccca ctacgtccgc tctaccgcat caccaacagc agtcttcagt catggacagc 6420

gcagttagcc aagttgcgct agagaatgat agaagagaag cgaataatac atggcgcttg 6480

gttttccgga tcgcagcctt acttttactg gtaataacct tagccgtctc tgcagtcgcc 6540

ctggcatata gtatggaggc tagcacacct ggcgaccttg taagcatacc gactgcgatc 6600

tctagggcag aggaaaggat tacatctgca ctcggttcca atcaagatgt agtagatagg 6660

atatacaagc aagtggccct tgagtctcca ttggcactgc taaataccga atctataatt 6720

atgaatgcaa taacgtctct ctcttatcaa atcaatggag ccacaaataa tagcgggtgt 6780

ggggcacctg ttcatgaccc agattacatc ggggggatag gtcaagaact tattgtagat 6840

gatacgagtg acgtcacatc attttatccc tctgcattcc aagaacacct gaattttatc 6900

ccggcgccca ctacaggatc aggctgcact cggataccct cattcgacat gagtgctacc 6960

cattactgtt acactcacaa tgtgatattg tctggctgca gagatcactc acactcacat 7020

cagtatttag cacttggtgt gcttcggaca tctgcaacag ggagggtatt cttttctact 7080

ctgcgttcca tcaacttgga tgacgcccaa aatcggaagt cttgcagtgt gagtgcaact 7140

cctttaggtt gtgatatgct gtgctctaaa atcacagaga ctgaggaaga ggattataaa 7200

tcagttatcc ccacatcgat ggtacatgga aggttagggc ttgacggcca ataccatgag 7260

aaggacctag acgtcacaac actatttagg gactgggtgg caaattaccc aggagtagga 7320

ggtgggtctt ttattaacaa tcgcgtatgg ttcccggtct acggagggct aaaacccagc 7380

tcgcctagtg acactgcaca agaagggaga tacgtaatat acaagcgata caatgacaca 7440

tgcccggatg agcaagatta ccagattcgg atggctaagt catcgtataa gcctaggcgg 7500

tttggtggaa aacgcgtaca gcaggccatc ctatccatca aggtgtcaac atccttgggt 7560

gaggacccgg tgttgactgt accgcctaat acggtcgcac tcatgggggc cgaaggcaga 7620

gttctcacag tggggacatc tcatttctta tatcagcgag ggtcatcata cttctctccc 7680

gctttgttat accctatgac agtcaacaac aaaacagcca ctcttcataa tccttataca 7740

ttcaatgctt tcactcggcc aggtagtgtc ccttgccagg cttcagcaag atgccccaac 7800

tcatgtgtta ccggagtcta tactgatcca tatcccttag tcttccatag gaaccacacc 7860

ttgcgagggg tattcggaac aatgcttgat gacgaacaag caagactcaa ccctgtatct 7920

gcagtatttg ataacatatc ccgcagtcgc ataactcggg taagttcaag cagtaccagg 7980

gcagcataca cgacatcaac gtgttttaaa gttgtcaaga ccaataaaac ctattgcctc 8040

agcattgcag aaatatccaa taccctcttc ggggaattca gaattgtccc cttactagtt 8100

gagattctca aggatggtgg ggtttaagaa gctaggaatg gtcggttgag tcaaccgtga 8160

gaaggcgggg aagatgatat tgcatcatct atcttctgta acaccaagaa tcaaatcgaa 8220

taccagtgcg agctcgaatc ctacgctgcc agtcagccat aatcggctgg tgctaatgtg 8280

gttagcctga atcttgtcga tagtcacttg attaagaaaa aatgtggacg gtagtgaaat 8340

ataaggcaaa gcagctcaca gaggacagca cgggtaggac atggcgggct ccggacccga 8400

aagggcagag catcagatta tcctaccaga gtcacatctg tcctcaccat tggtcaagca 8460

taaattgctt tattactgga aattaactgg gctaccgctt cctgacgaat gtgacttcga 8520

ccatcttatt atcagccgac aatggaagaa ggtacttgaa tcggccaccc ctgacattga 8580

gagaatgata aaactagggc gggcagtaca ccagactctc aaccacaatt ccaggataac 8640

cggagtactc catccccggt gtttagagga actggctagt attgaggttc ctgattcaac 8700

caacaaattt cggaagatcg aaaaaaagat ccagattcac aacacaaggt atggagaact 8760

gttcacaaga ttatgcacgc atgtagaaaa gaaactattg gggtcatctt ggtctaacaa 8820

tgtcccacgg tcagaggaat tcaacagcat ccgtacagat ccggcatttt ggtttcactc 8880

aaaatggtcc acagccaaat ttgcatggct ccatataaaa caggtccaga ggcatctgat 8940

tgtagcagca agaacaaggt ccgcagtcaa caaattagtg acgctgaccc ataaggtagg 9000

ccaaatcttt gttactcctg agcttgtcat tgtgacacat acagatgaga acaagttcac 9060

gtgtcttacc caggaacttg tgttgatgta tgcagatatg atggagggca gagatatggt 9120

cagcataata tcatccacgg cggcacatct taggagctta tcagagaaaa ttgatgatat 9180

tctgcggtta gtagatgctc tggcaagaga tttgggcaat caagtctacg atgttgtagc 9240

actaatggag ggattcgcat acggcgctgt tcagctgctt gaaccgtcag gtacatttgc 9300

gggggatttc ttcgcattca acctgcagga gctcaaagat actctaaccg gactcctccc 9360

caaggatatc gcagaatctg tgactcacgc aatcgcaacc atattctctg gcttagaaca 9420

aaatcaagca gctgagatgt tgtgcctgtt gcgtctgtgg ggtcacccac tactcgagtc 9480

ccgtattgca gcaaaagcag ttaggagtca aatgtgcgca ccaaaaatgg tagactttga 9540

tatgatcctc caggtattat ctttctttaa aggaacaatc atcaatggat atagaaagaa 9600

gaatgcaggc gtgtggccac gtgtcaagat agatacgata tacgggaagg tcatagggca 9660

gctacacgca gattctgcag agatttcaca tgatgtcatg ttgagggaat acaagagttt 9720

atctgcactt gaattcgagc catgtataga gtatgaccct gtcaccaatc tgagcatgtt 9780

tttaaaagac aaggcaatcg cacacccgaa agacaactgg ctcgcttcgt ttaggcgaaa 9840

ccttctctct gaggaccaga agaaacatgt aagagaggca acctcaacta accgcctctt 9900

gatagagttc ttagagtcaa atgattttga tccatataaa gagatggaat atctgacgac 9960

ccttgagtac ctaagagatg ataatgtggc agtatcatac tcactcaaag aaaaagaggt 10020

gaaagttaat gggcggattt tcgcaaagct aacaaataga ttaaggaatt gtcaggtaat 10080

ggcagaaggg atcctagctg accagattgc acctttcttc cagggaaatg gggtcattca 10140

ggatagcata tctttgacta aaagtatgct agcgatgagt caactgtctt tcaacagcaa 10200

caagaaacgt attactgact gcaaagaaag agtatcctca aaccgcaatc atgatccgaa 10260

gagcaagaat cgtcggagag ttgccacttt tgtaactacc gacctgcaaa agtattgtct 10320

caattggaga tatcagacag tcaagctgtt cgctcatgcc atcaatcagc tgatgggctt 10380

acctcacttc ttcgagtgga ttcatcttag actaatggat actacgatgt ttgtagggga 10440

ccctttcaat cctccaagtg accctacgga ctgtgatcta tcaagagtcc caaatgatga 10500

catatatatt gtcagtgcta ggggaggcat tgagggatta tgccagaagc tatggacaat 10560

gatctcaatt gctgcaatcc agcttgctgc agcaagatca cattgtcgcg ttgcctgtat 10620

ggtacaaggc gacaatcaag taatagctat aacgagagag gtaagatcag atgactcccc 10680

agagatggtg ttaacacaat tgcatcaagc cagtgataat ttcttcaagg aattgattca 10740

tgtcaatcat ttgatcggcc ataatttgaa ggatcgtgaa accatcaggt cagacacgtt 10800

cttcatatac agcaaacgaa tattcaaaga tggagcaata ctcagtcagg tcctcaaaaa 10860

ctcatctaaa ttagtgctaa tatcaggcga ccttagtgaa aacactgtaa tgtcttgtgc 10920

caacattgca tctactgtag cacggttatg cgagaacggg cttcctaagg atttctgtta 10980

ttacttaaac tacttaatga gttgcgtgca gacatacttc gattccgaat tttccatcac 11040

caacaactcg caactcgatt ctaaccagtc gtggatagag gacatttctt ttgtgcactc 11100

atatgtcctg acccctgctc aactgggggg actgagtaac cttcaatact caaggctcta 11160

cacaaggaac atcggcgacc cgggaaccac tgctttcgca gagatcaaga gattagaggc 11220

agtggggtta ctgagtccta gcattatgac taacatctta actaggccgc ctggaaatgg 11280

agattgggcc agtctgtgca acgatccata ctcctttaat tttgagactg tcgcaagccc 11340

aaatattgtc cttaagaaac atacacaaag agtcctattt gaaacttgct cgaatccctt 11400

attatctgga gtgcacacag aggataatga ggcagaagag aaggcattgg ctgaattctt 11460

gctcaatcaa gaaatgattc atccacgtgt cgcgcatgct atcatggaag caagctctgt 11520

aggtaggaga aagcaaattc aggggcttgt tgacacaaca aacaccgtga ttaagattgc 11580

actgactagg aggccactcg gcatcaagag gctgatgcgg atagtcaatt actcgagcat 11640

gcatgcaatg ctatttagag atgatgtttt ctcgtctaat agatccaacc atcccttagt 11700

ctcttctgat atgtgttctc agacactggc agactatgca cggaacagaa gctggtcacc 11760

tttgacaggg ggtagaaaaa tactgggtgt atctaatcct gataccatag aacttgtaga 11820

gggtgagatc cttagtgtca gtggagggtg cactagatgt gacagtgggg atgaacagtt 11880

tacttggttc catcttccaa gcaatataga gctaaacgat gacaccagca agaatcctcc 11940

aatgagagtg ccatatctcg ggtcaaagac tcaagagagg agagccgcct cgcttgcgaa 12000

aatagctcat atgtcaccac acgtgaaggc ggctctaagg gcgtcatctg tgttaatctg 12060

ggcttatggg gacaacgaaa taaactggac tgctgccctt aagattgcaa ggtctcggtg 12120

caacataagc tcagagtatc ttcgactatt gtcacccttg cctacagctg ggaatctcca 12180

acatagattg gacgacggca taactcagat gacattcacc cctgcatctc tatacagggt 12240

gtcaccttac attcatatat ctaatgattc tcaaaggcta tttactgaag aaggagtcaa 12300

agaggggaat gtggtttatc agcaaatcat gctcttgggt ttgtccttaa ttgagtcact 12360

cttcccaatg acaacaacca agacatatga tgaaatcaca ttgcacctcc acagtaaatt 12420

tagctgctgt atcagggaag cacctgttgc agttcctttc gagctactcg gggtggcacc 12480

ggaactaagg gcagtaacct caaataagtt tatgtatgat cctagccctg tatcagaggg 12540

agactttgcg agacttgact tagctatctt taagagttat gaacttaatt tagagtcata 12600

ttccacaata gagctaatga acgttctttc aatatctagt gggaagttga ttggccagtc 12660

tgtggtttct tatgatgaag atacctccat aaagaatgac gctataatag tgtatgacaa 12720

cacacggaat tggatcagcg aagctcagaa ttcagatgtg gtccgcctat tcgagtatgc 12780

ggcactcgaa gtgctcctcg actgttctta tcaactctac tatctgagag taagaggcct 12840

aaacaatatc gtcctgtaca tgagtgattt atacaagaat atgccaggaa ttctactctc 12900

taatattgca gccacaatat ctcaccctgt catccattca aggttgaatg cagtaggtct 12960

ggtcaaccat gacgggtcac accaacttgc agacacagat ttcattgaaa tgtctgcaaa 13020

gctgctagtc tcttgcactc gacgcgtggt ctcaggttta catgcaggga ataagtatga 13080

cctgctgttt ccatctgtct tggatgataa cctaagtgag aagatgcttc agttgatatc 13140

ccaattatgc tgtctgtata cggtgctctt tgctacaaca agagaaatcc cgaaaataag 13200

aggcttatct gcagaagaga aatgttcagt acttactgag tacctactgt cagatgctgt 13260

gaaaccgtta cttgggtccg agcaagtgag ctctatcatg tctcccaaca tagttacgtt 13320

cccagccaat ctgtattaca tgtctaggaa gagccttaat ttgatcaggg agagagagga 13380

cagggatact atcttggcat tgttgttccc tcaagaaccg ctgctcgagt ttcctctggt 13440

acgagatatt ggtgctcgtg taaaagatcc atttacccta caacctgcgg cgtttttaca 13500

agagttagat ttgagtgctc cggcaaggta tgacgcattc acaatcagtc aggcgcattc 13560

tgaacacata ttgccaaacc cagaggaaga tcacttagta cgatacttgt tcagaggaat 13620

agggactgcg tcctcctctt ggtataaggc atcccatctt ctttctgtac ccgaggtcag 13680

atgtgcaagg catgggaact ccttatattt agcagaagga agtggagcca tcatgagtct 13740

tctcgaattg catataccac acgaaactat ctattacaat acacttttct cgaacgagat 13800

gaacccccca cagcgacatt tcggaccaac tccaacacag tttctgaatt cggttgtttt 13860

taggaattta caggcggaag taccatgcaa ggatggattt gtccaggagt tccgtccgtt 13920

atggagagag aatacagaag aaagcgatct gacctcagat aaagcagtgg gatatatcac 13980

atctgtagtg ccgtacaggt ctgtatcatt gctgcattgt gacattgaaa tccctccagg 14040

atctaatcaa agcttactag atcagctggc taccaatctg tctctgattg ccatgcattc 14100

tgtgaaggag ggcggggtcg tgattatcaa agtactgtat gcaatgggat attacttcca 14160

tctactcatg aacttgttta ctccatgttc cacgaaagga tatattctct ctaatggcta 14220

tgcctgtaga ggggatatgg agtgttacct gatatttgtc atgggctacc taggcgggcc 14280

tacatttgta catgaggtgg tgaggatggc aaaaacgcta gtacagcggc atggcacact 14340

tttgtctaag tcagatgaga ttacactgac taggttattt acttcacagc aacagcgtgt 14400

aacagacatc ctatccagcc ctttaccgag actaatgaag tacttgagag agaatattga 14460

tactgcactg attgaagccg ggggacagcc tgtccgtcca ttctgtgcag agagtttagt 14520

gagcacacta acagacatga ctcagacaac ccagatcatc gccagccaca ttgatacagt 14580

cattcgatct gtgatctata tggaagcaga gggtgatctt gctgacacag tattcttatt 14640

taccccttac aacctctcta tggacgggaa aaagagaaca tcacttaaac agtgcacaag 14700

acagatctta gaggtcacaa tactgggtct cagagtcaaa aatctcaata aagtaggtga 14760

tgtaatcagc ctagtactca gaggtatgat ttctctggag gaccttctcc cactgagaac 14820

ctacttgaag tgtagtacct gccctaagta tttgaaggct gtcctaggta ttaccaaact 14880

caaagaaatg ttcacagacg cctctttatt atacttgact cgtgctcaac aaaaattcta 14940

catgaaaact ataggcaatg cagtcaaggg atactatagt aactgtgact cttaaaggca 15000

accacatatc aataggccct ctttctagcc gatcgtattc ttgttgactt cattatacca 15060

tattagaaaa aaattgaatt ccgacccttt aagactcgta ttcggattca aataattatc 15120

tcagaaaaaa agtgcacgta gttgttcttg attatagtcc cgtcattcac caagtctttg 15180

tttggt 15186

<210> 3
<211> 17412
<212> DNA
<213> Newcastle disease virus

<220>
<223> Mut HN (F277L)/M(G165W)/F(F117S)/F(F190L)/L(V757I)/L(F1551S)/L(R1
      700L)-Nivolumab

<400> 3
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360
ttctcaagtg atgaggaacc atgtcgccct tgcagggaaa cagaatgagg ccacactggc 420
tgttcttgag atcgatggtt tcaccaacag cgtgcctcaa tttaacaaca ggagtggagt 480
gtctgaggag agagcacaga gattcttgat gatagcaggg tccctccctc gggcatgcag 540
caacggtacc ccgttcatca cagctggggt tgaagatgat gcaccagaag acatcactga 600
tactctggaa agaatcatct ctatccaggc tcaagtatgg gtcacggtag caaaggccat 660
gactgcatat gagacagcgg atgagtcgga aacaagaaga atcaataagt atatgcagca 720
aggcagagtc caaaagaagt acatcctcca ccccgtatgt aggagcgcaa ttcaactcac 780
gattagacaa tctctggcag tccgcatttt cttggttagc gagcttaaga gaggccgcaa 840
cacggcaggt ggaagttcca cttattatag cttggtaggg gacatagact catacatcag 900
gaacaccggg cttactgcat tcttcctgac actcaggtat ggaattaaca ccaagacatc 960
agcccttgca ctcagcagcc tcgcaggcga tatccaaaaa atgaagcagc tcatgcgtct 1020
atatcggatg aaaggagata atgcgccgta catgacactg ctcggtgaca gtgaccagat 1080
gagctttgca ccggctgagt atgcacaact ctactctttt gcaatgggta tggcatccgt 1140
cctagacaaa ggaactggca agtaccagtt cgccagagac tttatgagca catcattctg 1200
gagacttgga gtagagtatg ctcgggctca gggaagcagc atcaatgagg atatggctgc 1260
cgagctaaag ctgaccccag cggcaaggag gggcctggca gctgctgccc aacgagcgtc 1320
cgaggagacc ggcagcatgg acattcccac tcaacaagct ggggtcctca cagggctcag 1380
cgacggaggc cctcaagccc cacaaggtgg actgaacagg tcgcaagggc aaccggacgc 1440
cggagatggg gagacccaat ttttagattt gatgagagca gtggcaaata gtatgagaga 1500
agcgccaaac tctgtgcaga acaccactca gcaggagcct ccatctactc ctgggccatc 1560
tcaagacaac gacaccgact ggggatactg accgacaaca ctcagcctgc ctccatcgaa 1620
ttatctcgat tcttctgcct gtaacctaac ccctggtcca cagtctcaca cggccaaacc 1680
cgcaaacgag cccccccctc tctccccccc cctccagcca cacggctcca accgcccaaa 1740
acaacacggg cacaactcga ctcactcata atctacacag agccgaagat cttagaaaaa 1800
aatacgggta gaagagagac attcagagat caggacaaat catcagggtc tccgctctcc 1860
cctctaccca gcagaccaga gtgaagatgg ccaccttcac ggatgcagag atcgacgatc 1920
tatttgaaac cagtggaact gtcattgaca gcataattac ggcccaaggc aacccagtag 1980
agactgttgg aaggagcgca atcccacaag gcaaaactaa agcattgagt gcagcatggg 2040
agaaacatgg aagcacccaa ccgccggccg gccaagacac ccctgatcga atggacagat 2100
cagacaaaca accatcccca cccgaacagg caaccacaca caacagcccg ccagccacat 2160
ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatgga gttcggcctg tcctgggtgt tcctggtggc cctgtttaga ggcgtgcagt 3240

gccaggtgca gctggtggag agcggaggag gagtggtgca gccaggccgg agcctgcgcc 3300

tggactgtaa ggccagcggc atcaccttta gcaactccgg aatgcactgg gtgaggcagg 3360

caccaggcaa gggcctggag tgggtggccg tgatctggta cgacggctct aagcggtact 3420

atgccgatag cgtgaagggc cggttcacaa tctccagaga caactctaag aataccctgt 3480

tcctccagat gaacagcctg agggccgagg atacagccgt gtactattgc gccaccaatg 3540

acgattactg gggccagggc acactggtga ccgtgagcag cgccagcacc aagggaccaa 3600

gcgtgttccc actggcacct tgctctcgca gcacatccga gtctaccgcc gccctgggat 3660

gtctggtgaa ggattatttc cccgagcctg tgaccgtgag ctggaacagc ggcgccctga 3720

catccggagt gcacaccttt cccgccgtgc tccagagcag cggcctgtac agcctgtcct 3780

ctgtggtgac agtgcccagc tcctctctgg gcaccaagac atatacctgc aacgtggacc 3840

acaagcctag caataccaag gtggataagc gggtggagtc caagtacgga ccaccttgcc 3900

caccatgtcc agcaccagag ttcctgggag gaccaagcgt gttcctgttt cctccaaagc 3960

ccaaggacac cctgatgatc tccagaacac cagaggtgac ctgcgtggtg gtggacgtgt 4020

ctcaggagga ccccgaggtg cagttcaact ggtatgtgga tggcgtggag gtgcacaatg 4080

ccaagaccaa gcccagggag gagcagttta actccacata ccgcgtggtg tctgtgctga 4140

ccgtgctgca ccaggactgg ctgaacggca aggagtataa gtgcaaggtg tccaataagg 4200

gcctgcctag ctccatcgag aagacaatca gcaaggcaaa gggacagcca agggagccac 4260

aggtgtacac cctgccccct tctcaggagg agatgacaaa gaaccaggtg agcctgacct 4320

gtctggtgaa gggcttctat ccttccgaca tcgccgtgga gtgggagtct aatggccagc 4380

cagagaacaa ttacaagacc acaccacccg tgctggactc tgatggcagc ttctttctgt 4440

attccaggct gacagtggat aagtctcgct ggcaggaggg caacgtgttt agctgctccg 4500

tgatgcacga ggccctgcac aatcactaca cccagaagtc tctgagcctg tccctgggca 4560

agcggagaaa gaggggcagc ggagagggaa ggggctccct gctgacatgt ggcgacgtgg 4620

aggagaatcc tggcccaatg aagtatctgc tgcccaccgc cgccgccggc ctgctgctgc 4680

tggcagcaca gcctgctatg gccgagatcg tgctgacaca gtccccagcc accctgtctc 4740

tgagccctgg agagagggcc accctgagct gtagagcatc ccagagcgtg agcagctacc 4800

tggcctggta tcagcagaag cctggacagg caccaaggct gctgatctac gatgcaagca 4860

acagggcaac aggcatcccc gcaagattca gcggctccgg ctctggcacc gactttacac 4920

tgaccatctc ctctctggag cctgaggatt tcgccgtgta ctattgccag cagagcagca 4980

attggccacg gacatttggc cagggcacca aggtggagat caagagaaca gtggccgccc 5040

ctagcgtgtt catctttcct ccatccgacg agcagctgaa gagcggcacc gcatccgtgg 5100

tgtgcctgct gaacaatttc tacccacggg aggccaaggt gcagtggaag gtggataacg 5160

ccctccagtc cggcaattct caggagagcg tgacagagca ggactccaag gattctacct 5220

atagcctgtc tagcacactg accctgtcca aggccgacta cgagaagcac aaggtgtatg 5280

cctgcgaggt gacacaccag ggcctgtcct ctcccgtgac caagagcttt aacagaggcg 5340

agtgttgatg agctgaccga gccctcctct ataaacccaa gattcaaggc tccaagcgat 5400

aaccttctcc cgcctcttcc atcccactga atgatcgcgc agctgcaatt aatctagcaa 5460

cattaaagat taagaaaaaa tacgggtaga atcggagtgc accgattgtg ccaagatgga 5520

ctcatctagg acaattgggt tatactttga ttctgccctt ccttctagca acctgttagc 5580

attcccgatt gtcttacgag atgtaggaga tggaaagaag caaattactc cgcaatatag 5640

gatccggcgt cttgactcgt ggacagacag taaagaagac tcggtattca tcactaccta 5700

cggattcatc ttccaggttg ggaatgaaga agtcactgtc ggcatgatca atgataatcc 5760

caagcgagag ttactctccg ctgcgatgct ctgcctagga agcgtcccga atgttggaga 5820

tcctgttgag ctggcaaggg cctgcctcac tatggtggta acatgcaaga agagtgcaac 5880

taatactgag agaatggtct tctcagtagt gcaggcaccc cgggtgctgc aaagctgtag 5940

ggtcgtggca gacaaatact cgtcagtgaa tgcagttaag cacgtgaaag caccagagaa 6000

gatcccttgg agtgaaaccc tagagtacaa ggtgaatttt gtctctttga ctgtggtgcc 6060

gaagaaggat gtctacaaga tcccaaccgc agtattgaag gtatccggct cgagcctgta 6120

taatcttgcg ctcaatgtca ctattgatgt ggaggtagac ccaaagagcc cgttagtcaa 6180

gtccctttct aggtccgaca gtggatacta tgctaatctc ttcttacata tcggacttat 6240

gtccactgta gataagaggg gaaagaaagt gacatttgac cagctggaga ggaagataag 6300

gagacttgat ctatctgtcg ggctcagtga tgtgctcgga ccttctgtgc ttgtgaaggc 6360

gagaggtgca cggactaggc tgctggcacc tttcttctct aacagcggga cagcctgcta 6420

ccctatagca aatgcctctc ctcaggtagc taagatactc tggagtcaga ccgcgtgcct 6480

gcggagtgta aaaatcatta ttcaagcggg cacccaacgc gctgtcgcag tgactgctga 6540

ccatgaggtt acctctacta agatagagaa gaggcatacc atcgctaaat acaatccctt 6600

caagaaatag gccgtatccc tgagactgca attcacccgc cttcccaaaa caccatgaca 6660

ccagataatg atctgtcttg attacttaca gttagtttcc ctgtctatca aattagaaaa 6720

aacacgggta gaagagttcg gatcccggcc ggcgcaccaa aagcgcaaga tgggccccag 6780

atcttctacc aggatcccag tacctctaat gctgaccata cggatcacgc tggcactgag 6840

ttatgtccgt ctgacaagtt ctcttgatgg caggcctctt gcagccgcag ggatcgtggt 6900

aacaggggat aaagcagtta acatatacac ctcatcccag acagggtcaa tcatagtcaa 6960

gttactccca aatatgccca aggacaaaga ggcatgtgca aaagccccat tggaggctta 7020

caacaggaca ctgactactt tgcttacccc ccttggtgat tctatccgca ggatacaaga 7080

gtctgtgact acatccggag gaaggagaca gagacgctct ataggtgcca ttattggcag 7140

tgtagctcta ggggttgcaa cagctgcaca gataacggca gcctcggctc tgatacaagc 7200

caaccagaat gctgctaaca tcctccggct taaggagagc attgctgcaa ccaatgaagc 7260

tgtacacgag gtcactggcg gattgtcaca gttagcagtg gcagttggaa agatgcagca 7320

atttgttaat gaccagctta ataacacagc tcaagagttg gactgtataa aaattacaca 7380

gcaggttggt gtagaactca acttatactt aactgaattg actacagtgt tcgggccaca 7440

aatcacttcc cctgccttaa ctcagttgac tgttcaggct ctttacaatc tggctggtgg 7500

taatgtagat tacttgttga ctaagttagg tgtagggaac aaccagctca gctcattgat 7560

tggtagcggc ttgatcaccg gtaaccctat tttttacgac tcacagactc aactcttggg 7620

catacaggtg actttaccct cagtcgggaa cctaaataat atgcgtgcca cctacttgga 7680

gaccttgtct gtaagcacaa caaagggatt tgcctcagca cttgtcccaa aagtagtgac 7740

acaggtcggt tctgtgatag aagagcttga cacctcatac tgtatagaag ctgatttgga 7800

tttatattgt acaagaatag tgacattccc tatgtcccct ggtatttatt cctgtttgag 7860

cggcaataca tcggcttgca tgtattcaag gactgaaggc gcacttacta caccatacat 7920

gactctcaaa ggctcagttg ttgccaattg ccagatgaca acatgtagat gtgcagaccc 7980

cccgggtatc atatcacaaa attatggaga ggctgtgtct ctaatagata agcactcatg 8040

caatgtcgta tccttagacg ggataacttt gaggctcagt ggggaatttg atgcaactta 8100

tcaaaagaat atctcaatat tagattctca agtactagtg acaggcaatc tcgatatctc 8160

aactgagctt gggaatgtca accactcaat aagtaatgct ttggataagt tagaggaaag 8220

caacagcaaa ctagacaaag tcaatgtcaa actgaccagc acatctgctc tcattaccta 8280

tattgtttta actgtcatat ctcttgttct tggtatgctt agcctggttc tagcatgcta 8340

tctgatgtac aagcaaaaag cgcaacgaaa gaccttgttg tggcttggaa ataataccct 8400

agatcagatg agagctacta caaaaatgtg aatgcagatg agaggcagag gtatccccaa 8460

tagcaatctg tgtgtcaatt ctggcagcct gttaatcaga agaattaaga aaaaactacc 8520

ggatgtaggt gaacaaaagg gaatatacgg gtagaacggc ctgagaggcc acccctcaat 8580

cgggagccag gccccactac gtccgctcta ccgcatcacc aacagcagtc ttcagtcatg 8640

gacagcgcag ttagccaagt tgcgctagag aatgatagaa gagaagcgaa taatacatgg 8700

cgcttggttt tccggatcgc agccttactt ttactggtaa taaccttagc cgtctctgca 8760

gtcgccctgg catatagtat ggaggctagc acacctggcg accttgtaag cataccgact 8820

gcgatctcta gggcagagga aaggattaca tctgcactcg gttccaatca agatgtagta 8880

gataggatat acaagcaagt ggcccttgag tctccattgg cactgctaaa taccgaatct 8940

ataattatga atgcaataac gtctctctct tatcaaatca atggagccac aaataatagc 9000

gggtgtgggg cacctgttca tgacccagat tacatcgggg ggataggtca agaacttatt 9060

gtagatgata cgagtgacgt cacatcattt tatccctctg cattccaaga acacctgaat 9120

tttatcccgg cgcccactac aggatcaggc tgcactcgga taccctcatt cgacatgagt 9180

gctacccatt actgttacac tcacaatgtg atattgtctg gctgcagaga tcactcacac 9240

tcacatcagt atttagcact tggtgtgctt cggacatctg caacagggag ggtattcttt 9300

tctactctgc gttccatcaa cttggatgac gcccaaaatc ggaagtcttg cagtgtgagt 9360

gcaactcctt taggttgtga tatgctgtgc tctaaaatca cagagactga ggaagaggat 9420

tataaatcag ttatccccac atcgatggta catggaaggt tagggcttga cggccaatac 9480

catgagaagg acctagacgt cacaacacta tttagggact gggtggcaaa ttacccagga 9540

gtaggaggtg ggtcttttat taacaatcgc gtatggttcc cggtctacgg agggctaaaa 9600

cccagctcgc ctagtgacac tgcacaagaa gggagatacg taatatacaa gcgatacaat 9660

gacacatgcc cggatgagca agattaccag attcggatgg ctaagtcatc gtataagcct 9720

aggcggtttg gtggaaaacg cgtacagcag gccatcctat ccatcaaggt gtcaacatcc 9780

ttgggtgagg acccggtgtt gactgtaccg cctaatacgg tcgcactcat gggggccgaa 9840

ggcagagttc tcacagtggg gacatctcat ttcttatatc agcgagggtc atcatacttc 9900

tctcccgctt tgttataccc tatgacagtc aacaacaaaa cagccactct tcataatcct 9960

tatacattca atgctttcac tcggccaggt agtgtccctt gccaggcttc agcaagatgc 10020

cccaactcat gtgttaccgg agtctatact gatccatatc ccttagtctt ccataggaac 10080

cacaccttgc gaggggtatt cggaacaatg cttgatgacg aacaagcaag actcaaccct 10140

gtatctgcag tatttgataa catatcccgc agtcgcataa ctcgggtaag ttcaagcagt 10200

accagggcag catacacgac atcaacgtgt tttaaagttg tcaagaccaa taaaacctat 10260

tgcctcagca ttgcagaaat atccaatacc ctcttcgggg aattcagaat tgtcccctta 10320

ctagttgaga ttctcaagga tggtggggtt taagaagcta ggaatggtcg gttgagtcaa 10380

ccgtgagaag gcggggaaga tgatattgca tcatctatct tctgtaacac caagaatcaa 10440

atcgaatacc agtgcgagct cgaatcctac gctgccagtc agccataatc ggctggtgct 10500

aatgtggtta gcctgaatct tgtcgatagt cacttgatta agaaaaaatg tggacggtag 10560

tgaaatataa ggcaaagcag ctcacagagg acagcacggg taggacatgg cgggctccgg 10620

acccgaaagg gcagagcatc agattatcct accagagtca catctgtcct caccattggt 10680

caagcataaa ttgctttatt actggaaatt aactgggcta ccgcttcctg acgaatgtga 10740

cttcgaccat cttattatca gccgacaatg gaagaaggta cttgaatcgg ccacccctga 10800

cattgagaga atgataaaac tagggcgggc agtacaccag actctcaacc acaattccag 10860

gataaccgga gtactccatc cccggtgttt agaggaactg gctagtattg aggttcctga 10920

ttcaaccaac aaatttcgga agatcgaaaa aaagatccag attcacaaca caaggtatgg 10980

agaactgttc acaagattat gcacgcatgt agaaaagaaa ctattggggt catcttggtc 11040

taacaatgtc ccacggtcag aggaattcaa cagcatccgt acagatccgg cattttggtt 11100

tcactcaaaa tggtccacag ccaaatttgc atggctccat ataaaacagg tccagaggca 11160

tctgattgta gcagcaagaa caaggtccgc agtcaacaaa ttagtgacgc tgacccataa 11220

ggtaggccaa atctttgtta ctcctgagct tgtcattgtg acacatacag atgagaacaa 11280

gttcacgtgt cttacccagg aacttgtgtt gatgtatgca gatatgatgg agggcagaga 11340

tatggtcagc ataatatcat ccacggcggc acatcttagg agcttatcag agaaaattga 11400

tgatattctg cggttagtag atgctctggc aagagatttg ggcaatcaag tctacgatgt 11460

tgtagcacta atggagggat tcgcatacgg cgctgttcag ctgcttgaac cgtcaggtac 11520

atttgcgggg gatttcttcg cattcaacct gcaggagctc aaagatactc taaccggact 11580

cctccccaag gatatcgcag aatctgtgac tcacgcaatc gcaaccatat tctctggctt 11640

agaacaaaat caagcagctg agatgttgtg cctgttgcgt ctgtggggtc acccactact 11700
cgagtcccgt attgcagcaa aagcagttag gagtcaaatg tgcgcaccaa aaatggtaga 11760
ctttgatatg atcctccagg tattatcttt ctttaaagga acaatcatca atggatatag 11820
aaagaagaat gcaggcgtgt ggccacgtgt caagatagat acgatatacg ggaaggtcat 11880
agggcagcta cacgcagatt ctgcagagat ttcacatgat gtcatgttga gggaatacaa 11940
gagtttatct gcacttgaat tcgagccatg tatagagtat gaccctgtca ccaatctgag 12000
catgttttta aaagacaagg caatcgcaca cccgaaagac aactggctcg cttcgtttag 12060
gcgaaacctt ctctctgagg accagaagaa acatgtaaga gaggcaacct caactaaccg 12120
cctcttgata gagttcttag agtcaaatga ttttgatcca tataaagaga tggaatatct 12180
gacgaccctt gagtacctaa gagatgataa tgtggcagta tcatactcac tcaaagaaaa 12240
agaggtgaaa gttaatgggc ggattttcgc aaagctaaca aatagattaa ggaattgtca 12300
ggtaatggca gaagggatcc tagctgacca gattgcacct ttcttccagg gaaatggggt 12360
cattcaggat agcatatctt tgactaaaag tatgctagcg atgagtcaac tgtctttcaa 12420
cagcaacaag aaacgtatta ctgactgcaa agaaagagta tcctcaaacc gcaatcatga 12480
tccgaagagc aagaatcgtc ggagagttgc cacttttgta actaccgacc tgcaaaagta 12540
ttgtctcaat tggagatatc agacagtcaa gctgttcgct catgccatca atcagctgat 12600
gggcttacct cacttcttcg agtggattca tcttagacta atggatacta cgatgtttgt 12660
aggggaccct ttcaatcctc caagtgaccc tacggactgt gatctatcaa gagtcccaaa 12720
tgatgacata tatattgtca gtgctagggg aggcattgag ggattatgcc agaagctatg 12780
gacaatgatc tcaattgctg caatccagct tgctgcagca agatcacatt gtcgcgttgc 12840
ctgtatggta caaggcgaca atcaagtaat agctataacg agagaggtaa gatcagatga 12900
ctccccagag atggtgttaa cacaattgca tcaagccagt gataatttct tcaaggaatt 12960
gattcatgtc aatcatttga tcggccataa tttgaaggat cgtgaaacca tcaggtcaga 13020
cacgttcttc atatacagca aacgaatatt caaagatgga gcaatactca gtcaggtcct 13080
caaaaactca tctaaattag tgctaatatc aggcgacctt agtgaaaaca ctgtaatgtc 13140
ttgtgccaac attgcatcta ctgtagcacg gttatgcgag aacgggcttc ctaaggattt 13200
ctgttattac ttaaactact taatgagttg cgtgcagaca tacttcgatt ccgaattttc 13260
catcaccaac aactcgcaac tcgattctaa ccagtcgtgg atagaggaca tttcttttgt 13320
gcactcatat gtcctgaccc ctgctcaact ggggggactg agtaaccttc aatactcaag 13380
gctctacaca aggaacatcg gcgacccggg aaccactgct ttcgcagaga tcaagagatt 13440
agaggcagtg gggttactga gtcctagcat tatgactaac atcttaacta ggccgcctgg 13500
aaatggagat tgggccagtc tgtgcaacga tccatactcc tttaattttg agactgtcgc 13560

aagcccaaat attgtcctta agaaacatac acaaagagtc ctatttgaaa cttgctcgaa 13620

tcccttatta tctggagtgc acacagagga taatgaggca gaagagaagg cattggctga 13680

attcttgctc aatcaagaaa tgattcatcc acgtgtcgcg catgctatca tggaagcaag 13740

ctctgtaggt aggagaaagc aaattcaggg gcttgttgac acaacaaaca ccgtgattaa 13800

gattgcactg actaggaggc cactcggcat caagaggctg atgcggatag tcaattactc 13860

gagcatgcat gcaatgctat ttagagatga tgttttctcg tctaatagat ccaaccatcc 13920

cttagtctct tctgatatgt gttctcagac actggcagac tatgcacgga acagaagctg 13980

gtcacctttg acagggggta gaaaaatact gggtgtatct aatcctgata ccatagaact 14040

tgtagagggt gagatcctta gtgtcagtgg agggtgcact agatgtgaca gtggggatga 14100

acagtttact tggttccatc ttccaagcaa tatagagcta aacgatgaca ccagcaagaa 14160

tcctccaatg agagtgccat atctcgggtc aaagactcaa gagaggagag ccgcctcgct 14220

tgcgaaaata gctcatatgt caccacacgt gaaggcggct ctaagggcgt catctgtgtt 14280

aatctgggct tatggggaca acgaaataaa ctggactgct gcccttaaga ttgcaaggtc 14340

tcggtgcaac ataagctcag agtatcttcg actattgtca cccttgccta cagctgggaa 14400

tctccaacat agattggacg acggcataac tcagatgaca ttcacccctg catctctata 14460

cagggtgtca ccttacattc atatatctaa tgattctcaa aggctattta ctgaagaagg 14520

agtcaaagag gggaatgtgg tttatcagca aatcatgctc ttgggtttgt ccttaattga 14580

gtcactcttc ccaatgacaa caaccaagac atatgatgaa atcacattgc acctccacag 14640

taaatttagc tgctgtatca gggaagcacc tgttgcagtt cctttcgagc tactcggggt 14700

ggcaccggaa ctaagggcag taacctcaaa taagtttatg tatgatccta gccctgtatc 14760

agagggagac tttgcgagac ttgacttagc tatctttaag agttatgaac ttaatttaga 14820

gtcatattcc acaatagagc taatgaacgt tctttcaata tctagtggga agttgattgg 14880

ccagtctgtg gtttcttatg atgaagatac ctccataaag aatgacgcta taatagtgta 14940

tgacaacaca cggaattgga tcagcgaagc tcagaattca gatgtggtcc gcctattcga 15000

gtatgcggca ctcgaagtgc tcctcgactg ttcttatcaa ctctactatc tgagagtaag 15060

aggcctaaac aatatcgtcc tgtacatgag tgatttatac aagaatatgc caggaattct 15120

actctctaat attgcagcca caatatctca ccctgtcatc cattcaaggt tgaatgcagt 15180

aggtctggtc aaccatgacg ggtcacacca acttgcagac acagatttca ttgaaatgtc 15240

tgcaaagctg ctagtctctt gcactcgacg cgtggtctca ggtttacatg cagggaataa 15300

gtatgacctg ctgtttccat ctgtcttgga tgataaccta agtgagaaga tgcttcagtt 15360

gatatcccaa ttatgctgtc tgtatacggt gctctttgct acaacaagag aaatcccgaa 15420

aataagaggc ttatctgcag aagagaaatg ttcagtactt actgagtacc tactgtcaga 15480

tgctgtgaaa ccgttacttg ggtccgagca agtgagctct atcatgtctc ccaacatagt 15540

tacgttccca gccaatctgt attacatgtc taggaagagc cttaatttga tcagggagag 15600

agaggacagg gatactatct tggcattgtt gttccctcaa gaaccgctgc tcgagtttcc 15660

tctggtacga gatattggtg ctcgtgtaaa agatccattt accctacaac ctgcggcgtt 15720

tttacaagag ttagatttga gtgctccggc aaggtatgac gcattcacaa tcagtcaggc 15780

gcattctgaa cacatattgc caaacccaga ggaagatcac ttagtacgat acttgttcag 15840

aggaataggg actgcgtcct cctcttggta taaggcatcc catcttcttt ctgtacccga 15900

ggtcagatgt gcaaggcatg ggaactcctt atatttagca gaaggaagtg gagccatcat 15960

gagtcttctc gaattgcata taccacacga aactatctat tacaatacac ttttctcgaa 16020

cgagatgaac cccccacagc gacatttcgg accaactcca acacagtttc tgaattcggt 16080

tgtttttagg aatttacagg cggaagtacc atgcaaggat ggatttgtcc aggagttccg 16140

tccgttatgg agagagaata cagaagaaag cgatctgacc tcagataaag cagtgggata 16200

tatcacatct gtagtgccgt acaggtctgt atcattgctg cattgtgaca ttgaaatccc 16260

tccaggatct aatcaaagct tactagatca gctggctacc aatctgtctc tgattgccat 16320

gcattctgtg aaggagggcg gggtcgtgat tatcaaagta ctgtatgcaa tgggatatta 16380

cttccatcta ctcatgaact tgtttactcc atgttccacg aaaggatata ttctctctaa 16440

tggctatgcc tgtagagggg atatggagtg ttacctgata tttgtcatgg gctacctagg 16500

cgggcctaca tttgtacatg aggtggtgag gatggcaaaa acgctagtac agcggcatgg 16560

cacacttttg tctaagtcag atgagattac actgactagg ttatttactt cacagcaaca 16620

gcgtgtaaca gacatcctat ccagcccttt accgagacta atgaagtact tgagagagaa 16680

tattgatact gcactgattg aagccggggg acagcctgtc cgtccattct gtgcagagag 16740

tttagtgagc acactaacag acatgactca gacaacccag atcatcgcca gccacattga 16800

tacagtcatt cgatctgtga tctatatgga agcagagggt gatcttgctg acacagtatt 16860

cttatttacc ccttacaacc tctctatgga cgggaaaaag agaacatcac ttaaacagtg 16920

cacaagacag atcttagagg tcacaatact gggtctcaga gtcaaaaatc tcaataaagt 16980

aggtgatgta atcagcctag tactcagagg tatgatttct ctggaggacc ttctcccact 17040

gagaacctac ttgaagtgta gtacctgccc taagtatttg aaggctgtcc taggtattac 17100

caaactcaaa gaaatgttca cagacgcctc tttattatac ttgactcgtg ctcaacaaaa 17160

attctacatg aaaactatag gcaatgcagt caagggatac tatagtaact gtgactctta 17220

aaggcaacca catatcaata ggccctcttt ctagccgatc gtattcttgt tgacttcatt 17280

ataccatatt agaaaaaaat tgaattccga ccctttaaga ctcgtattcg gattcaaata 17340

attatctcag aaaaaaagtg cacgtagttg ttcttgatta tagtcccgtc attcaccaag 17400

tctttgtttg gt 17412

<210> 4
<211> 17436
<212> DNA
<213> Newcastle disease virus

<220>
<223> Mut HN (F277L)/M(G165W)/F(F117S)/F(F190L)/L(V757I)/L(F1551S)/L(R1
      700L)-Ipilimumab

<400> 4
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatgga gtttggcctg tcatgggtct ttctggtcgc tctgtttagg ggggtgcagt 3240

gtcaggtgca gctggtcgag tcaggggggg gcgtggtgca gccaggcagg tccctgaggc 3300

tgtcttgcgc agccagcggc ttcaccttta gctcctacac aatgcactgg gtgcggcagg 3360

cacctggcaa gggcctggag tgggtgacct tcatctccta tgacggcaac aataagtact 3420

atgccgattc tgtgaagggc cggtttacca tctccagaga caactctaag aatacactgt 3480

acctgcagat gaacagcctg agggccgagg acaccgccat ctactattgc gcaaggacag 3540

gatggctggg acctttcgat tattggggcc agggcaccct ggtgacagtg tctagcgcct 3600

ctaccaaggg cccaagcgtg tttcccctgg ccccttcctc taagtccacc tctggaggca 3660

cagccgccct gggctgtctg gtgaaggatt acttccctga gccagtgacc gtgagctgga 3720

actccggcgc cctgaccagc ggagtgcaca catttccagc cgtgctgcag agcagcggcc 3780

tgtactccct gtctagcgtg gtgaccgtgc cctcctctag cctgggcacc cagacatata 3840

tctgcaacgt gaatcacaag cctagcaata caaaggtgga caagcgggtg gagccaaagt 3900

cctgtgataa gacccacaca tgcccacctt gtccagcacc tgagctgctg ggaggaccat 3960

ccgtgttcct gtttccaccc aagcccaagg acaccctgat gatcagccgg acccctgagg 4020

tgacatgcgt ggtggtggac gtgagccacg aggaccccga ggtgaagttc aactggtacg 4080

tggatggcgt ggaggtgcac aatgccaaga ccaagccccg ggaggagcag tacaactcca 4140

cctatagagt ggtgtctgtg ctgacagtgc tgcaccagga ctggctgaac ggcaaggagt 4200

ataagtgcaa ggtgtccaat aaggccctgc cagcccccat cgagaagacc atctctaagg 4260

caaagggaca gccacgggag ccacaggtgt acacactgcc tcccagccgg gacgagctga 4320

ccaagaacca ggtgagcctg acatgtctgg tgaagggctt ctatcccagc gacatcgccg 4380

tggagtggga gtccaatggc cagcctgaga acaattacaa gaccacaccc cctgtgctgg 4440

actctgatgg cagcttcttt ctgtattcta agctgaccgt ggacaagagc agatggcagc 4500

agggcaacgt gtttagctgc tccgtgatgc acgaggccct gcacaatcac tacacacaga 4560

agtctctgag cctgtcccct ggcaagcgga gaaagagggg cagcggagag ggaaggggct 4620

ccctgctgac ctgtggcgat gtggaggaga atcctggccc aatgaagtat ctgctgccaa 4680

cagctgccgc cggcctgctg ctgctggccg cacagcccgc tatggccgag atcgtgctga 4740

cccagagccc aggcacactg tctctgagcc ctggagagag ggccaccctg tcctgcagag 4800

cctcccagtc tgtgggctcc tcttacctgg cctggtatca gcagaagcct ggccaggccc 4860

caaggctgct gatctacgga gccttcagca gggccaccgg catccccgac cgcttcagcg 4920

gctccggctc tggcacagac ttcaccctga caatctccag gctggagccc gaggacttcg 4980

ccgtgtacta ttgccagcag tacggcagct ccccttggac ctttggccag ggcacaaagg 5040

tggagatcaa gaggaccgtg gcagcaccat ccgtgttcat ctttccaccc tctgacgagc 5100

agctgaagag cggcacagcc tccgtggtgt gcctgctgaa caacttctac ccccgcgagg 5160

ccaaggtgca gtggaaggtg gataacgccc tgcagtccgg caattctcag gagagcgtga 5220

ccgagcagga ctccaaggat tctacatata gcctgtctag caccctgaca ctgagcaagg 5280

ccgattacga gaagcacaag gtgtatgcct gtgaggtcac ccaccaggga ctgtcatcac 5340

ccgtcaccaa aagtttcaat agaggagagt gctgagctga ccgagccctc ctctataaac 5400

ccaagattca aggctccaag cgataacctt ctcccgcctc ttccatccca ctgaatgatc 5460

gcgcagctgc aattaatcta gcaacattaa agattaagaa aaaatacggg tagaatcgga 5520

gtgcaccgat tgtgccaaga tggactcatc taggacaatt gggttatact ttgattctgc 5580

ccttccttct agcaacctgt tagcattccc gattgtctta cgagatgtag gagatggaaa 5640

gaagcaaatt actccgcaat ataggatccg gcgtcttgac tcgtggacag acagtaaaga 5700

agactcggta ttcatcacta cctacggatt catcttccag gttgggaatg aagaagtcac 5760

tgtcggcatg atcaatgata atcccaagcg agagttactc tccgctgcga tgctctgcct 5820

aggaagcgtc ccgaatgttg gagatcctgt tgagctggca agggcctgcc tcactatggt 5880

ggtaacatgc aagaagagtg caactaatac tgagagaatg gtcttctcag tagtgcaggc 5940

accccgggtg ctgcaaagct gtagggtcgt ggcagacaaa tactcgtcag tgaatgcagt 6000

taagcacgtg aaagcaccag agaagatccc ttggagtgaa accctagagt acaaggtgaa 6060

ttttgtctct ttgactgtgg tgccgaagaa ggatgtctac aagatcccaa ccgcagtatt 6120

gaaggtatcc ggctcgagcc tgtataatct tgcgctcaat gtcactattg atgtggaggt 6180

agacccaaag agcccgttag tcaagtccct ttctaggtcc gacagtggat actatgctaa 6240

tctcttctta catatcggac ttatgtccac tgtagataag aggggaaaga aagtgacatt 6300

tgaccagctg gagaggaaga taaggagact tgatctatct gtcgggctca gtgatgtgct 6360

cggaccttct gtgcttgtga aggcgagagg tgcacggact aggctgctgg cacctttctt 6420

ctctaacagc gggacagcct gctaccctat agcaaatgcc tctcctcagg tagctaagat 6480

actctggagt cagaccgcgt gcctgcggag tgtaaaaatc attattcaag cgggcaccca 6540

acgcgctgtc gcagtgactg ctgaccatga ggttacctct actaagatag agaagaggca 6600

taccatcgct aaatacaatc ccttcaagaa ataggccgta tccctgagac tgcaattcac 6660

ccgccttccc aaaacaccat gacaccagat aatgatctgt cttgattact tacagttagt 6720

ttccctgtct atcaaattag aaaaaacacg ggtagaagag ttcggatccc ggccggcgca 6780

ccaaaagcgc aagatgggcc ccagatcttc taccaggatc ccagtacctc taatgctgac 6840

catacggatc acgctggcac tgagttatgt ccgtctgaca agttctcttg atggcaggcc 6900

tcttgcagcc gcagggatcg tggtaacagg ggataaagca gttaacatat acacctcatc 6960

ccagacaggg tcaatcatag tcaagttact cccaaatatg cccaaggaca aagaggcatg 7020

tgcaaaagcc ccattggagg cttacaacag gacactgact actttgctta ccccccttgg 7080

tgattctatc cgcaggatac aagagtctgt gactacatcc ggaggaagga gacagagacg 7140

ctctataggt gccattattg gcagtgtagc tctaggggtt gcaacagctg cacagataac 7200

ggcagcctcg gctctgatac aagccaacca gaatgctgct aacatcctcc ggcttaagga 7260

gagcattgct gcaaccaatg aagctgtaca cgaggtcact ggcggattgt cacagttagc 7320

agtggcagtt ggaaagatgc agcaatttgt taatgaccag cttaataaca cagctcaaga 7380

gttggactgt ataaaaatta cacagcaggt tggtgtagaa ctcaacttat acttaactga 7440

attgactaca gtgttcgggc cacaaatcac ttcccctgcc ttaactcagt tgactgttca 7500

ggctctttac aatctggctg gtggtaatgt agattacttg ttgactaagt taggtgtagg 7560

gaacaaccag ctcagctcat tgattggtag cggcttgatc accggtaacc ctatttttta 7620

cgactcacag actcaactct tgggcataca ggtgacttta ccctcagtcg ggaacctaaa 7680

taatatgcgt gccacctact tggagacctt gtctgtaagc acaacaaagg gatttgcctc 7740

agcacttgtc ccaaaagtag tgacacaggt cggttctgtg atagaagagc ttgacacctc 7800

atactgtata gaagctgatt tggatttata ttgtacaaga atagtgacat tccctatgtc 7860

ccctggtatt tattcctgtt tgagcggcaa tacatcggct tgcatgtatt caaggactga 7920

aggcgcactt actacaccat acatgactct caaaggctca gttgttgcca attgccagat 7980

gacaacatgt agatgtgcag accccccggg tatcatatca caaaattatg gagaggctgt 8040

gtctctaata gataagcact catgcaatgt cgtatcctta gacgggataa ctttgaggct 8100

cagtggggaa tttgatgcaa cttatcaaaa gaatatctca atattagatt ctcaagtact 8160

agtgacaggc aatctcgata tctcaactga gcttgggaat gtcaaccact caataagtaa 8220

tgctttggat aagttagagg aaagcaacag caaactagac aaagtcaatg tcaaactgac 8280

cagcacatct gctctcatta cctatattgt tttaactgtc atatctcttg ttcttggtat 8340

gcttagcctg gttctagcat gctatctgat gtacaagcaa aaagcgcaac gaaagacctt 8400

gttgtggctt ggaaataata ccctagatca gatgagagct actacaaaaa tgtgaatgca 8460

gatgagaggc agaggtatcc ccaatagcaa tctgtgtgtc aattctggca gcctgttaat 8520

cagaagaatt aagaaaaaac taccggatgt aggtgaacaa aagggaatat acgggtagaa 8580

cggcctgaga ggccacccct caatcgggag ccaggcccca ctacgtccgc tctaccgcat 8640

caccaacagc agtcttcagt catggacagc gcagttagcc aagttgcgct agagaatgat 8700

agaagagaag cgaataatac atggcgcttg gttttccgga tcgcagcctt acttttactg 8760

gtaataacct tagccgtctc tgcagtcgcc ctggcatata gtatggaggc tagcacacct 8820

ggcgaccttg taagcatacc gactgcgatc tctagggcag aggaaaggat tacatctgca 8880

ctcggttcca atcaagatgt agtagatagg atatacaagc aagtggccct tgagtctcca 8940

ttggcactgc taaataccga atctataatt atgaatgcaa taacgtctct ctcttatcaa 9000

atcaatggag ccacaaataa tagcgggtgt ggggcacctg ttcatgaccc agattacatc 9060

ggggggatag gtcaagaact tattgtagat gatacgagtg acgtcacatc attttatccc 9120

tctgcattcc aagaacacct gaattttatc ccggcgccca ctacaggatc aggctgcact 9180

cggataccct cattcgacat gagtgctacc cattactgtt acactcacaa tgtgatattg 9240

tctggctgca gagatcactc acactcacat cagtatttag cacttggtgt gcttcggaca 9300

tctgcaacag ggagggtatt cttttctact ctgcgttcca tcaacttgga tgacgcccaa 9360

aatcggaagt cttgcagtgt gagtgcaact cctttaggtt gtgatatgct gtgctctaaa 9420

atcacagaga ctgaggaaga ggattataaa tcagttatcc ccacatcgat ggtacatgga 9480

aggttagggc ttgacggcca ataccatgag aaggacctag acgtcacaac actatttagg 9540

gactgggtgg caaattaccc aggagtagga ggtgggtctt ttattaacaa tcgcgtatgg 9600

ttcccggtct acggagggct aaaacccagc tcgcctagtg acactgcaca agaagggaga 9660

tacgtaatat acaagcgata caatgacaca tgcccggatg agcaagatta ccagattcgg 9720

atggctaagt catcgtataa gcctaggcgg tttggtggaa aacgcgtaca gcaggccatc 9780

ctatccatca aggtgtcaac atccttgggt gaggacccgg tgttgactgt accgcctaat 9840

acggtcgcac tcatgggggc cgaaggcaga gttctcacag tggggacatc tcatttctta 9900

tatcagcgag ggtcatcata cttctctccc gctttgttat accctatgac agtcaacaac 10020

aaaacagcca ctcttcataa tccttataca ttcaatgctt tcactcggcc aggtagtgtc 10020

ccttgccagg cttcagcaag atgccccaac tcatgtgtta ccggagtcta tactgatcca 10080

tatcccttag tcttccatag gaaccacacc ttgcgagggg tattcggaac aatgcttgat 10140

gacgaacaag caagactcaa ccctgtatct gcagtatttg ataacatatc ccgcagtcgc 10200

ataactcggg taagttcaag cagtaccagg gcagcataca cgacatcaac gtgttttaaa 10260

gttgtcaaga ccaataaaac ctattgcctc agcattgcag aaatatccaa taccctcttc 10320

ggggaattca gaattgtccc cttactagtt gagattctca aggatggtgg ggtttaagaa 10380

gctaggaatg gtcggttgag tcaaccgtga gaaggcgggg aagatgatat tgcatcatct 10440

atcttctgta acaccaagaa tcaaatcgaa taccagtgcg agctcgaatc ctacgctgcc 10500

agtcagccat aatcggctgg tgctaatgtg gttagcctga atcttgtcga tagtcacttg 10560

attaagaaaa aatgtggacg gtagtgaaat ataaggcaaa gcagctcaca gaggacagca 10620

cgggtaggac atggcgggct ccggacccga aagggcagag catcagatta tcctaccaga 10680

gtcacatctg tcctcaccat tggtcaagca taaattgctt tattactgga aattaactgg 10740

gctaccgctt cctgacgaat gtgacttcga ccatcttatt atcagccgac aatggaagaa 10800

ggtacttgaa tcggccaccc ctgacattga gagaatgata aaactagggc gggcagtaca 10860

ccagactctc aaccacaatt ccaggataac cggagtactc catccccggt gtttagagga 10920

actggctagt attgaggttc ctgattcaac caacaaattt cggaagatcg aaaaaaagat 10980

ccagattcac aacacaaggt atggagaact gttcacaaga ttatgcacgc atgtagaaaa 11040

gaaactattg gggtcatctt ggtctaacaa tgtcccacgg tcagaggaat tcaacagcat 11100

ccgtacagat ccggcatttt ggtttcactc aaaatggtcc acagccaaat ttgcatggct 11160

ccatataaaa caggtccaga ggcatctgat tgtagcagca agaacaaggt ccgcagtcaa 11220

caaattagtg acgctgaccc ataaggtagg ccaaatcttt gttactcctg agcttgtcat 11280

tgtgacacat acagatgaga acaagttcac gtgtcttacc caggaacttg tgttgatgta 11340

tgcagatatg atggagggca gagatatggt cagcataata tcatccacgg cggcacatct 11400

taggagctta tcagagaaaa ttgatgatat tctgcggtta gtagatgctc tggcaagaga 11460

tttgggcaat caagtctacg atgttgtagc actaatggag ggattcgcat acggcgctgt 11520

tcagctgctt gaaccgtcag gtacatttgc gggggatttc ttcgcattca acctgcagga 11580

gctcaaagat actctaaccg gactcctccc caaggatatc gcagaatctg tgactcacgc 11640

aatcgcaacc atattctctg gcttagaaca aaatcaagca gctgagatgt tgtgcctgtt 11700

gcgtctgtgg ggtcacccac tactcgagtc ccgtattgca gcaaaagcag ttaggagtca 11760

aatgtgcgca ccaaaaatgg tagactttga tatgatcctc caggtattat ctttctttaa 11820

aggaacaatc atcaatggat atagaaagaa gaatgcaggc gtgtggccac gtgtcaagat 11880

agatacgata tacgggaagg tcatagggca gctacacgca gattctgcag agatttcaca 11940

tgatgtcatg ttgagggaat acaagagttt atctgcactt gaattcgagc catgtataga 12000

gtatgaccct gtcaccaatc tgagcatgtt tttaaaagac aaggcaatcg cacacccgaa 12060

agacaactgg ctcgcttcgt ttaggcgaaa ccttctctct gaggaccaga agaaacatgt 12120

aagagaggca acctcaacta accgcctctt gatagagttc ttagagtcaa atgattttga 12180

tccatataaa gagatggaat atctgacgac ccttgagtac ctaagagatg ataatgtggc 12240

agtatcatac tcactcaaag aaaaagaggt gaaagttaat gggcggattt tcgcaaagct 12300

aacaaataga ttaaggaatt gtcaggtaat ggcagaaggg atcctagctg accagattgc 12360

acctttcttc cagggaaatg gggtcattca ggatagcata tctttgacta aaagtatgct 12420

agcgatgagt caactgtctt tcaacagcaa caagaaacgt attactgact gcaaagaaag 12480

agtatcctca aaccgcaatc atgatccgaa gagcaagaat cgtcggagag ttgccacttt 12540

tgtaactacc gacctgcaaa agtattgtct caattggaga tatcagacag tcaagctgtt 12600

cgctcatgcc atcaatcagc tgatgggctt acctcacttc ttcgagtgga ttcatcttag 12660

actaatggat actacgatgt ttgtagggga ccctttcaat cctccaagtg accctacgga 12720

ctgtgatcta tcaagagtcc caaatgatga catatatatt gtcagtgcta ggggaggcat 12780

tgagggatta tgccagaagc tatggacaat gatctcaatt gctgcaatcc agcttgctgc 12840

agcaagatca cattgtcgcg ttgcctgtat ggtacaaggc gacaatcaag taatagctat 12900

aacgagagag gtaagatcag atgactcccc agagatggtg ttaacacaat tgcatcaagc 12960

cagtgataat ttcttcaagg aattgattca tgtcaatcat ttgatcggcc ataatttgaa 13020

ggatcgtgaa accatcaggt cagacacgtt cttcatatac agcaaacgaa tattcaaaga 13080

tggagcaata ctcagtcagg tcctcaaaaa ctcatctaaa ttagtgctaa tatcaggcga 13140

ccttagtgaa aacactgtaa tgtcttgtgc caacattgca tctactgtag cacggttatg 13200

cgagaacggg cttcctaagg atttctgtta ttacttaaac tacttaatga gttgcgtgca 13260

gacatacttc gattccgaat tttccatcac caacaactcg caactcgatt ctaaccagtc 13320

gtggatagag gacatttctt ttgtgcactc atatgtcctg acccctgctc aactgggggg 13380

actgagtaac cttcaatact caaggctcta cacaaggaac atcggcgacc cgggaaccac 13440

tgctttcgca gagatcaaga gattagaggc agtggggtta ctgagtccta gcattatgac 13500

taacatctta actaggccgc ctggaaatgg agattgggcc agtctgtgca acgatccata 13560

ctcctttaat tttgagactg tcgcaagccc aaatattgtc cttaagaaac atacacaaag 13620

agtcctattt gaaacttgct cgaatccctt attatctgga gtgcacacag aggataatga 13680

ggcagaagag aaggcattgg ctgaattctt gctcaatcaa gaaatgattc atccacgtgt 13740

cgcgcatgct atcatggaag caagctctgt aggtaggaga aagcaaattc aggggcttgt 13800

tgacacaaca aacaccgtga ttaagattgc actgactagg aggccactcg gcatcaagag 13860

gctgatgcgg atagtcaatt actcgagcat gcatgcaatg ctatttagag atgatgtttt 13920

ctcgtctaat agatccaacc atcccttagt ctcttctgat atgtgttctc agacactggc 13980

agactatgca cggaacagaa gctggtcacc tttgacaggg ggtagaaaaa tactgggtgt 14040

atctaatcct gataccatag aacttgtaga gggtgagatc cttagtgtca gtggagggtg 14100

cactagatgt gacagtgggg atgaacagtt tacttggttc catcttccaa gcaatataga 14160

gctaaacgat gacaccagca agaatcctcc aatgagagtg ccatatctcg ggtcaaagac 14220

tcaagagagg agagccgcct cgcttgcgaa aatagctcat atgtcaccac acgtgaaggc 14280

ggctctaagg gcgtcatctg tgttaatctg ggcttatggg gacaacgaaa taaactggac 14340

tgctgccctt aagattgcaa ggtctcggtg caacataagc tcagagtatc ttcgactatt 14400

gtcacccttg cctacagctg ggaatctcca acatagattg gacgacggca taactcagat 14460

gacattcacc cctgcatctc tatacagggt gtcaccttac attcatatat ctaatgattc 14520

tcaaaggcta tttactgaag aaggagtcaa agaggggaat gtggtttatc agcaaatcat 14580

gctcttgggt ttgtccttaa ttgagtcact cttcccaatg acaacaacca agacatatga 14640

tgaaatcaca ttgcacctcc acagtaaatt tagctgctgt atcagggaag cacctgttgc 14700

agttcctttc gagctactcg gggtggcacc ggaactaagg gcagtaacct caaataagtt 14760

tatgtatgat cctagccctg tatcagaggg agactttgcg agacttgact tagctatctt 14820

taagagttat gaacttaatt tagagtcata ttccacaata gagctaatga acgttctttc 14880

aatatctagt gggaagttga ttggccagtc tgtggtttct tatgatgaag atacctccat 14940

aaagaatgac gctataatag tgtatgacaa cacacggaat tggatcagcg aagctcagaa 15000

ttcagatgtg gtccgcctat tcgagtatgc ggcactcgaa gtgctcctcg actgttctta 15060

tcaactctac tatctgagag taagaggcct aaacaatatc gtcctgtaca tgagtgattt 15120

atacaagaat atgccaggaa ttctactctc taatattgca gccacaatat ctcaccctgt 15180

catccattca aggttgaatg cagtaggtct ggtcaaccat gacgggtcac accaacttgc 15240

agacacagat ttcattgaaa tgtctgcaaa gctgctagtc tcttgcactc gacgcgtggt 15300

ctcaggttta catgcaggga ataagtatga cctgctgttt ccatctgtct tggatgataa 15360

cctaagtgag aagatgcttc agttgatatc ccaattatgc tgtctgtata cggtgctctt 15420

tgctacaaca agagaaatcc cgaaaataag aggcttatct gcagaagaga aatgttcagt 15480

acttactgag tacctactgt cagatgctgt gaaaccgtta cttgggtccg agcaagtgag 15540

ctctatcatg tctcccaaca tagttacgtt cccagccaat ctgtattaca tgtctaggaa 15600

gagccttaat ttgatcaggg agagagagga cagggatact atcttggcat tgttgttccc 15660

tcaagaaccg ctgctcgagt ttcctctggt acgagatatt ggtgctcgtg taaaagatcc 15720

atttacccta caacctgcgg cgtttttaca agagttagat ttgagtgctc cggcaaggta 15780

tgacgcattc acaatcagtc aggcgcattc tgaacacata ttgccaaacc cagaggaaga 15840

tcacttagta cgatacttgt tcagaggaat agggactgcg tcctcctctt ggtataaggc 15900

atcccatctt ctttctgtac ccgaggtcag atgtgcaagg catgggaact ccttatattt 15960

agcagaagga agtggagcca tcatgagtct tctcgaattg catataccac acgaaactat 16020

ctattacaat acacttttct cgaacgagat gaacccccca cagcgacatt tcggaccaac 16080

tccaacacag tttctgaatt cggttgtttt taggaattta caggcggaag taccatgcaa 16140

ggatggattt gtccaggagt tccgtccgtt atggagagag aatacagaag aaagcgatct 16200

gacctcagat aaagcagtgg gatatatcac atctgtagtg ccgtacaggt ctgtatcatt 16260

gctgcattgt gacattgaaa tccctccagg atctaatcaa agcttactag atcagctggc 16320

taccaatctg tctctgattg ccatgcattc tgtgaaggag ggcggggtcg tgattatcaa 16380

agtactgtat gcaatgggat attacttcca tctactcatg aacttgttta ctccatgttc 16440

cacgaaagga tatattctct ctaatggcta tgcctgtaga ggggatatgg agtgttacct 16500

gatatttgtc atgggctacc taggcgggcc tacatttgta catgaggtgg tgaggatggc 16560

aaaaacgcta gtacagcggc atggcacact tttgtctaag tcagatgaga ttacactgac 16620

taggttattt acttcacagc aacagcgtgt aacagacatc ctatccagcc ctttaccgag 16680

actaatgaag tacttgagag agaatattga tactgcactg attgaagccg ggggacagcc 16740

tgtccgtcca ttctgtgcag agagtttagt gagcacacta acagacatga ctcagacaac 16800

ccagatcatc gccagccaca ttgatacagt cattcgatct gtgatctata tggaagcaga 16860

gggtgatctt gctgacacag tattcttatt taccccttac aacctctcta tggacgggaa 16920

aaagagaaca tcacttaaac agtgcacaag acagatctta gaggtcacaa tactgggtct 16980

cagagtcaaa aatctcaata aagtaggtga tgtaatcagc ctagtactca gaggtatgat 17040

ttctctggag gaccttctcc cactgagaac ctacttgaag tgtagtacct gccctaagta 17100

tttgaaggct gtcctaggta ttaccaaact caaagaaatg ttcacagacg cctctttatt 17160

atacttgact cgtgctcaac aaaaattcta catgaaaact ataggcaatg cagtcaaggg 17220

atactatagt aactgtgact cttaaaggca accacatatc aataggccct ctttctagcc 17280

gatcgtattc ttgttgactt cattatacca tattagaaaa aaattgaatt ccgacccttt 17340

aagactcgta ttcggattca aataattatc tcagaaaaaa agtgcacgta gttgttcttg 17400

attatagtcc cgtcattcac caagtctttg tttggt 17436

<210> 5
<211> 16860
<212> DNA
<213> Newcastle disease virus

<220>
<223> Mut HN (F277L)/M(G165W)/F(F117S)/F(F190L)/L(V757I)/L(F1551S)/L(R1
700L)-IL-12

<400> 5
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat tttagatttt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatggg tcaccagcag ttggtcatct cttggttttc cctggttttt ctggcatctc 3240

ccctcgtggc catatgggaa ctgaagaaag atgtttatgt cgtagaattg gattggtatc 3300

cggatgcccc tggagaaatg gtggtcctca cctgtgacac ccctgaagaa gatggtatca 3360

cctggacctt ggaccagagc agtgaggtct taggctctgg caaaaccctg accatccaag 3420

tcaaagagtt tggagatgct ggccagtaca cctgtcacaa aggaggcgag gttctaagcc 3480

attcgctcct gctgcttcac aaaaaggaag atggaatttg gtccactgat attttaaagg 3540

accagaaaga acccaaaaat aagacctttc taagatgcga ggccaagaat tattctggac 3600

gtttcacctg ctggtggctg acgacaatca gtactgattt gacattcagt gtcaaaagca 3660

gcagaggctc ttctgacccc caaggggtga cgtgcggagc tgctacactc tctgcagaga 3720

gagtcagagg ggacaacaag gagtatgagt actcagtgga gtgccaggag gacagtgcct 3780

gcccagctgc tgaggagagt ctgcccattg aggtcatggt ggatgccgtt cacaagctca 3840

agtatgaaaa ctacaccagc agcttcttca tcagggacat catcaaacct gacccaccca 3900

agaacttgca gctgaagcca ttaaagaatt ctcggcaggt ggaggtcagc tgggagtacc 3960

ctgacacctg gagtactcca cattcctact tctccctgac attctgcgtt caggtccagg 4020

gcaagagcaa gagagaaaag aaagatagag tcttcacgga caagacctca gccacggtca 4080

tctgccgcaa aaatgccagc attagcgtgc gggcccagga ccgctactat agctcatctt 4140

ggagcgaatg ggcatctgtg ccctgcagtg ttcctggagt aggggtacct ggggtgggcg 4200

ccagaaacct ccccgtggcc actccagacc caggaatgtt cccatgcctt caccactccc 4260

aaaacctgct gagggccgtc agcaacatgc tccagaaggc cagacaaact ctagaatttt 4320

acccttgcac ttctgaagag attgatcatg aagatatcac aaaagataaa accagcacag 4380

tggaggcctg tttaccattg gaattaacca agaatgagag ttgcctaaat tccagagaga 4440

cctctttcat aactaatggg agttgcctgg cctccagaaa gacctctttt atgatggccc 4500

tgtgccttag tagtatttat gaagacttga agatgtacca ggtggagttc aagaccatga 4560

atgcaaagct gctgatggat cctaagaggc agatctttct agatcaaaac atgctggcag 4620

ttattgatga gctgatgcag gccctgaatt tcaacagtga gactgtgcca caaaaatcct 4680

cccttgaaga accggatttt tataaaacta aaatcaagct ctgcatactt cttcatgctt 4740

tcagaattcg ggcagtgact attgatagag tgatgagcta tctgaatgct tcctaatgag 4800

ctgaccgagc cctcctctat aaacccaaga ttcaaggctc caagcgataa ccttctcccg 4860

cctcttccat cccactgaat gatcgcgcag ctgcaattaa tctagcaaca ttaaagatta 4920

agaaaaaata cgggtagaat cggagtgcac cgattgtgcc aagatggact catctaggac 4980

aattgggtta tactttgatt ctgcccttcc ttctagcaac ctgttagcat tcccgattgt 5040

cttacgagat gtaggagatg gaaagaagca aattactccg caatatagga tccggcgtct 5100

tgactcgtgg acagacagta aagaagactc ggtattcatc actacctacg gattcatctt 5160

ccaggttggg aatgaagaag tcactgtcgg catgatcaat gataatccca agcgagagtt 5220

actctccgct gcgatgctct gcctaggaag cgtcccgaat gttggagatc ctgttgagct 5280

ggcaagggcc tgcctcacta tggtggtaac atgcaagaag agtgcaacta atactgagag 5340

aatggtcttc tcagtagtgc aggcaccccg ggtgctgcaa agctgtaggg tcgtggcaga 5400

caaatactcg tcagtgaatg cagttaagca cgtgaaagca ccagagaaga tcccttggag 5460

tgaaacccta gagtacaagg tgaattttgt ctctttgact gtggtgccga agaaggatgt 5520

ctacaagatc ccaaccgcag tattgaaggt atccggctcg agcctgtata atcttgcgct 5580

caatgtcact attgatgtgg aggtagaccc aaagagcccg ttagtcaagt ccctttctag 5640

gtccgacagt ggatactatg ctaatctctt cttacatatc ggacttatgt ccactgtaga 5700

taagagggga aagaaagtga catttgacca gctggagagg aagataagga gacttgatct 5760

atctgtcggg ctcagtgatg tgctcggacc ttctgtgctt gtgaaggcga gaggtgcacg 5820

gactaggctg ctggcacctt tcttctctaa cagcgggaca gcctgctacc ctatagcaaa 5880

tgcctctcct caggtagcta agatactctg gagtcagacc gcgtgcctgc ggagtgtaaa 5940

aatcattatt caagcgggca cccaacgcgc tgtcgcagtg actgctgacc atgaggttac 6000

ctctactaag atagagaaga ggcataccat cgctaaatac aatcccttca agaaataggc 6060

cgtatccctg agactgcaat tcacccgcct tcccaaaaca ccatgacacc agataatgat 6120

ctgtcttgat tacttacagt tagtttccct gtctatcaaa ttagaaaaaa cacgggtaga 6180

agagttcgga tcccggccgg cgcaccaaaa gcgcaagatg ggccccagat cttctaccag 6240

gatcccagta cctctaatgc tgaccatacg gatcacgctg gcactgagtt atgtccgtct 6300

gacaagttct cttgatggca ggcctcttgc agccgcaggg atcgtggtaa caggggataa 6360

agcagttaac atatacacct catcccagac agggtcaatc atagtcaagt tactcccaaa 6420

tatgcccaag gacaaagagg catgtgcaaa agccccattg gaggcttaca acaggacact 6480

gactactttg cttacccccc ttggtgattc tatccgcagg atacaagagt ctgtgactac 6540

atccggagga aggagacaga gacgctctat aggtgccatt attggcagtg tagctctagg 6600

ggttgcaaca gctgcacaga taacggcagc ctcggctctg atacaagcca accagaatgc 6660

tgctaacatc ctccggctta aggagagcat tgctgcaacc aatgaagctg tacacgaggt 6720

cactggcgga ttgtcacagt tagcagtggc agttggaaag atgcagcaat ttgttaatga 6780

ccagcttaat aacacagctc aagagttgga ctgtataaaa attacacagc aggttggtgt 6840

agaactcaac ttatacttaa ctgaattgac tacagtgttc gggccacaaa tcacttcccc 6900

tgccttaact cagttgactg ttcaggctct ttacaatctg gctggtggta atgtagatta 6960

cttgttgact aagttaggtg tagggaacaa ccagctcagc tcattgattg gtagcggctt 7020

gatcaccggt aaccctattt tttacgactc acagactcaa ctcttgggca tacaggtgac 7080

tttaccctca gtcgggaacc taaataatat gcgtgccacc tacttggaga ccttgtctgt 7140

aagcacaaca aagggatttg cctcagcact tgtcccaaaa gtagtgacac aggtcggttc 7200

tgtgatagaa gagcttgaca cctcatactg tatagaagct gatttggatt tatattgtac 7260

aagaatagtg acattcccta tgtcccctgg tatttattcc tgtttgagcg gcaatacatc 7320

ggcttgcatg tattcaagga ctgaaggcgc acttactaca ccatacatga ctctcaaagg 7380

ctcagttgtt gccaattgcc agatgacaac atgtagatgt gcagaccccc cgggtatcat 7440

atcacaaaat tatggagagg ctgtgtctct aatagataag cactcatgca atgtcgtatc 7500

cttagacggg ataactttga ggctcagtgg ggaatttgat gcaacttatc aaaagaatat 7560

ctcaatatta gattctcaag tactagtgac aggcaatctc gatatctcaa ctgagcttgg 7620

gaatgtcaac cactcaataa gtaatgcttt ggataagtta gaggaaagca acagcaaact 7680

agacaaagtc aatgtcaaac tgaccagcac atctgctctc attacctata ttgttttaac 7740

tgtcatatct cttgttcttg gtatgcttag cctggttcta gcatgctatc tgatgtacaa 7800

gcaaaaagcg caacgaaaga ccttgttgtg gcttggaaat aataccctag atcagatgag 7860

agctactaca aaaatgtgaa tgcagatgag aggcagaggt atccccaata gcaatctgtg 7920

tgtcaattct ggcagcctgt taatcagaag aattaagaaa aaactaccgg atgtaggtga 7980

acaaaaggga atatacgggt agaacggcct gagaggccac ccctcaatcg ggagccaggc 8040

cccactacgt ccgctctacc gcatcaccaa cagcagtctt cagtcatgga cagcgcagtt 8100

agccaagttg cgctagagaa tgatagaaga gaagcgaata atacatggcg cttggttttc 8160

cggatcgcag ccttactttt actggtaata accttagccg tctctgcagt cgccctggca 8220

tatagtatgg aggctagcac acctggcgac cttgtaagca taccgactgc gatctctagg 8280

gcagaggaaa ggattacatc tgcactcggt tccaatcaag atgtagtaga taggatatac 8340

aagcaagtgg cccttgagtc tccattggca ctgctaaata ccgaatctat aattatgaat 8400

gcaataacgt ctctctctta tcaaatcaat ggagccacaa ataatagcgg gtgtggggca 8460

cctgttcatg acccagatta catcgggggg ataggtcaag aacttattgt agatgatacg 8520

agtgacgtca catcatttta tccctctgca ttccaagaac acctgaattt tatcccggcg 8580

cccactacag gatcaggctg cactcggata ccctcattcg acatgagtgc tacccattac 8640

tgttacactc acaatgtgat attgtctggc tgcagagatc actcacactc acatcagtat 8700

ttagcacttg gtgtgcttcg gacatctgca acagggaggg tattcttttc tactctgcgt 8760

tccatcaact tggatgacgc ccaaaatcgg aagtcttgca gtgtgagtgc aactccttta 8820

ggttgtgata tgctgtgctc taaaatcaca gagactgagg aagaggatta taaatcagtt 8880

atccccacat cgatggtaca tggaaggtta gggcttgacg gccaatacca tgagaaggac 8940

ctagacgtca caacactatt tagggactgg gtggcaaatt acccaggagt aggaggtggg 9000

tcttttatta acaatcgcgt atggttcccg gtctacggag ggctaaaacc cagctcgcct 9060

agtgacactg cacaagaagg gagatacgta atatacaagc gatacaatga cacatgcccg 9120

gatgagcaag attaccagat tcggatggct aagtcatcgt ataagcctag gcggtttggt 9180

ggaaaacgcg tacagcaggc catcctatcc atcaaggtgt caacatcctt gggtgaggac 9240

ccggtgttga ctgtaccgcc taatacggtc gcactcatgg gggccgaagg cagagttctc 9300

acagtgggga catctcattt cttatatcag cgagggtcat catacttctc tcccgctttg 9360

ttatacccta tgacagtcaa caacaaaaca gccactcttc ataatcctta tacattcaat 9420

gctttcactc ggccaggtag tgtcccttgc caggcttcag caagatgccc caactcatgt 9480

gttaccggag tctatactga tccatatccc ttagtcttcc ataggaacca caccttgcga 9540

ggggtattcg gaacaatgct tgatgacgaa caagcaagac tcaaccctgt atctgcagta 9600

tttgataaca tatcccgcag tcgcataact cgggtaagtt caagcagtac cagggcagca 9660

tacacgacat caacgtgttt taaagttgtc aagaccaata aaacctattg cctcagcatt 9720

gcagaaatat ccaataccct cttcggggaa ttcagaattg tccccttact agttgagatt 9780

ctcaaggatg gtggggttta agaagctagg aatggtcggt tgagtcaacc gtgagaaggc 9840

ggggaagatg atattgcatc atctatcttc tgtaacacca agaatcaaat cgaataccag 9900

tgcgagctcg aatcctacgc tgccagtcag ccataatcgg ctggtgctaa tgtggttagc 9960

ctgaatcttg tcgatagtca cttgattaag aaaaaatgtg gacggtagtg aaatataagg 10020

caaagcagct cacagaggac agcacgggta ggacatggcg ggctccggac ccgaaagggc 10080
agagcatcag attatcctac cagagtcaca tctgtcctca ccattggtca agcataaatt 10140
gctttattac tggaaattaa ctgggctacc gcttcctgac gaatgtgact tcgaccatct 10200
tattatcagc cgacaatgga agaaggtact tgaatcggcc acccctgaca ttgagagaat 10260
gataaaacta gggcgggcag tacaccagac tctcaaccac aattccagga taaccggagt 10320
actccatccc cggtgtttag aggaactggc tagtattgag gttcctgatt caaccaacaa 10380
atttcggaag atcgaaaaaa agatccagat tcacaacaca aggtatggag aactgttcac 10440
aagattatgc acgcatgtag aaaagaaact attggggtca tcttggtcta acaatgtccc 10500
acggtcagag gaattcaaca gcatccgtac agatccggca ttttggtttc actcaaaatg 10560
gtccacagcc aaatttgcat ggctccatat aaaacaggtc cagaggcatc tgattgtagc 10620
agcaagaaca aggtccgcag tcaacaaatt agtgacgctg acccataagg taggccaaat 10680
ctttgttact cctgagcttg tcattgtgac acatacagat gagaacaagt tcacgtgtct 10740
tacccaggaa cttgtgttga tgtatgcaga tatgatggag ggcagagata tggtcagcat 10800
aatatcatcc acggcggcac atcttaggag cttatcagag aaaattgatg atattctgcg 10860
gttagtagat gctctggcaa gagatttggg caatcaagtc tacgatgttg tagcactaat 10920
ggagggattc gcatacggcg ctgttcagct gcttgaaccg tcaggtacat ttgcggggga 10980
tttcttcgca ttcaacctgc aggagctcaa agatactcta accggactcc tccccaagga 11040
tatcgcagaa tctgtgactc acgcaatcgc aaccatattc tctggcttag aacaaaatca 11100
agcagctgag atgttgtgcc tgttgcgtct gtggggtcac ccactactcg agtcccgtat 11160
tgcagcaaaa gcagttagga gtcaaatgtg cgcaccaaaa atggtagact ttgatatgat 11220
cctccaggta ttatctttct ttaaaggaac aatcatcaat ggatatagaa agaagaatgc 11280
aggcgtgtgg ccacgtgtca agatagatac gatatacggg aaggtcatag ggcagctaca 11340
cgcagattct gcagagattt cacatgatgt catgttgagg gaatacaaga gtttatctgc 11400
acttgaattc gagccatgta tagagtatga ccctgtcacc aatctgagca tgtttttaaa 11460
agacaaggca atcgcacacc cgaaagacaa ctggctcgct tcgtttaggc gaaaccttct 11520
ctctgaggac cagaagaaac atgtaagaga ggcaacctca actaaccgcc tcttgataga 11580
gttcttagag tcaaatgatt ttgatccata taaagagatg gaatatctga cgacccttga 11640
gtacctaaga gatgataatg tggcagtatc atactcactc aaagaaaaag aggtgaaagt 11700
taatgggcgg attttcgcaa agctaacaaa tagattaagg aattgtcagg taatggcaga 11760
agggatccta gctgaccaga ttgcaccttt cttccaggga aatggggtca ttcaggatag 11820
catatctttg actaaaagta tgctagcgat gagtcaactg tctttcaaca gcaacaagaa 11880
acgtattact gactgcaaag aaagagtatc ctcaaaccgc aatcatgatc cgaagagcaa 11940

gaatcgtcgg agagttgcca cttttgtaac taccgacctg caaaagtatt gtctcaattg 12000

gagatatcag acagtcaagc tgttcgctca tgccatcaat cagctgatgg gcttacctca 12060

cttcttcgag tggattcatc ttagactaat ggatactacg atgtttgtag ggaccctttt 12120

caatcctcca agtgacccta cggactgtga tctatcaaga gtcccaaatg atgacatata 12180

tattgtcagt gctaggggag gcattgaggg attatgccag aagctatgga caatgatctc 12240

aattgctgca atccagcttg ctgcagcaag atcacattgt cgcgttgcct gtatggtaca 12300

aggcgacaat caagtaatag ctataacgag agaggtaaga tcagatgact ccccagagat 12360

ggtgttaaca caattgcatc aagccagtga taatttcttc aaggaattga ttcatgtcaa 12420

tcatttgatc ggccataatt tgaaggatcg tgaaaccatc aggtcagaca cgttcttcat 12480

atacagcaaa cgaatattca aagatggagc aatactcagt caggtcctca aaaactcatc 12540

taaattagtg ctaatatcag gcgaccttag tgaaaacact gtaatgtctt gtgccaacat 12600

tgcatctact gtagcacggt tatgcgagaa cgggcttcct aaggatttct gttattactt 12660

aaactactta atgagttgcg tgcagacata cttcgattcc gaattttcca tcaccaacaa 12720

ctcgcaactc gattctaacc agtcgtggat agaggacatt tcttttgtgc actcatatgt 12780

cctgacccct gctcaactgg ggggactgag taaccttcaa tactcaaggc tctacacaag 12840

gaacatcggc gacccgggaa ccactgcttt cgcagagatc aagagattag aggcagtggg 12900

gttactgagt cctagcatta tgactaacat cttaactagg ccgcctggaa atggagattg 12960

ggccagtctg tgcaacgatc catactcctt taattttgag actgtcgcaa gcccaaatat 13020

tgtccttaag aaacatacac aaagagtcct atttgaaact tgctcgaatc ccttattatc 13080

tggagtgcac acagaggata atgaggcaga agagaaggca ttggctgaat tcttgctcaa 13140

tcaagaaatg attcatccac gtgtcgcgca tgctatcatg gaagcaagct ctgtaggtag 13200

gagaaagcaa attcaggggc ttgttgacac aacaaacacc gtgattaaga ttgcactgac 13260

taggaggcca ctcggcatca agaggctgat gcggatagtc aattactcga gcatgcatgc 13320

aatgctattt agagatgatg ttttctcgtc taatagatcc aaccatccct tagtctcttc 13380

tgatatgtgt tctcagacac tggcagacta tgcacggaac agaagctggt cacctttgac 13440

agggggtaga aaaatactgg gtgtatctaa tcctgatacc atagaacttg tagagggtga 13500

gatccttagt gtcagtggag ggtgcactag atgtgacagt ggggatgaac agtttacttg 13560

gttccatctt ccaagcaata tagagctaaa cgatgacacc agcaagaatc ctccaatgag 13620

agtgccatat ctcgggtcaa agactcaaga gaggagagcc gcctcgcttg cgaaaatagc 13680

tcatatgtca ccacacgtga aggcggctct aagggcgtca tctgtgttaa tctgggctta 13740

tggggacaac gaaataaact ggactgctgc ccttaagatt gcaaggtctc ggtgcaacat 13800

aagctcagag tatcttcgac tattgtcacc cttgcctaca gctgggaatc tccaacatag 13860

attggacgac ggcataactc agatgacatt cacccctgca tctctataca gggtgtcacc 13920

ttacattcat atatctaatg attctcaaag gctatttact gaagaaggag tcaaagaggg 13980

gaatgtggtt tatcagcaaa tcatgctctt gggtttgtcc ttaattgagt cactcttccc 14040

aatgacaaca accaagacat atgatgaaat cacattgcac ctccacagta aatttagctg 14100

ctgtatcagg gaagcacctg ttgcagttcc tttcgagcta ctcggggtgg caccggaact 14160

aagggcagta acctcaaata agtttatgta tgatcctagc cctgtatcag agggagactt 14220

tgcgagactt gacttagcta tctttaagag ttatgaactt aatttagagt catattccac 14280

aatagagcta atgaacgttc tttcaatatc tagtgggaag ttgattggcc agtctgtggt 14340

ttcttatgat gaagatacct ccataaagaa tgacgctata atagtgtatg acaacacacg 14400

gaattggatc agcgaagctc agaattcaga tgtggtccgc ctattcgagt atgcggcact 14460

cgaagtgctc ctcgactgtt cttatcaact ctactatctg agagtaagag gcctaaacaa 14520

tatcgtcctg tacatgagtg atttatacaa gaatatgcca ggaattctac tctctaatat 14580

tgcagccaca atatctcacc ctgtcatcca ttcaaggttg aatgcagtag gtctggtcaa 14640

ccatgacggg tcacaccaac ttgcagacac agatttcatt gaaatgtctg caaagctgct 14700

agtctcttgc actcgacgcg tggtctcagg tttacatgca gggaataagt atgacctgct 14760

gtttccatct gtcttggatg ataacctaag tgagaagatg cttcagttga tatcccaatt 14820

atgctgtctg tatacggtgc tctttgctac aacaagagaa atcccgaaaa taagaggctt 14880

atctgcagaa gagaaatgtt cagtacttac tgagtaccta ctgtcagatg ctgtgaaacc 14940

gttacttggg tccgagcaag tgagctctat catgtctccc aacatagtta cgttcccagc 15000

caatctgtat tacatgtcta ggaagagcct taatttgatc agggagagag aggacaggga 15060

tactatcttg gcattgttgt tccctcaaga accgctgctc gagtttcctc tggtacgaga 15120

tattggtgct cgtgtaaaag atccatttac cctacaacct gcggcgtttt tacaagagtt 15180

agatttgagt gctccggcaa ggtatgacgc attcacaatc agtcaggcgc attctgaaca 15240

catattgcca aacccagagg aagatcactt agtacgatac ttgttcagag gaatagggac 15300

tgcgtcctcc tcttggtata aggcatccca tcttctttct gtacccgagg tcagatgtgc 15360

aaggcatggg aactccttat atttagcaga aggaagtgga gccatcatga gtcttctcga 15420

attgcatata ccacacgaaa ctatctatta caatacactt ttctcgaacg agatgaaccc 15480

cccacagcga catttcggac caactccaac acagtttctg aattcggttg tttttaggaa 15540

tttacaggcg gaagtaccat gcaaggatgg atttgtccag gagttccgtc cgttatggag 15600

agagaataca gaagaaagcg atctgacctc agataaagca gtgggatata tcacatctgt 15660

agtgccgtac aggtctgtat cattgctgca ttgtgacatt gaaatccctc caggatctaa 15720

tcaaagctta ctagatcagc tggctaccaa tctgtctctg attgccatgc attctgtgaa 15780

ggagggcggg gtcgtgatta tcaaagtact gtatgcaatg ggatattact tccatctact 15840

catgaacttg tttactccat gttccacgaa aggatatatt ctctctaatg gctatgcctg 15900

tagaggggat atggagtgtt acctgatatt tgtcatgggc tacctaggcg ggcctacatt 15960

tgtacatgag gtggtgagga tggcaaaaac gctagtacag cggcatggca cacttttgtc 16020

taagtcagat gagattacac tgactaggtt atttacttca cagcaacagc gtgtaacaga 16080

catcctatcc agccctttac cgagactaat gaagtacttg agagagaata ttgatactgc 16140

actgattgaa gccgggggac agcctgtccg tccattctgt gcagagagtt tagtgagcac 16200

actaacagac atgactcaga caacccagat catcgccagc cacattgata cagtcattcg 16260

atctgtgatc tatatggaag cagagggtga tcttgctgac acagtattct tatttacccc 16320

ttacaacctc tctatggacg ggaaaaagag aacatcactt aaacagtgca caagacagat 16380

cttagaggtc acaatactgg gtctcagagt caaaaatctc aataaagtag gtgatgtaat 16440

cagcctagta ctcagaggta tgatttctct ggaggacctt ctcccactga gaacctactt 16500

gaagtgtagt acctgcccta agtatttgaa ggctgtccta ggtattacca aactcaaaga 16560

aatgttcaca gacgcctctt tattatactt gactcgtgct caacaaaaat tctacatgaa 16620

aactataggc aatgcagtca agggatacta tagtaactgt gactcttaaa ggcaaccaca 16680

tatcaatagg ccctctttct agccgatcgt attcttgttg acttcattat accatattag 16740

aaaaaaattg aattccgacc ctttaagact cgtattcgga ttcaaataat tatctcagaa 16800

aaaaagtgca cgtagttgtt cttgattata gtcccgtcat tcaccaagtc tttgtttggt 16860

<210> 6
<211> 15906
<212> DNA
<213> Newcastle disease virus

<220>
<223> Mut HN (F277L)/M(G165W)/F(F117S)/F(F190L)/L(V757I)/L(F1551S)/L(R1
      700L)-NS1

<400> 6
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420
tgttcttgag atcgatggtt tcaccaacag cgtgcctcaa tttaacaaca ggagtggagt 480
gtctgaggag agagcacaga gattcttgat gatagcaggg tccctccctc gggcatgcag 540
caacggtacc ccgttcatca cagctggggt tgaagatgat gcaccagaag acatcactga 600
tactctggaa agaatcatct ctatccaggc tcaagtatgg gtcacggtag caaaggccat 660
gactgcatat gagacagcgg atgagtcgga aacaagaaga atcaataagt atatgcagca 720
aggcagagtc caaaagaagt acatcctcca ccccgtatgt aggagcgcaa ttcaactcac 780
gattagacaa tctctggcag tccgcatttt cttggttagc gagcttaaga gaggccgcaa 840
cacggcaggt ggaagttcca cttattatag cttggtaggg gacatagact catacatcag 900
gaacaccggg cttactgcat tcttcctgac actcaggtat ggaattaaca ccaagacatc 960
agcccttgca ctcagcagcc tcgcaggcga tatccaaaaa atgaagcagc tcatgcgtct 1020
atatcggatg aaaggagata atgcgccgta catgacactg ctcggtgaca gtgaccagat 1080
gagctttgca ccggctgagt atgcacaact ctactctttt gcaatgggta tggcatccgt 1140
cctagacaaa ggaactggca agtaccagtt cgccagagac tttatgagca catcattctg 1200
gagacttgga gtagagtatg ctcgggctca gggaagcagc atcaatgagg atatggctgc 1260
cgagctaaag ctgaccccag cggcaaggag gggcctggca gctgctgccc aacgagcgtc 1320
cgaggagacc ggcagcatgg acattcccac tcaacaagct ggggtcctca cagggctcag 1380
cgacggaggc cctcaagccc cacaaggtgg actgaacagg tcgcaagggc aaccggacgc 1440
cggagatggg gagacccaat ttttagattt gatgagagca gtggcaaata gtatgagaga 1500
agcgccaaac tctgtgcaga acaccactca gcaggagcct ccatctactc ctgggccatc 1560
tcaagacaac gacaccgact ggggatactg accgacaaca ctcagcctgc ctccatcgaa 1620
ttatctcgat tcttctgcct gtaacctaac ccctggtcca cagtctcaca cggccaaacc 1680
cgcaaacgag cccccccctc tctccccccc cctccagcca cacggctcca accgcccaaa 1740
acaacacggg cacaactcga ctcactcata atctacacag agccgaagat cttagaaaaa 1800
aatacgggta gaagagagac attcagagat caggacaaat catcagggtc tccgctctcc 1860
cctctaccca gcagaccaga gtgaagatgg ccaccttcac ggatgcagag atcgacgatc 1920
tatttgaaac cagtggaact gtcattgaca gcataattac ggcccaaggc aacccagtag 1980
agactgttgg aaggagcgca atcccacaag gcaaaactaa agcattgagt gcagcatggg 2040
agaaacatgg aagcacccaa ccgccggccg gccaagacac ccctgatcga atggacagat 2100
cagacaaaca accatcccca cccgaacagg caaccacaca caacagcccg ccagccacat 2160
ccaccgacca gcccccaccc caggccgcag gcgaagccgg tgacacacag ctcaagactg 2220
gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatgga tagcaatacc atgtcctcct ttcaggttga ctgtttcctg tggcatattc 3240

gcaagcgttt cgccgataat ggtctgggtg acgccccgtt cctggaccgt ctgcgtcgcg 3300

atcagaagag cctgaaaggc cgcggtaaca ccctgggcct ggacatcgaa accgcgaccc 3360

tggtgggcaa gcagatcgtg gagtggattc tgaaagagga aagcagcgaa accctgcgta 3420

tgaccattgc cagcgtgccg accagccgtt acctgagcga catgaccctg gaggaaatga 3480

gccgtgattg gttcatgctg atgccgcgcc agaagatcat tggtccactg tgcgtgcgtc 3540

tggaccaggc tatcatggag aagaacattg tgctgaaagc caacttcagc gtgatcttta 3600

accgtctgga aaccctgatt ctgctgcgcg cctttaccga ggaaggcgcg atcgtgggcg 3660

agattagccc gctgccgagc ctgccaggtc acacctatga agatgtgaaa aatgccgtgg 3720

gcgtgctgat tggcggtctg gagtggaacg gtaacaccgt gcgtgtgagc gaaaacattc 3780

agcgcttcgc atggcgtaac tgtgatgaga atggtcgtcc aagcctgcca ccggaacaaa 3840

aataagctga ccgagccctc ctctataaac ccaagattca aggctccaag cgataacctt 3900

ctcccgcctc ttccatccca ctgaatgatc gcgcagctgc aattaatcta gcaacattaa 3960

agattaagaa aaaatacggg tagaatcgga gtgcaccgat tgtgccaaga tggactcatc 4020

taggacaatt gggttatact ttgattctgc ccttccttct agcaacctgt tagcattccc 4080

gattgtctta cgagatgtag gagatggaaa gaagcaaatt actccgcaat ataggatccg 4140

gcgtcttgac tcgtggacag acagtaaaga agactcggta ttcatcacta cctacggatt 4200
catcttccag gttgggaatg aagaagtcac tgtcggcatg atcaatgata atcccaagcg 4260
agagttactc tccgctgcga tgctctgcct aggaagcgtc ccgaatgttg gagatcctgt 4320
tgagctggca agggcctgcc tcactatggt ggtaacatgc aagaagagtg caactaatac 4380
tgagagaatg gtcttctcag tagtgcaggc accccgggtg ctgcaaagct gtagggtcgt 4440
ggcagacaaa tactcgtcag tgaatgcagt taagcacgtg aaagcaccag agaagatccc 4500
ttggagtgaa accctagagt acaaggtgaa ttttgtctct ttgactgtgg tgccgaagaa 4560
ggatgtctac aagatcccaa ccgcagtatt gaaggtatcc ggctcgagcc tgtataatct 4620
tgcgctcaat gtcactattg atgtggaggt agacccaaag agcccgttag tcaagtccct 4680
ttctaggtcc gacagtggat actatgctaa tctcttctta catatcggac ttatgtccac 4740
tgtagataag aggggaaaga aagtgacatt tgaccagctg gagaggaaga taaggagact 4800
tgatctatct gtcgggctca gtgatgtgct cggaccttct gtgcttgtga aggcgagagg 4860
tgcacggact aggctgctgg cacctttctt ctctaacagc gggacagcct gctaccctat 4920
agcaaatgcc tctcctcagg tagctaagat actctggagt cagaccgcgt gcctgcggag 4980
tgtaaaaatc attattcaag cgggcaccca acgcgctgtc gcagtgactg ctgaccatga 5040
ggttacctct actaagatag agaagaggca taccatcgct aaatacaatc ccttcaagaa 5100
ataggccgta tccctgagac tgcaattcac ccgccttccc aaaacaccat gacaccagat 5160
aatgatctgt cttgattact tacagttagt ttccctgtct atcaaattag aaaaaacacg 5220
ggtagaagag ttcggatccc ggccggcgca ccaaaagcgc aagatgggcc ccagatcttc 5280
taccaggatc ccagtacctc taatgctgac catacggatc acgctggcac tgagttatgt 5340
ccgtctgaca agttctcttg atggcaggcc tcttgcagcc gcagggatcg tggtaacagg 5400
ggataaagca gttaacatat acacctcatc ccagacaggg tcaatcatag tcaagttact 5460
cccaaatatg cccaaggaca aagaggcatg tgcaaaagcc ccattggagg cttacaacag 5520
gacactgact actttgctta ccccccttgg tgattctatc cgcaggatac aagagtctgt 5580
gactacatcc ggaggaagga gacagagacg ctctataggt gccattattg gcagtgtagc 5640
tctaggggtt gcaacagctg cacagataac ggcagcctcg gctctgatac aagccaacca 5700
gaatgctgct aacatcctcc ggcttaagga gagcattgct gcaaccaatg aagctgtaca 5760
cgaggtcact ggcggattgt cacagttagc agtggcagtt ggaaagatgc agcaatttgt 5820
taatgaccag cttaataaca cagctcaaga gttggactgt ataaaaatta cacagcaggt 5880
tggtgtagaa ctcaacttat acttaactga attgactaca gtgttcgggc cacaaatcac 5940
ttcccctgcc ttaactcagt tgactgttca ggctctttac aatctggctg gtggtaatgt 6000
agattacttg ttgactaagt taggtgtagg gaacaaccag ctcagctcat tgattggtag 6060

cggcttgatc accggtaacc ctatttttta cgactcacag actcaactct tgggcataca 6120

ggtgacttta ccctcagtcg ggaacctaaa taatatgcgt gccacctact tggagacctt 6180

gtctgtaagc acaacaaagg gatttgcctc agcacttgtc ccaaaagtag tgacacaggt 6240

cggttctgtg atagaagagc ttgacacctc atactgtata gaagctgatt tggatttata 6300

ttgtacaaga atagtgacat tccctatgtc ccctggtatt tattcctgtt tgagcggcaa 6360

tacatcggct tgcatgtatt caaggactga aggcgcactt actacaccat acatgactct 6420

caaaggctca gttgttgcca attgccagat gacaacatgt agatgtgcag acccccgggg 6480

tatcatatca caaaattatg gagaggctgt gtctctaata gataagcact catgcaatgt 6540

cgtatcctta gacgggataa ctttgaggct cagtggggaa tttgatgcaa cttatcaaaa 6600

gaatatctca atattagatt ctcaagtact agtgacaggc aatctcgata tctcaactga 6660

gcttgggaat gtcaaccact caataagtaa tgctttggat aagttagagg aaagcaacag 6720

caaactagac aaagtcaatg tcaaactgac cagcacatct gctctcatta cctatattgt 6780

tttaactgtc atatctcttg ttcttggtat gcttagcctg gttctagcat gctatctgat 6840

gtacaagcaa aaagcgcaac gaaagacctt gttgtggctt ggaaataata ccctagatca 6900

gatgagagct actacaaaaa tgtgaatgca gatgagaggc agaggtatcc ccaatagcaa 6960

tctgtgtgtc aattctggca gcctgttaat cagaagaatt aagaaaaaac taccggatgt 7020

aggtgaacaa aagggaatat acgggtagaa cggcctgaga ggccacccct caatcgggag 7080

ccaggcccca ctacgtccgc tctaccgcat caccaacagc agtcttcagt catggacagc 7140

gcagttagcc aagttgcgct agagaatgat agaagagaag cgaataatac atggcgcttg 7200

gttttccgga tcgcagcctt acttttactg gtaataacct tagccgtctc tgcagtcgcc 7260

ctggcatata gtatggaggc tagcacacct ggcgaccttg taagcatacc gactgcgatc 7320

tctagggcag aggaaaggat tacatctgca ctcggttcca atcaagatgt agtagatagg 7380

atatacaagc aagtggccct tgagtctcca ttggcactgc taaataccga atctataatt 7440

atgaatgcaa taacgtctct ctcttatcaa atcaatggag ccacaaataa tagcgggtgt 7500

ggggcacctg ttcatgaccc agattacatc ggggggatag gtcaagaact tattgtagat 7560

gatacgagtg acgtcacatc attttatccc tctgcattcc aagaacacct gaattttatc 7620

ccggcgccca ctacaggatc aggctgcact cggataccct cattcgacat gagtgctacc 7680

cattactgtt acactcacaa tgtgatattg tctggctgca gagatcactc acactcacat 7740

cagtatttag cacttggtgt gcttcggaca tctgcaacag ggagggtatt cttttctact 7800

ctgcgttcca tcaacttgga tgacgcccaa aatcggaagt cttgcagtgt gagtgcaact 7860

cctttaggtt gtgatatgct gtgctctaaa atcacagaga ctgaggaaga ggattataaa 7920

tcagttatcc ccacatcgat ggtacatgga aggttagggc ttgacggcca ataccatgag 7980

aaggacctag acgtcacaac actatttagg gactgggtgg caaattaccc aggagtagga 8040

ggtgggtctt ttattaacaa tcgcgtatgg ttcccggtct acggagggct aaaacccagc 8100

tcgcctagtg acactgcaca agaagggaga tacgtaatat acaagcgata caatgacaca 8160

tgcccggatg agcaagatta ccagattcgg atggctaagt catcgtataa gcctaggcgg 8220

tttggtggaa aacgcgtaca gcaggccatc ctatccatca aggtgtcaac atccttgggt 8280

gaggacccgg tgttgactgt accgcctaat acggtcgcac tcatgggggc cgaaggcaga 8340

gttctcacag tggggacatc tcatttctta tatcagcgag ggtcatcata cttctctccc 8400

gctttgttat accctatgac agtcaacaac aaaacagcca ctcttcataa tccttataca 8460

ttcaatgctt tcactcggcc aggtagtgtc ccttgccagg cttcagcaag atgccccaac 8520

tcatgtgtta ccggagtcta tactgatcca tatcccttag tcttccatag gaaccacacc 8580

ttgcgagggg tattcggaac aatgcttgat gacgaacaag caagactcaa ccctgtatct 8640

gcagtatttg ataacatatc ccgcagtcgc ataactcggg taagttcaag cagtaccagg 8700

gcagcataca cgacatcaac gtgttttaaa gttgtcaaga ccaataaaac ctattgcctc 8760

agcattgcag aaatatccaa taccctcttc ggggaattca gaattgtccc cttactagtt 8820

gagattctca aggatggtgg ggtttaagaa gctaggaatg gtcggttgag tcaaccgtga 8880

gaaggcgggg aagatgatat tgcatcatct atcttctgta acaccaagaa tcaaatcgaa 8940

taccagtgcg agctcgaatc ctacgctgcc agtcagccat aatcggctgg tgctaatgtg 9000

gttagcctga atcttgtcga tagtcacttg attaagaaaa aatgtggacg gtagtgaaat 9060

ataaggcaaa gcagctcaca gaggacagca cgggtaggac atggcgggct ccggacccga 9120

aagggcagag catcagatta tcctaccaga gtcacatctg tcctcaccat tggtcaagca 9180

taaattgctt tattactgga aattaactgg gctaccgctt cctgacgaat gtgacttcga 9240

ccatcttatt atcagccgac aatggaagaa ggtacttgaa tcggccaccc ctgacattga 9300

gagaatgata aaactagggc gggcagtaca ccagactctc aaccacaatt ccaggataac 9360

cggagtactc catccccggt gtttagagga actggctagt attgaggttc ctgattcaac 9420

caacaaattt cggaagatcg aaaaaaagat ccagattcac aacacaaggt atggagaact 9480

gttcacaaga ttatgcacgc atgtagaaaa gaaactattg gggtcatctt ggtctaacaa 9540

tgtcccacgg tcagaggaat tcaacagcat ccgtacagat ccggcatttt ggtttcactc 9600

aaaatggtcc acagccaaat ttgcatggct ccatataaaa caggtccaga ggcatctgat 9660

tgtagcagca agaacaaggt ccgcagtcaa caaattagtg acgctgaccc ataaggtagg 9720

ccaaatcttt gttactcctg agcttgtcat tgtgacacat acagatgaga acaagttcac 9780

gtgtcttacc caggaacttg tgttgatgta tgcagatatg atggagggca gagatatggt 9840

cagcataata tcatccacgg cggcacatct taggagctta tcagagaaaa ttgatgatat 9900

tctgcggtta gtagatgctc tggcaagaga tttgggcaat caagtctacg atgttgtagc 9960

actaatggag ggattcgcat acggcgctgt tcagctgctt gaaccgtcag gtacatttgc 10020

gggggatttc ttcgcattca acctgcagga gctcaaagat actctaaccg gactcctccc 10080

caaggatatc gcagaatctg tgactcacgc aatcgcaacc atattctctg gcttagaaca 10140

aaatcaagca gctgagatgt tgtgcctgtt gcgtctgtgg ggtcacccac tactcgagtc 10200

ccgtattgca gcaaaagcag ttaggagtca aatgtgcgca ccaaaaatgg tagactttga 10260

tatgatcctc caggtattat ctttctttaa aggaacaatc atcaatggat atagaaagaa 10320

gaatgcaggc gtgtggccac gtgtcaagat agatacgata tacgggaagg tcatagggca 10380

gctacacgca gattctgcag agatttcaca tgatgtcatg ttgagggaat acaagagttt 10440

atctgcactt gaattcgagc catgtataga gtatgaccct gtcaccaatc tgagcatgtt 10500

tttaaaagac aaggcaatcg cacacccgaa agacaactgg ctcgcttcgt ttaggcgaaa 10560

ccttctctct gaggaccaga agaaacatgt aagagaggca acctcaacta accgcctctt 10620

gatagagttc ttagagtcaa atgattttga tccatataaa gagatggaat atctgacgac 10680

ccttgagtac ctaagagatg ataatgtggc agtatcatac tcactcaaag aaaaagaggt 10740

gaaagttaat gggcggattt tcgcaaagct aacaaataga ttaaggaatt gtcaggtaat 10800

ggcagaaggg atcctagctg accagattgc acctttcttc cagggaaatg gggtcattca 10860

ggatagcata tctttgacta aaagtatgct agcgatgagt caactgtctt tcaacagcaa 10920

caagaaacgt attactgact gcaaagaaag agtatcctca aaccgcaatc atgatccgaa 10980

gagcaagaat cgtcggagag ttgccacttt tgtaactacc gacctgcaaa agtattgtct 11040

caattggaga tatcagacag tcaagctgtt cgctcatgcc atcaatcagc tgatgggctt 11100

acctcacttc ttcgagtgga ttcatcttag actaatggat actacgatgt ttgtagggga 11160

ccctttcaat cctccaagtg accctacgga ctgtgatcta tcaagagtcc caaatgatga 11220

catatatatt gtcagtgcta ggggaggcat tgagggatta tgccagaagc tatggacaat 11280

gatctcaatt gctgcaatcc agcttgctgc agcaagatca cattgtcgcg ttgcctgtat 11340

ggtacaaggc gacaatcaag taatagctat aacgagagag gtaagatcag atgactcccc 11400

agagatggtg ttaacacaat tgcatcaagc cagtgataat ttcttcaagg aattgattca 11460

tgtcaatcat ttgatcggcc ataatttgaa ggatcgtgaa accatcaggt cagacacgtt 11520

cttcatatac agcaaacgaa tattcaaaga tggagcaata ctcagtcagg tcctcaaaaa 11580

ctcatctaaa ttagtgctaa tatcaggcga ccttagtgaa aacactgtaa tgtcttgtgc 11640

caacattgca tctactgtag cacggttatg cgagaacggg cttcctaagg atttctgtta 11700

ttacttaaac tacttaatga gttgcgtgca gacatacttc gattccgaat tttccatcac 11760

caacaactcg caactcgatt ctaaccagtc gtggatagag gacatttctt ttgtgcactc 11820

atatgtcctg acccctgctc aactgggggg actgagtaac cttcaatact caaggctcta 11880

cacaaggaac atcggcgacc cgggaaccac tgctttcgca gagatcaaga gattagaggc 11940

agtggggtta ctgagtccta gcattatgac taacatctta actaggccgc ctggaaatgg 12000

agattgggcc agtctgtgca acgatccata ctcctttaat tttgagactg tcgcaagccc 12060

aaatattgtc cttaagaaac atacacaaag agtcctattt gaaacttgct cgaatccctt 12120

attatctgga gtgcacacag aggataatga ggcagaagag aaggcattgg ctgaattctt 12180

gctcaatcaa gaaatgattc atccacgtgt cgcgcatgct atcatggaag caagctctgt 12240

aggtaggaga aagcaaattc aggggcttgt tgacacaaca aacaccgtga ttaagattgc 12300

actgactagg aggccactcg gcatcaagag gctgatgcgg atagtcaatt actcgagcat 12360

gcatgcaatg ctatttagag atgatgtttt ctcgtctaat agatccaacc atcccttagt 12420

ctcttctgat atgtgttctc agacactggc agactatgca cggaacagaa gctggtcacc 12480

tttgacaggg ggtagaaaaa tactgggtgt atctaatcct gataccatag aacttgtaga 12540

gggtgagatc cttagtgtca gtggagggtg cactagatgt gacagtgggg atgaacagtt 12600

tacttggttc catcttccaa gcaatataga gctaaacgat gacaccagca agaatcctcc 12660

aatgagagtg ccatatctcg ggtcaaagac tcaagagagg agagccgcct cgcttgcgaa 12720

aatagctcat atgtcaccac acgtgaaggc ggctctaagg gcgtcatctg tgttaatctg 12780

ggcttatggg gacaacgaaa taaactggac tgctgccctt aagattgcaa ggtctcggtg 12840

caacataagc tcagagtatc ttcgactatt gtcacccttg cctacagctg ggaatctcca 12900

acatagattg gacgacggca taactcagat gacattcacc cctgcatctc tatacagggt 12960

gtcaccttac attcatatat ctaatgattc tcaaaggcta tttactgaag aaggagtcaa 13020

agaggggaat gtggtttatc agcaaatcat gctcttgggt ttgtccttaa ttgagtcact 13080

cttcccaatg acaacaacca agacatatga tgaaatcaca ttgcacctcc acagtaaatt 13140

tagctgctgt atcagggaag cacctgttgc agttcctttc gagctactcg gggtggcacc 13200

ggaactaagg gcagtaacct caaataagtt tatgtatgat cctagccctg tatcagaggg 13260

agactttgcg agacttgact tagctatctt taagagttat gaacttaatt tagagtcata 13320

ttccacaata gagctaatga acgttctttc aatatctagt gggaagttga ttggccagtc 13380

tgtggtttct tatgatgaag atacctccat aaagaatgac gctataatag tgtatgacaa 13440

cacacggaat tggatcagcg aagctcagaa ttcagatgtg gtccgcctat tcgagtatgc 13500

ggcactcgaa gtgctcctcg actgttctta tcaactctac tatctgagag taagaggcct 13560

aaacaatatc gtcctgtaca tgagtgattt atacaagaat atgccaggaa ttctactctc 13620

taatattgca gccacaatat ctcaccctgt catccattca aggttgaatg cagtaggtct 13680

ggtcaaccat gacgggtcac accaacttgc agacacagat ttcattgaaa tgtctgcaaa 13740

gctgctagtc tcttgcactc gacgcgtggt ctcaggttta catgcaggga ataagtatga 13800

cctgctgttt ccatctgtct tggatgataa cctaagtgag aagatgcttc agttgatatc 13860

ccaattatgc tgtctgtata cggtgctctt tgctacaaca agagaaatcc cgaaaataag 13920

aggcttatct gcagaagaga aatgttcagt acttactgag tacctactgt cagatgctgt 13980

gaaaccgtta cttgggtccg agcaagtgag ctctatcatg tctcccaaca tagttacgtt 14040

cccagccaat ctgtattaca tgtctaggaa gagccttaat ttgatcaggg agagagagga 14100

cagggatact atcttggcat tgttgttccc tcaagaaccg ctgctcgagt ttcctctggt 14160

acgagatatt ggtgctcgtg taaaagatcc atttacccta caacctgcgg cgtttttaca 14220

agagttagat ttgagtgctc cggcaaggta tgacgcattc acaatcagtc aggcgcattc 14280

tgaacacata ttgccaaacc cagaggaaga tcacttagta cgatacttgt tcagaggaat 14340

agggactgcg tcctcctctt ggtataaggc atcccatctt ctttctgtac ccgaggtcag 14400

atgtgcaagg catgggaact ccttatattt agcagaagga agtggagcca tcatgagtct 14460

tctcgaattg catataccac acgaaactat ctattacaat acacttttct cgaacgagat 14520

gaacccccca cagcgacatt tcggaccaac tccaacacag tttctgaatt cggttgtttt 14580

taggaattta caggcggaag taccatgcaa ggatggattt gtccaggagt tccgtccgtt 14640

atggagagag aatacagaag aaagcgatct gacctcagat aaagcagtgg gatatatcac 14700

atctgtagtg ccgtacaggt ctgtatcatt gctgcattgt gacattgaaa tccctccagg 14760

atctaatcaa agcttactag atcagctggc taccaatctg tctctgattg ccatgcattc 14820

tgtgaaggag ggcggggtcg tgattatcaa agtactgtat gcaatgggat attacttcca 14880

tctactcatg aacttgttta ctccatgttc cacgaaagga tatattctct ctaatggcta 14940

tgcctgtaga ggggatatgg agtgttacct gatatttgtc atgggctacc taggcgggcc 15000

tacatttgta catgaggtgg tgaggatggc aaaaacgcta gtacagcggc atggcacact 15060

tttgtctaag tcagatgaga ttacactgac taggttattt acttcacagc aacagcgtgt 15120

aacagacatc ctatccagcc ctttaccgag actaatgaag tacttgagag agaatattga 15180

tactgcactg attgaagccg ggggacagcc tgtccgtcca ttctgtgcag agagtttagt 15240

gagcacacta acagacatga ctcagacaac ccagatcatc gccagccaca ttgatacagt 15300

cattcgatct gtgatctata tggaagcaga gggtgatctt gctgacacag tattcttatt 15360

taccccttac aacctctcta tggacgggaa aaagagaaca tcacttaaac agtgcacaag 15420

acagatctta gaggtcacaa tactgggtct cagagtcaaa aatctcaata aagtaggtga 15480

tgtaatcagc ctagtactca gaggtatgat ttctctggag gaccttctcc cactgagaac 15540

ctacttgaag tgtagtacct gccctaagta tttgaaggct gtcctaggta ttaccaaact 15600

caaagaaatg ttcacagacg cctctttatt atacttgact cgtgctcaac aaaaattcta 15660

catgaaaact ataggcaatg cagtcaaggg atactatagt aactgtgact cttaaaggca 15720

accacatatc aataggccct ctttctagcc gatcgtattc ttgttgactt cattatacca 15780

tattagaaaa aaattgaatt ccgacccttt aagactcgta ttcggattca aataattatc 15840

tcagaaaaaa agtgcacgta gttgttcttg attatagtcc cgtcattcac caagtctttg 15900

tttggt 15906

<210> 7
<211> 15612
<212> DNA
<213> Newcastle disease virus

<220>
<223> Mut HN (F277L)/M(G165W)/F(F117S)/F(F190L)/L(V757I)/L(F1551S)/L(R1
      700L)-Apoptin

<400> 7
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gcccccaccc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatgaa cgctctccaa gaagatactc cacccggacc atcaacggtg ttcaggccac 3240

caacaagttc acggccgttg gaaacccctc actgcagaga gatccggatt ggtatcgctg 3300

gaattacaat cactctatcg ctgtgtggct gcgcgaatgc tcgcgctccc acgctaagat 3360

ctgcaactgc ggacaattca gaaagcactg gtttcaagaa tgtgccggac ttgaggaccg 3420

atcaacccaa gcctccctcg aagaagcgat cctgcgaccc ctccgagtac agggtaagcg 3480

agctaaaaga aagcttgatt accactactc ccagccgacc ccgaaccgca aaaaggcgta 3540

taagactgta agctgaccga gccctcctct ataaacccaa gattcaaggc tccaagcgat 3600

aaccttctcc cgcctcttcc atcccactga atgatcgcgc agctgcaatt aatctagcaa 3660

cattaaagat taagaaaaaa tacgggtaga atcggagtgc accgattgtg ccaagatgga 3720

ctcatctagg acaattgggt tatactttga ttctgccctt ccttctagca acctgttagc 3780

attcccgatt gtcttacgag atgtaggaga tggaaagaag caaattactc cgcaatatag 3840

gatccggcgt cttgactcgt ggacagacag taaagaagac tcggtattca tcactaccta 3900

cggattcatc ttccaggttg ggaatgaaga agtcactgtc ggcatgatca atgataatcc 3960

caagcgagag ttactctccg ctgcgatgct ctgcctagga agcgtcccga atgttggaga 4020

tcctgttgag ctggcaaggg cctgcctcac tatggtggta acatgcaaga agagtgcaac 4080

taatactgag agaatggtct tctcagtagt gcaggcaccc cgggtgctgc aaagctgtag 4140

ggtcgtggca gacaaatact cgtcagtgaa tgcagttaag cacgtgaaag caccagagaa 4200

gatcccttgg agtgaaaccc tagagtacaa ggtgaatttt gtctctttga ctgtggtgcc 4260

gaagaaggat gtctacaaga tcccaaccgc agtattgaag gtatccggct cgagcctgta 4320

taatcttgcg ctcaatgtca ctattgatgt ggaggtagac ccaaagagcc cgttagtcaa 4380

gtccctttct aggtccgaca gtggatacta tgctaatctc ttcttacata tcggacttat 4440

gtccactgta gataagaggg gaaagaaagt gacatttgac cagctggaga ggaagataag 4500

gagacttgat ctatctgtcg ggctcagtga tgtgctcgga ccttctgtgc ttgtgaaggc 4560

gagaggtgca cggactaggc tgctggcacc tttcttctct aacagcggga cagcctgcta 4620

ccctatagca aatgcctctc ctcaggtagc taagatactc tggagtcaga ccgcgtgcct 4680

gcggagtgta aaaatcatta ttcaagcggg cacccaacgc gctgtcgcag tgactgctga 4740

ccatgaggtt acctctacta agatagagaa gaggcatacc atcgctaaat acaatccctt 4800

caagaaatag gccgtatccc tgagactgca attcacccgc cttcccaaaa caccatgaca 4860

ccagataatg atctgtcttg attacttaca gttagtttcc ctgtctatca aattagaaaa 4920

aacacgggta gaagagttcg gatcccggcc ggcgcaccaa aagcgcaaga tgggccccag 4980

atcttctacc aggatcccag tacctctaat gctgaccata cggatcacgc tggcactgag 5040

ttatgtccgt ctgacaagtt ctcttgatgg caggcctctt gcagccgcag ggatcgtggt 5100

aacaggggat aaagcagtta acatatacac ctcatcccag acagggtcaa tcatagtcaa 5160

gttactccca aatatgccca aggacaaaga ggcatgtgca aaagccccat tggaggctta 5220

caacaggaca ctgactactt tgcttacccc ccttggtgat tctatccgca ggatacaaga 5280

gtctgtgact acatccggag gaaggagaca gagacgctct ataggtgcca ttattggcag 5340

tgtagctcta ggggttgcaa cagctgcaca gataacggca gcctcggctc tgatacaagc 5400

caaccagaat gctgctaaca tcctccggct taaggagagc attgctgcaa ccaatgaagc 5460

tgtacacgag gtcactggcg gattgtcaca gttagcagtg gcagttggaa agatgcagca 5520

atttgttaat gaccagctta ataacacagc tcaagagttg gactgtataa aaattacaca 5580

gcaggttggt gtagaactca acttatactt aactgaattg actacagtgt tcgggccaca 5640

aatcacttcc cctgccttaa ctcagttgac tgttcaggct ctttacaatc tggctggtgg 5700

taatgtagat tacttgttga ctaagttagg tgtagggaac aaccagctca gctcattgat 5760

tggtagcggc ttgatcaccg gtaaccctat tttttacgac tcacagactc aactcttggg 5820

catacaggtg actttaccct cagtcgggaa cctaaataat atgcgtgcca cctacttgga 5880

gaccttgtct gtaagcacaa caaagggatt tgcctcagca cttgtcccaa aagtagtgac 5940

acaggtcggt tctgtgatag aagagcttga cacctcatac tgtatagaag ctgatttgga 6000

tttatattgt acaagaatag tgacattccc tatgtcccct ggtatttatt cctgtttgag 6060

cggcaataca tcggcttgca tgtattcaag gactgaaggc gcacttacta caccatacat 6120

gactctcaaa ggctcagttg ttgccaattg ccagatgaca acatgtagat gtgcagaccc 6180

cccgggtatc atatcacaaa attatggaga ggctgtgtct ctaatagata agcactcatg 6240

caatgtcgta tccttagacg ggataacttt gaggctcagt ggggaatttg atgcaactta 6300

tcaaaagaat atctcaatat tagattctca agtactagtg acaggcaatc tcgatatctc 6360

aactgagctt gggaatgtca accactcaat aagtaatgct ttggataagt tagaggaaag 6420

caacagcaaa ctagacaaag tcaatgtcaa actgaccagc acatctgctc tcattaccta 6480

tattgtttta actgtcatat ctcttgttct tggtatgctt agcctggttc tagcatgcta 6540

tctgatgtac aagcaaaaag cgcaacgaaa gaccttgttg tggcttggaa ataataccct 6600

agatcagatg agagctacta caaaaatgtg aatgcagatg agaggcagag gtatccccaa 6660

tagcaatctg tgtgtcaatt ctggcagcct gttaatcaga agaattaaga aaaaactacc 6720

ggatgtaggt gaacaaaagg gaatatacgg gtagaacggc ctgagaggcc acccctcaat 6780

cgggagccag gccccactac gtccgctcta ccgcatcacc aacagcagtc ttcagtcatg 6840

gacagcgcag ttagccaagt tgcgctagag aatgatagaa gagaagcgaa taatacatgg 6900

cgcttggttt tccggatcgc agccttactt ttactggtaa taaccttagc cgtctctgca 6960

gtcgccctgg catatagtat ggaggctagc acacctggcg accttgtaag cataccgact 7020

gcgatctcta gggcagagga aaggattaca tctgcactcg gttccaatca agatgtagta 7080

gataggatat acaagcaagt ggcccttgag tctccattgg cactgctaaa taccgaatct 7140

ataattatga atgcaataac gtctctctct tatcaaatca atggagccac aaataatagc 7200

gggtgtgggg cacctgttca tgacccagat tacatcgggg ggataggtca agaacttatt 7260

gtagatgata cgagtgacgt cacatcattt tatccctctg cattccaaga acacctgaat 7320

tttatcccgg cgcccactac aggatcaggc tgcactcgga taccctcatt cgacatgagt 7380

gctacccatt actgttacac tcacaatgtg atattgtctg gctgcagaga tcactcacac 7440

tcacatcagt atttagcact tggtgtgctt cggacatctg caacagggag ggtattcttt 7500

tctactctgc gttccatcaa cttggatgac gcccaaaatc ggaagtcttg cagtgtgagt 7560

gcaactcctt taggttgtga tatgctgtgc tctaaaatca cagagactga ggaagaggat 7620

tataaatcag ttatccccac atcgatggta catggaaggt tagggcttga cggccaatac 7680

catgagaagg acctagacgt cacaacacta tttagggact gggtggcaaa ttacccagga 7740

gtaggaggtg ggtcttttat taacaatcgc gtatggttcc cggtctacgg agggctaaaa 7800

cccagctcgc ctagtgacac tgcacaagaa gggagatacg taatatacaa gcgatacaat 7860

gacacatgcc cggatgagca agattaccag attcggatgg ctaagtcatc gtataagcct 7920

aggcggtttg gtggaaaacg cgtacagcag gccatcctat ccatcaaggt gtcaacatcc 7980

ttgggtgagg acccggtgtt gactgtaccg cctaatacgg tcgcactcat gggggccgaa 8040

ggcagagttc tcacagtggg gacatctcat ttcttatatc agcgagggtc atcatacttc 8100

tctcccgctt tgttataccc tatgacagtc aacaacaaaa cagccactct tcataatcct 8160

tatacattca atgctttcac tcggccaggt agtgtccctt gccaggcttc agcaagatgc 8220

cccaactcat gtgttaccgg agtctatact gatccatatc ccttagtctt ccataggaac 8280

cacaccttgc gaggggtatt cggaacaatg cttgatgacg aacaagcaag actcaaccct 8340

gtatctgcag tatttgataa catatcccgc agtcgcataa ctcgggtaag ttcaagcagt 8400

accagggcag catacacgac atcaacgtgt tttaaagttg tcaagaccaa taaaacctat 8460

tgcctcagca ttgcagaaat atccaatacc ctcttcgggg aattcagaat tgtcccctta 8520

ctagttgaga ttctcaagga tggtggggtt taagaagcta ggaatggtcg gttgagtcaa 8580

ccgtgagaag gcggggaaga tgatattgca tcatctatct tctgtaacac caagaatcaa 8640

atcgaatacc agtgcgagct cgaatcctac gctgccagtc agccataatc ggctggtgct 8700

aatgtggtta gcctgaatct tgtcgatagt cacttgatta agaaaaaatg tggacggtag 8760

tgaaatataa ggcaaagcag ctcacagagg acagcacggg taggacatgg cgggctccgg 8820

acccgaaagg gcagagcatc agattatcct accagagtca catctgtcct caccattggt 8880

caagcataaa ttgctttatt actggaaatt aactgggcta ccgcttcctg acgaatgtga 8940

cttcgaccat cttattatca gccgacaatg gaagaaggta cttgaatcgg ccacccctga 9000

cattgagaga atgataaaac tagggcgggc agtacaccag actctcaacc acaattccag 9060

gataaccgga gtactccatc cccggtgttt agaggaactg gctagtattg aggttcctga 9120

ttcaaccaac aaatttcgga agatcgaaaa aaagatccag attcacaaca caaggtatgg 9180

agaactgttc acaagattat gcacgcatgt agaaaagaaa ctattggggt catcttggtc 9240

taacaatgtc ccacggtcag aggaattcaa cagcatccgt acagatccgg cattttggtt 9300

tcactcaaaa tggtccacag ccaaatttgc atggctccat ataaaacagg tccagaggca 9360

tctgattgta gcagcaagaa caaggtccgc agtcaacaaa ttagtgacgc tgacccataa 9420

ggtaggccaa atctttgtta ctcctgagct tgtcattgtg acacatacag atgagaacaa 9480

gttcacgtgt cttacccagg aacttgtgtt gatgtatgca gatatgatgg agggcagaga 9540

tatggtcagc ataatatcat ccacggcggc acatcttagg agcttatcag agaaaattga 9600

tgatattctg cggttagtag atgctctggc aagagatttg ggcaatcaag tctacgatgt 9660

tgtagcacta atggagggat tcgcatacgg cgctgttcag ctgcttgaac cgtcaggtac 9720

atttgcgggg gatttcttcg cattcaacct gcaggagctc aaagatactc taaccggact 9780

cctccccaag gatatcgcag aatctgtgac tcacgcaatc gcaaccatat tctctggctt 9840

agaacaaaat caagcagctg agatgttgtg cctgttgcgt ctgtggggtc acccactact 9900

cgagtcccgt attgcagcaa aagcagttag gagtcaaatg tgcgcaccaa aaatggtaga 9960

ctttgatatg atcctccagg tattatcttt ctttaaagga acaatcatca atggatatag 10020

aaagaagaat gcaggcgtgt ggccacgtgt caagatagat acgatatacg ggaaggtcat 10080

agggcagcta cacgcagatt ctgcagagat ttcacatgat gtcatgttga gggaatacaa 10140

gagtttatct gcacttgaat tcgagccatg tatagagtat gaccctgtca ccaatctgag 10200

catgttttta aaagacaagg caatcgcaca cccgaaagac aactggctcg cttcgtttag 10260

gcgaaacctt ctctctgagg accagaagaa acatgtaaga gaggcaacct caactaaccg 10320

cctcttgata gagttcttag agtcaaatga ttttgatcca tataaagaga tggaatatct 10380

gacgaccctt gagtacctaa gagatgataa tgtggcagta tcatactcac tcaaagaaaa 10440

agaggtgaaa gttaatgggc ggattttcgc aaagctaaca aatagattaa ggaattgtca 10500

ggtaatggca gaagggatcc tagctgacca gattgcacct ttcttccagg gaaatggggt 10560

cattcaggat agcatatctt tgactaaaag tatgctagcg atgagtcaac tgtctttcaa 10620

cagcaacaag aaacgtatta ctgactgcaa agaaagagta tcctcaaacc gcaatcatga 10680

tccgaagagc aagaatcgtc ggagagttgc cacttttgta actaccgacc tgcaaaagta 10740

ttgtctcaat tggagatatc agacagtcaa gctgttcgct catgccatca atcagctgat 10800

gggcttacct cacttcttcg agtggattca tcttagacta atggatacta cgatgtttgt 10860

aggggaccct ttcaatcctc caagtgaccc tacggactgt gatctatcaa gagtcccaaa 10920

tgatgacata tatattgtca gtgctagggg aggcattgag ggattatgcc agaagctatg 10980

gacaatgatc tcaattgctg caatccagct tgctgcagca agatcacatt gtcgcgttgc 11040

ctgtatggta caaggcgaca atcaagtaat agctataacg agagaggtaa gatcagatga 11100

ctccccagag atggtgttaa cacaattgca tcaagccagt gataatttct tcaaggaatt 11160

gattcatgtc aatcatttga tcggccataa tttgaaggat cgtgaaacca tcaggtcaga 11220

cacgttcttc atatacagca aacgaatatt caaagatgga gcaatactca gtcaggtcct 11280

caaaaactca tctaaattag tgctaatatc aggcgacctt agtgaaaaca ctgtaatgtc 11340

ttgtgccaac attgcatcta ctgtagcacg gttatgcgag aacgggcttc ctaaggattt 11400

ctgttattac ttaaactact taatgagttg cgtgcagaca tacttcgatt ccgaattttc 11460

catcaccaac aactcgcaac tcgattctaa ccagtcgtgg atagaggaca tttcttttgt 11520

gcactcatat gtcctgaccc ctgctcaact ggggggactg agtaaccttc aatactcaag 11580

gctctacaca aggaacatcg gcgacccggg aaccactgct ttcgcagaga tcaagagatt 11640

agaggcagtg gggttactga gtcctagcat tatgactaac atcttaacta ggccgcctgg 11700

aaatggagat tgggccagtc tgtgcaacga tccatactcc tttaattttg agactgtcgc 11760

aagcccaaat attgtcctta agaaacatac acaaagagtc ctatttgaaa cttgctcgaa 11820

tcccttatta tctggagtgc acacagagga taatgaggca gaagagaagg cattggctga 11880

attcttgctc aatcaagaaa tgattcatcc acgtgtcgcg catgctatca tggaagcaag 11940

ctctgtaggt aggagaaagc aaattcaggg gcttgttgac acaacaaaca ccgtgattaa 12000

gattgcactg actaggaggc cactcggcat caagaggctg atgcggatag tcaattactc 12060

gagcatgcat gcaatgctat ttagagatga tgttttctcg tctaatagat ccaaccatcc 12120

cttagtctct tctgatatgt gttctcagac actggcagac tatgcacgga acagaagctg 12180

gtcacctttg acagggggta gaaaaatact gggtgtatct aatcctgata ccatagaact 12240

tgtagagggt gagatcctta gtgtcagtgg agggtgcact agatgtgaca gtggggatga 12300

acagtttact tggttccatc ttccaagcaa tatagagcta aacgatgaca ccagcaagaa 12360

tcctccaatg agagtgccat atctcgggtc aaagactcaa gagaggagag ccgcctcgct 12420

tgcgaaaata gctcatatgt caccacacgt gaaggcggct ctaagggcgt catctgtgtt 12480

aatctgggct tatggggaca acgaaataaa ctggactgct gcccttaaga ttgcaaggtc 12540

tcggtgcaac ataagctcag agtatcttcg actattgtca cccttgccta cagctgggaa 12600

tctccaacat agattggacg acggcataac tcagatgaca ttcacccctg catctctata 12660

cagggtgtca ccttacattc atatatctaa tgattctcaa aggctattta ctgaagaagg 12720

agtcaaagag gggaatgtgg tttatcagca aatcatgctc ttgggtttgt ccttaattga 12780

gtcactcttc ccaatgacaa caaccaagac atatgatgaa atcacattgc acctccacag 12840

taaatttagc tgctgtatca gggaagcacc tgttgcagtt cctttcgagc tactcggggt 12900

ggcaccggaa ctaagggcag taacctcaaa taagtttatg tatgatccta gccctgtatc 12960

agagggagac tttgcgagac ttgacttagc tatctttaag agttatgaac ttaatttaga 13020

gtcatattcc acaatagagc taatgaacgt tctttcaata tctagtggga agttgattgg 13080

ccagtctgtg gtttcttatg atgaagatac ctccataaag aatgacgcta taatagtgta 13140

tgacaacaca cggaattgga tcagcgaagc tcagaattca gatgtggtcc gcctattcga 13200

gtatgcggca ctcgaagtgc tcctcgactg ttcttatcaa ctctactatc tgagagtaag 13260

aggcctaaac aatatcgtcc tgtacatgag tgatttatac aagaatatgc caggaattct 13320

actctctaat attgcagcca caatatctca ccctgtcatc cattcaaggt tgaatgcagt 13380

aggtctggtc aaccatgacg ggtcacacca acttgcagac acagatttca ttgaaatgtc 13440

tgcaaagctg ctagtctctt gcactcgacg cgtggtctca ggtttacatg cagggaataa 13500

gtatgacctg ctgtttccat ctgtcttgga tgataaccta agtgagaaga tgcttcagtt 13560

gatatcccaa ttatgctgtc tgtatacggt gctctttgct acaacaagag aaatcccgaa 13620

aataagaggc ttatctgcag aagagaaatg ttcagtactt actgagtacc tactgtcaga 13680

tgctgtgaaa ccgttacttg ggtccgagca agtgagctct atcatgtctc ccaacatagt 13740

tacgttccca gccaatctgt attacatgtc taggaagagc cttaatttga tcagggagag 13800

agaggacagg gatactatct tggcattgtt gttccctcaa gaaccgctgc tcgagtttcc 13860

tctggtacga gatattggtg ctcgtgtaaa agatccattt accctacaac ctgcggcgtt 13920

tttacaagag ttagatttga gtgctccggc aaggtatgac gcattcacaa tcagtcaggc 13980

gcattctgaa cacatattgc caaacccaga ggaagatcac ttagtacgat acttgttcag 14040

aggaataggg actgcgtcct cctcttggta taaggcatcc catcttcttt ctgtacccga 14100

ggtcagatgt gcaaggcatg ggaactcctt atatttagca gaaggaagtg gagccatcat 14160

gagtcttctc gaattgcata taccacacga aactatctat tacaatacac ttttctcgaa 14220

cgagatgaac cccccacagc gacatttcgg accaactcca acacagtttc tgaattcggt 14280

tgtttttagg aatttacagg cggaagtacc atgcaaggat ggatttgtcc aggagttccg 14340

tccgttatgg agagagaata cagaagaaag cgatctgacc tcagataaag cagtgggata 14400

tatcacatct gtagtgccgt acaggtctgt atcattgctg cattgtgaca ttgaaatccc 14460

tccaggatct aatcaaagct tactagatca gctggctacc aatctgtctc tgattgccat 14520

gcattctgtg aaggagggcg gggtcgtgat tatcaaagta ctgtatgcaa tgggatatta 14580

cttccatcta ctcatgaact tgtttactcc atgttccacg aaaggatata ttctctctaa 14640

tggctatgcc tgtagagggg atatggagtg ttacctgata tttgtcatgg gctacctagg 14700

cgggcctaca tttgtacatg aggtggtgag gatggcaaaa acgctagtac agcggcatgg 14760

cacacttttg tctaagtcag atgagattac actgactagg ttatttactt cacagcaaca 14820

gcgtgtaaca gacatcctat ccagcccttt accgagacta atgaagtact tgagagagaa 14880

tattgatact gcactgattg aagccggggg acagcctgtc cgtccattct gtgcagagag 14940

tttagtgagc acactaacag acatgactca gacaacccag atcatcgcca gccacattga 15000

tacagtcatt cgatctgtga tctatatgga agcagagggt gatcttgctg acacagtatt 15060

cttatttacc ccttacaacc tctctatgga cgggaaaaag agaacatcac ttaaacagtg 15120

cacaagacag atcttagagg tcacaatact gggtctcaga gtcaaaaatc tcaataaagt 15180

aggtgatgta atcagcctag tactcagagg tatgatttct ctggaggacc ttctcccact 15240

gagaacctac ttgaagtgta gtacctgccc taagtatttg aaggctgtcc taggtattac 15300

caaactcaaa gaaatgttca cagacgcctc tttattatac ttgactcgtg ctcaacaaaa 15360

attctacatg aaaactatag gcaatgcagt caagggatac tatagtaact gtgactctta 15420

aaggcaacca catatcaata ggccctcttt ctagccgatc gtattcttgt tgacttcatt 15480

ataccatatt agaaaaaaat tgaattccga ccctttaaga ctcgtattcg gattcaaata 15540

attatctcag aaaaaaagtg cacgtagttg ttcttgatta tagtcccgtc attcaccaag 15600

tctttgtttg gt 15612

<210> 8
<211> 16302
<212> DNA
<213> Newcastle disease virus

<220>
<223> Mut HN (F277L)/M(G165W)/F(F117S)/F(F190L)/L(V757I)/L(F1551S)/L(R1
700L)-B18R

<400> 8
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gcccccaccc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaat acaattaacg atctttatag 3180

ccaccatgac gatgaaaatg atggtacata tatatttcgt atcattattg ttattgctat 3240

tccacagtta cgccatagac atcgaaaatg aaatcacaga attcttcaat aaaatgagag 3300

atactctacc agctaaagac tctaaatggt tgaatccagc atgtatgttc ggaggcacaa 3360

tgaatgatat agccgctcta ggagagccat tcagcgcaaa gtgtcctcct attgaagaca 3420

gtcttttatc gcacagatat aaagactatg tggttaaatg ggaaaggcta gaaaaaaata 3480

gacggcgaca ggtttctaat aaacgtgtta aacatggtga tttatggata gccaactata 3540

catctaaatt cagtaaccgt aggtatttgt gcaccgtaac tacaaagaat ggtgactgtg 3600

ttcagggtat agttagatct catattagaa aacctccttc atgcattcca aaaacatatg 3660

aactaggtac tcatgataag tatggcatag acttatactg tggaattctt tacgcaaaac 3720

attataataa tataacttgg tataaagata ataaggaaat taatatcgac gacattaagt 3780

attcacaaac gggaaaggaa ttaattattc ataatccaga gttagaagat agcggaagat 3840

acgactgtta cgttcattac gacgacgtta gaatcaagaa tgatatcgta gtatcaagat 3900

gtaaaatact tacggttata ccgtcacaag accacaggtt taaactaata ctagatccaa 3960

aaatcaacgt aacgatagga gaacctgcca atataacatg cactgctgtg tcaacgtcat 4020

tattgattga cgatgtactg attgaatggg aaaatccatc cggatggctt ataggattcg 4080

attttgatgt atactctgtt ttaactagta gaggcggtat taccgaggcg accttgtact 4140

ttgaaaatgt tactgaagaa tatataggta atacatataa atgtcgtgga cacaactatt 4200

attttgaaaa aacccttaca actacagtag tattggagta agctgaccga gccctcctct 4260

ataaacccaa gattcaaggc tccaagcgat aaccttctcc cgcctcttcc atcccactga 4320

atgatcgcgc agctgcaatt aatctagcaa cattaaagat taagaaaaaa tacgggtaga 4380

atcggagtgc accgattgtg ccaagatgga ctcatctagg acaattgggt tatactttga 4440

ttctgccctt ccttctagca acctgttagc attcccgatt gtcttacgag atgtaggaga 4500

tggaaagaag caaattactc cgcaatatag gatccggcgt cttgactcgt ggacagacag 4560

taaagaagac tcggtattca tcactaccta cggattcatc ttccaggttg ggaatgaaga 4620

agtcactgtc ggcatgatca atgataatcc caagcgagag ttactctccg ctgcgatgct 4680

ctgcctagga agcgtcccga atgttggaga tcctgttgag ctggcaaggg cctgcctcac 4740

tatggtggta acatgcaaga agagtgcaac taatactgag agaatggtct tctcagtagt 4800

gcaggcaccc cgggtgctgc aaagctgtag ggtcgtggca gacaaatact cgtcagtgaa 4860

tgcagttaag cacgtgaaag caccagagaa gatcccttgg agtgaaaccc tagagtacaa 4920

ggtgaatttt gtctctttga ctgtggtgcc gaagaaggat gtctacaaga tcccaaccgc 4980

agtattgaag gtatccggct cgagcctgta taatcttgcg ctcaatgtca ctattgatgt 5040

ggaggtagac ccaaagagcc cgttagtcaa gtccctttct aggtccgaca gtggatacta 5100

tgctaatctc ttcttacata tcggacttat gtccactgta gataagaggg gaaagaaagt 5160

gacatttgac cagctggaga ggaagataag gagacttgat ctatctgtcg ggctcagtga 5220

tgtgctcgga ccttctgtgc ttgtgaaggc gagaggtgca cggactaggc tgctggcacc 5280

tttcttctct aacagcggga cagcctgcta ccctatagca aatgcctctc ctcaggtagc 5340

taagatactc tggagtcaga ccgcgtgcct gcggagtgta aaaatcatta ttcaagcggg 5400

cacccaacgc gctgtcgcag tgactgctga ccatgaggtt acctctacta agatagagaa 5460

gaggcatacc atcgctaaat acaatccctt caagaaatag gccgtatccc tgagactgca 5520

attcacccgc cttcccaaaa caccatgaca ccagataatg atctgtcttg attacttaca 5580

gttagtttcc ctgtctatca aattagaaaa aacacgggta gaagagttcg gatcccggcc 5640

ggcgcaccaa aagcgcaaga tgggccccag atcttctacc aggatcccag tacctctaat 5700

gctgaccata cggatcacgc tggcactgag ttatgtccgt ctgacaagtt ctcttgatgg 5760

caggcctctt gcagccgcag ggatcgtggt aacaggggat aaagcagtta acatatacac 5820

ctcatcccag acagggtcaa tcatagtcaa gttactccca aatatgccca aggacaaaga 5880

ggcatgtgca aaagccccat tggaggctta caacaggaca ctgactactt tgcttacccc 5940

ccttggtgat tctatccgca ggatacaaga gtctgtgact acatccggag gaaggagaca 6000

gagacgctct ataggtgcca ttattggcag tgtagctcta ggggttgcaa cagctgcaca 6060

gataacggca gcctcggctc tgatacaagc caaccagaat gctgctaaca tcctccggct 6120

taaggagagc attgctgcaa ccaatgaagc tgtacacgag gtcactggcg gattgtcaca 6180

gttagcagtg gcagttggaa agatgcagca atttgttaat gaccagctta ataacacagc 6240

tcaagagttg gactgtataa aaattacaca gcaggttggt gtagaactca acttatactt 6300

aactgaattg actacagtgt tcgggccaca aatcacttcc cctgccttaa ctcagttgac 6360

tgttcaggct ctttacaatc tggctggtgg taatgtagat tacttgttga ctaagttagg 6420

tgtagggaac aaccagctca gctcattgat tggtagcggc ttgatcaccg gtaaccctat 6480

tttttacgac tcacagactc aactcttggg catacaggtg actttaccct cagtcgggaa 6540

cctaaataat atgcgtgcca cctacttgga gaccttgtct gtaagcacaa caaagggatt 6600

tgcctcagca cttgtcccaa aagtagtgac acaggtcggt tctgtgatag aagagcttga 6660

cacctcatac tgtatagaag ctgatttgga tttatattgt acaagaatag tgacattccc 6720

tatgtcccct ggtatttatt cctgtttgag cggcaataca tcggcttgca tgtattcaag 6780

gactgaaggc gcacttacta caccatacat gactctcaaa ggctcagttg ttgccaattg 6840

ccagatgaca acatgtagat gtgcagaccc cccgggtatc atatcacaaa attatggaga 6900

ggctgtgtct ctaatagata agcactcatg caatgtcgta tccttagacg ggataacttt 6960

gaggctcagt ggggaatttg atgcaactta tcaaaagaat atctcaatat tagattctca 7020

agtactagtg acaggcaatc tcgatatctc aactgagctt gggaatgtca accactcaat 7080

aagtaatgct ttggataagt tagaggaaag caacagcaaa ctagacaaag tcaatgtcaa 7140

actgaccagc acatctgctc tcattaccta tattgtttta actgtcatat ctcttgttct 7200

tggtatgctt agcctggttc tagcatgcta tctgatgtac aagcaaaaag cgcaacgaaa 7260

gaccttgttg tggcttggaa ataataccct agatcagatg agagctacta caaaaatgtg 7320

aatgcagatg agaggcagag gtatccccaa tagcaatctg tgtgtcaatt ctggcagcct 7380

gttaatcaga agaattaaga aaaaactacc ggatgtaggt gaacaaaagg gaatatacgg 7440

gtagaacggc ctgagaggcc acccctcaat cgggagccag gccccactac gtccgctcta 7500

ccgcatcacc aacagcagtc ttcagtcatg gacagcgcag ttagccaagt tgcgctagag 7560

aatgatagaa gagaagcgaa taatacatgg cgcttggttt tccggatcgc agccttactt 7620

ttactggtaa taaccttagc cgtctctgca gtcgccctgg catatagtat ggaggctagc 7680

acacctggcg accttgtaag cataccgact gcgatctcta gggcagagga aaggattaca 7740

tctgcactcg gttccaatca agatgtagta gataggatat acaagcaagt ggcccttgag 7800

tctccattgg cactgctaaa taccgaatct ataattatga atgcaataac gtctctctct 7860

tatcaaatca atggagccac aaataatagc gggtgtgggg cacctgttca tgacccagat 7920

tacatcgggg ggataggtca agaacttatt gtagatgata cgagtgacgt cacatcattt 7980

tatccctctg cattccaaga acacctgaat tttatcccgg cgcccactac aggatcaggc 8040

tgcactcgga taccctcatt cgacatgagt gctacccatt actgttacac tcacaatgtg 8100

atattgtctg gctgcagaga tcactcacac tcacatcagt atttagcact tggtgtgctt 8160

cggacatctg caacagggag ggtattcttt tctactctgc gttccatcaa cttggatgac 8220

gcccaaaatc ggaagtcttg cagtgtgagt gcaactcctt taggttgtga tatgctgtgc 8280

tctaaaatca cagagactga ggaagaggat tataaatcag ttatccccac atcgatggta 8340

catggaaggt tagggcttga cggccaatac catgagaagg acctagacgt cacaacacta 8400

tttagggact gggtggcaaa ttacccagga gtaggaggtg ggtcttttat taacaatcgc 8460

gtatggttcc cggtctacgg agggctaaaa cccagctcgc ctagtgacac tgcacaagaa 8520

gggagatacg taatatacaa gcgatacaat gacacatgcc cggatgagca agattaccag 8580

attcggatgg ctaagtcatc gtataagcct aggcggtttg gtggaaaacg cgtacagcag 8640

gccatcctat ccatcaaggt gtcaacatcc ttgggtgagg acccggtgtt gactgtaccg 8700

cctaatacgg tcgcactcat gggggccgaa ggcagagttc tcacagtggg gacatctcat 8760

ttcttatatc agcgagggtc atcatacttc tctcccgctt tgttataccc tatgacagtc 8820

aacaacaaaa cagccactct tcataatcct tatacattca atgctttcac tcggccaggt 8880

agtgtccctt gccaggcttc agcaagatgc cccaactcat gtgttaccgg agtctatact 8940

gatccatatc ccttagtctt ccataggaac cacaccttgc gaggggtatt cggaacaatg 9000

cttgatgacg aacaagcaag actcaaccct gtatctgcag tatttgataa catatcccgc 9060

agtcgcataa ctcgggtaag ttcaagcagt accagggcag catacacgac atcaacgtgt 9120

tttaaagttg tcaagaccaa taaaacctat tgcctcagca ttgcagaaat atccaatacc 9180

ctcttcgggg aattcagaat tgtcccctta ctagttgaga ttctcaagga tggtggggtt 9240

taagaagcta ggaatggtcg gttgagtcaa ccgtgagaag gcggggaaga tgatattgca 9300

tcatctatct tctgtaacac caagaatcaa atcgaatacc agtgcgagct cgaatcctac 9360

gctgccagtc agccataatc ggctggtgct aatgtggtta gcctgaatct tgtcgatagt 9420

cacttgatta agaaaaaatg tggacggtag tgaaatataa ggcaaagcag ctcacagagg 9480

acagcacggg taggacatgg cgggctccgg acccgaaagg gcagagcatc agattatcct 9540

accagagtca catctgtcct caccattggt caagcataaa ttgctttatt actggaaatt 9600

aactgggcta ccgcttcctg acgaatgtga cttcgaccat cttattatca gccgacaatg 9660
gaagaaggta cttgaatcgg ccacccctga cattgagaga atgataaaac tagggcgggc 9720
agtacaccag actctcaacc acaattccag gataaccgga gtactccatc cccggtgttt 9780
agaggaactg gctagtattg aggttcctga ttcaaccaac aaatttcgga agatcgaaaa 9840
aaagatccag attcacaaca caaggtatgg agaactgttc acaagattat gcacgcatgt 9900
agaaaagaaa ctattggggt catcttggtc taacaatgtc ccacggtcag aggaattcaa 9960
cagcatccgt acagatccgg cattttggtt tcactcaaaa tggtccacag ccaaatttgc 10020
atggctccat ataaaacagg tccagaggca tctgattgta gcagcaagaa caaggtccgc 10080
agtcaacaaa ttagtgacgc tgacccataa ggtaggccaa atctttgtta ctcctgagct 10140
tgtcattgtg acacatacag atgagaacaa gttcacgtgt cttacccagg aacttgtgtt 10200
gatgtatgca gatatgatgg agggcagaga tatggtcagc ataatatcat ccacggcggc 10260
acatcttagg agcttatcag agaaaattga tgatattctg cggttagtag atgctctggc 10320
aagagatttg ggcaatcaag tctacgatgt tgtagcacta atggagggat tcgcatacgg 10380
cgctgttcag ctgcttgaac cgtcaggtac atttgcgggg gatttcttcg cattcaacct 10440
gcaggagctc aaagatactc taaccggact cctccccaag gatatcgcag aatctgtgac 10500
tcacgcaatc gcaaccatat tctctggctt agaacaaaat caagcagctg agatgttgtg 10560
cctgttgcgt ctgtggggtc acccactact cgagtcccgt attgcagcaa aagcagttag 10620
gagtcaaatg tgcgcaccaa aaatggtaga ctttgatatg atcctccagg tattatcttt 10680
ctttaaagga acaatcatca atggatatag aaagaagaat gcaggcgtgt ggccacgtgt 10740
caagatagat acgatatacg ggaaggtcat agggcagcta cacgcagatt ctgcagagat 10800
ttcacatgat gtcatgttga gggaatacaa gagtttatct gcacttgaat tcgagccatg 10860
tatagagtat gaccctgtca ccaatctgag catgttttta aaagacaagg caatcgcaca 10920
cccgaaagac aactggctcg cttcgtttag gcgaaacctt ctctctgagg accagaagaa 10980
acatgtaaga gaggcaacct caactaaccg cctcttgata gagttcttag agtcaaatga 11040
ttttgatcca tataaagaga tggaatatct gacgaccctt gagtacctaa gagatgataa 11100
tgtggcagta tcatactcac tcaaagaaaa agaggtgaaa gttaatgggc ggattttcgc 11160
aaagctaaca aatagattaa ggaattgtca ggtaatggca gaagggatcc tagctgacca 11220
gattgcacct ttcttccagg gaaatggggt cattcaggat agcatatctt tgactaaaag 11280
tatgctagcg atgagtcaac tgtctttcaa cagcaacaag aaacgtatta ctgactgcaa 11340
agaaagagta tcctcaaacc gcaatcatga tccgaagagc aagaatcgtc ggagagttgc 11400
cacttttgta actaccgacc tgcaaaagta ttgtctcaat tggagatatc agacagtcaa 11460
gctgttcgct catgccatca atcagctgat gggcttacct cacttcttcg agtggattca 11520

tcttagacta atggatacta cgatgtttgt aggggaccct ttcaatcctc caagtgaccc 11580

tacggactgt gatctatcaa gagtcccaaa tgatgacata tatattgtca gtgctagggg 11640

aggcattgag ggattatgcc agaagctatg gacaatgatc tcaattgctg caatccagct 11700

tgctgcagca agatcacatt gtcgcgttgc ctgtatggta caaggcgaca atcaagtaat 11760

agctataacg agagaggtaa gatcagatga ctccccagag atggtgttaa cacaattgca 11820

tcaagccagt gataatttct tcaaggaatt gattcatgtc aatcatttga tcggccataa 11880

tttgaaggat cgtgaaacca tcaggtcaga cacgttcttc atatacagca aacgaatatt 11940

caaagatgga gcaatactca gtcaggtcct caaaaactca tctaaattag tgctaatatc 12000

aggcgacctt agtgaaaaca ctgtaatgtc ttgtgccaac attgcatcta ctgtagcacg 12060

gttatgcgag aacgggcttc ctaaggattt ctgttattac ttaaactact taatgagttg 12120

cgtgcagaca tacttcgatt ccgaattttc catcaccaac aactcgcaac tcgattctaa 12180

ccagtcgtgg atagaggaca tttcttttgt gcactcatat gtcctgaccc ctgctcaact 12240

ggggggactg agtaaccttc aatactcaag gctctacaca aggaacatcg gcgacccggg 12300

aaccactgct ttcgcagaga tcaagagatt agaggcagtg gggttactga gtcctagcat 12360

tatgactaac atcttaacta ggccgcctgg aaatggagat tgggccagtc tgtgcaacga 12420

tccatactcc tttaattttg agactgtcgc aagcccaaat attgtcctta agaaacatac 12480

acaaagagtc ctatttgaaa cttgctcgaa tcccttatta tctggagtgc acacagagga 12540

taatgaggca gaagagaagg cattggctga attcttgctc aatcaagaaa tgattcatcc 12600

acgtgtcgcg catgctatca tggaagcaag ctctgtaggt aggagaaagc aaattcaggg 12660

gcttgttgac acaacaaaca ccgtgattaa gattgcactg actaggaggc cactcggcat 12720

caagaggctg atgcggatag tcaattactc gagcatgcat gcaatgctat ttagagatga 12780

tgttttctcg tctaatagat ccaaccatcc cttagtctct tctgatatgt gttctcagac 12840

actggcagac tatgcacgga acagaagctg gtcacctttg acagggggta gaaaaatact 12900

gggtgtatct aatcctgata ccatagaact tgtagagggt gagatcctta gtgtcagtgg 12960

agggtgcact agatgtgaca gtggggatga acagtttact tggttccatc ttccaagcaa 13020

tatagagcta aacgatgaca ccagcaagaa tcctccaatg agagtgccat atctcgggtc 13080

aaagactcaa gagaggagag ccgcctcgct tgcgaaaata gctcatatgt caccacacgt 13140

gaaggcggct ctaagggcgt catctgtgtt aatctgggct tatggggaca acgaaataaa 13200

ctggactgct gcccttaaga ttgcaaggtc tcggtgcaac ataagctcag agtatcttcg 13260

actattgtca cccttgccta cagctgggaa tctccaacat agattggacg acggcataac 13320

tcagatgaca ttcacccctg catctctata cagggtgtca ccttacattc atatatctaa 13380

tgattctcaa aggctattta ctgaagaagg agtcaaagag gggaatgtgg tttatcagca 13440

aatcatgctc ttgggtttgt ccttaattga gtcactcttc ccaatgacaa caaccaagac 13500

atatgatgaa atcacattgc acctccacag taaatttagc tgctgtatca gggaagcacc 13560

tgttgcagtt cctttcgagc tactcggggt ggcaccggaa ctaagggcag taacctcaaa 13620

taagtttatg tatgatccta gccctgtatc agagggagac tttgcgagac ttgacttagc 13680

tatctttaag agttatgaac ttaatttaga gtcatattcc acaatagagc taatgaacgt 13740

tctttcaata tctagtggga agttgattgg ccagtctgtg gtttcttatg atgaagatac 13800

ctccataaag aatgacgcta taatagtgta tgacaacaca cggaattgga tcagcgaagc 13860

tcagaattca gatgtggtcc gcctattcga gtatgcggca ctcgaagtgc tcctcgactg 13920

ttcttatcaa ctctactatc tgagagtaag aggcctaaac aatatcgtcc tgtacatgag 13980

tgatttatac aagaatatgc caggaattct actctctaat attgcagcca caatatctca 14040

ccctgtcatc cattcaaggt tgaatgcagt aggtctggtc aaccatgacg ggtcacacca 14100

acttgcagac acagatttca ttgaaatgtc tgcaaagctg ctagtctctt gcactcgacg 14160

cgtggtctca ggtttacatg cagggaataa gtatgacctg ctgtttccat ctgtcttgga 14220

tgataaccta agtgagaaga tgcttcagtt gatatcccaa ttatgctgtc tgtatacggt 14280

gctctttgct acaacaagag aaatcccgaa aataagaggc ttatctgcag aagagaaatg 14340

ttcagtactt actgagtacc tactgtcaga tgctgtgaaa ccgttacttg ggtccgagca 14400

agtgagctct atcatgtctc ccaacatagt tacgttccca gccaatctgt attacatgtc 14460

taggaagagc cttaatttga tcagggagag agaggacagg gatactatct tggcattgtt 14520

gttccctcaa gaaccgctgc tcgagtttcc tctggtacga gatattggtg ctcgtgtaaa 14580

agatccattt accctacaac ctgcggcgtt tttacaagag ttagatttga gtgctccggc 14640

aaggtatgac gcattcacaa tcagtcaggc gcattctgaa cacatattgc caaacccaga 14700

ggaagatcac ttagtacgat acttgttcag aggaataggg actgcgtcct cctcttggta 14760

taaggcatcc catcttcttt ctgtacccga ggtcagatgt gcaaggcatg ggaactcctt 14820

atatttagca gaaggaagtg gagccatcat gagtcttctc gaattgcata taccacacga 14880

aactatctat tacaatacac ttttctcgaa cgagatgaac cccccacagc gacatttcgg 14940

accaactcca acacagtttc tgaattcggt tgtttttagg aatttacagg cggaagtacc 15000

atgcaaggat ggatttgtcc aggagttccg tccgttatgg agagagaata cagaagaaag 15060

cgatctgacc tcagataaag cagtgggata tatcacatct gtagtgccgt acaggtctgt 15120

atcattgctg cattgtgaca ttgaaatccc tccaggatct aatcaaagct tactagatca 15180

gctggctacc aatctgtctc tgattgccat gcattctgtg aaggagggcg gggtcgtgat 15240

tatcaaagta ctgtatgcaa tgggatatta cttccatcta ctcatgaact tgtttactcc 15300

atgttccacg aaaggatata ttctctctaa tggctatgcc tgtagagggg atatggagtg 15360

ttacctgata tttgtcatgg gctacctagg cgggcctaca tttgtacatg aggtggtgag 15420

gatggcaaaa acgctagtac agcggcatgg cacacttttg tctaagtcag atgagattac 15480

actgactagg ttatttactt cacagcaaca gcgtgtaaca gacatcctat ccagcccttt 15540

accgagacta atgaagtact tgagagagaa tattgatact gcactgattg aagccggggg 15600

acagcctgtc cgtccattct gtgcagagag tttagtgagc acactaacag acatgactca 15660

gacaacccag atcatcgcca gccacattga tacagtcatt cgatctgtga tctatatgga 15720

agcagagggt gatcttgctg acacagtatt cttatttacc ccttacaacc tctctatgga 15780

cgggaaaaag agaacatcac ttaaacagtg cacaagacag atcttagagg tcacaatact 15840

gggtctcaga gtcaaaaatc tcaataaagt aggtgatgta atcagcctag tactcagagg 15900

tatgatttct ctggaggacc ttctcccact gagaacctac ttgaagtgta gtacctgccc 15960

taagtatttg aaggctgtcc taggtattac caaactcaaa gaaatgttca cagacgcctc 16020

tttattatac ttgactcgtg ctcaacaaaa attctacatg aaaactatag gcaatgcagt 16080

caagggatac tatagtaact gtgactctta aaggcaacca catatcaata ggccctcttt 16140

ctagccgatc gtattcttgt tgacttcatt ataccatatt agaaaaaaat tgaattccga 16200

ccctttaaga ctcgtattcg gattcaaata attatctcag aaaaaaagtg cacgtagttg 16260

ttcttgatta tagtcccgtc attcaccaag tctttgtttg gt 16302

<210> 9
<211> 571
<212> PRT
<213> Newcastle disease virus

<220>
<223> HN protein

<400> 9
Met Asp Ser Ala Val Ser Gln Val Ala Leu Glu Asn Asp Arg Arg Glu
1               5                   10                  15
Ala Asn Asn Thr Trp Arg Leu Val Phe Arg Ile Ala Ala Leu Leu Leu
            20                  25                  30
Leu Val Ile Thr Leu Ala Val Ser Ala Val Ala Leu Ala Tyr Ser Met
        35                  40                  45
Glu Ala Ser Thr Pro Gly Asp Leu Val Ser Ile Pro Thr Ala Ile Ser
    50                  55                  60
Arg Ala Glu Glu Arg Ile Thr Ser Ala Leu Gly Ser Asn Gln Asp Val
65                  70                  75                  80
Val Asp Arg Ile Tyr Lys Gln Val Ala Leu Glu Ser Pro Leu Ala Leu
                85                  90                  95
Leu Asn Thr Glu Ser Ile Ile Met Asn Ala Ile Thr Ser Leu Ser Tyr
            100                 105                 110
Gln Ile Asn Gly Ala Thr Asn Asn Ser Gly Cys Gly Ala Pro Val His
        115                 120                 125

Asp Pro Asp Tyr Ile Gly Gly Ile Gly Gln Glu Leu Ile Val Asp Asp
    130                 135                 140
Thr Ser Asp Val Thr Ser Phe Tyr Pro Ser Ala Phe Gln Glu His Leu
145                 150                 155                 160
Asn Phe Ile Pro Ala Pro Thr Thr Gly Ser Gly Cys Thr Arg Ile Pro
                165                 170                 175
Ser Phe Asp Met Ser Ala Thr His Tyr Cys Tyr Thr His Asn Val Ile
            180                 185                 190
Leu Ser Gly Cys Arg Asp His Ser His Ser His Gln Tyr Leu Ala Leu
        195                 200                 205
Gly Val Leu Arg Thr Ser Ala Thr Gly Arg Val Phe Phe Ser Thr Leu
    210                 215                 220
Arg Ser Ile Asn Leu Asp Asp Ala Gln Asn Arg Lys Ser Cys Ser Val
225                 230                 235                 240
Ser Ala Thr Pro Leu Gly Cys Asp Met Leu Cys Ser Lys Ile Thr Glu
                245                 250                 255
Thr Glu Glu Glu Asp Tyr Lys Ser Val Ile Pro Thr Ser Met Val His
            260                 265                 270
Gly Arg Leu Gly Leu Asp Gly Gln Tyr His Glu Lys Asp Leu Asp Val
        275                 280                 285
Thr Thr Leu Phe Arg Asp Trp Val Ala Asn Tyr Pro Gly Val Gly Gly
    290                 295                 300
Gly Ser Phe Ile Asn Asn Arg Val Trp Phe Pro Val Tyr Gly Gly Leu
305                 310                 315                 320
Lys Pro Ser Ser Pro Ser Asp Thr Ala Gln Glu Gly Arg Tyr Val Ile
                325                 330                 335
Tyr Lys Arg Tyr Asn Asp Thr Cys Pro Asp Glu Gln Asp Tyr Gln Ile
            340                 345                 350
Arg Met Ala Lys Ser Ser Tyr Lys Pro Arg Arg Phe Gly Gly Lys Arg
        355                 360                 365
Val Gln Gln Ala Ile Leu Ser Ile Lys Val Ser Thr Ser Leu Gly Glu
    370                 375                 380
Asp Pro Val Leu Thr Val Pro Pro Asn Thr Val Ala Leu Met Gly Ala
385                 390                 395                 400
Glu Gly Arg Val Leu Thr Val Gly Thr Ser His Phe Leu Tyr Gln Arg
                405                 410                 415
Gly Ser Ser Tyr Phe Ser Pro Ala Leu Leu Tyr Pro Met Thr Val Asn
            420                 425                 430
Asn Lys Thr Ala Thr Leu His Asn Pro Tyr Thr Phe Asn Ala Phe Thr
        435                 440                 445
Arg Pro Gly Ser Val Pro Cys Gln Ala Ser Ala Arg Cys Pro Asn Ser
    450                 455                 460
Cys Val Thr Gly Val Tyr Thr Asp Pro Tyr Pro Leu Val Phe His Arg
465                 470                 475                 480
Asn His Thr Leu Arg Gly Val Phe Gly Thr Met Leu Asp Asp Glu Gln
                485                 490                 495
Ala Arg Leu Asn Pro Val Ser Ala Val Phe Asp Asn Ile Ser Arg Ser
            500                 505                 510
Arg Ile Thr Arg Val Ser Ser Ser Ser Thr Arg Ala Ala Tyr Thr Thr
        515                 520                 525
Ser Thr Cys Phe Lys Val Val Lys Thr Asn Lys Thr Tyr Cys Leu Ser
    530                 535                 540
Ile Ala Glu Ile Ser Asn Thr Leu Phe Gly Glu Phe Arg Ile Val Pro
545                 550                 555                 560
Leu Leu Val Glu Ile Leu Lys Asp Gly Gly Val
                565                 570

<210> 10
<211> 364
<212> PRT
<213> Newcastle disease virus

<220>
<223> M protein

<400> 10
Met Asp Ser Ser Arg Thr Ile Gly Leu Tyr Phe Asp Ser Ala Leu Pro
1               5                   10                  15
Ser Ser Asn Leu Leu Ala Phe Pro Ile Val Leu Arg Asp Val Gly Asp
            20                  25                  30
Gly Lys Lys Gln Ile Thr Pro Gln Tyr Arg Ile Arg Arg Leu Asp Ser
        35                  40                  45
Trp Thr Asp Ser Lys Glu Asp Ser Val Phe Ile Thr Thr Tyr Gly Phe
    50                  55                  60
Ile Phe Gln Val Gly Asn Glu Glu Val Thr Val Gly Met Ile Asn Asp
65                  70                  75                  80
Asn Pro Lys Arg Glu Leu Leu Ser Ala Ala Met Leu Cys Leu Gly Ser
                85                  90                  95
Val Pro Asn Val Gly Asp Pro Val Glu Leu Ala Arg Ala Cys Leu Thr
            100                 105                 110
Met Val Val Thr Cys Lys Lys Ser Ala Thr Asn Thr Glu Arg Met Val
        115                 120                 125
Phe Ser Val Val Gln Ala Pro Arg Val Leu Gln Ser Cys Arg Val Val
    130                 135                 140
Ala Asp Lys Tyr Ser Ser Val Asn Ala Val Lys His Val Lys Ala Pro
145                 150                 155                 160
Glu Lys Ile Pro Trp Ser Glu Thr Leu Glu Tyr Lys Val Asn Phe Val
                165                 170                 175
Ser Leu Thr Val Val Pro Lys Lys Asp Val Tyr Lys Ile Pro Thr Ala
            180                 185                 190
Val Leu Lys Val Ser Gly Ser Ser Leu Tyr Asn Leu Ala Leu Asn Val
        195                 200                 205
Thr Ile Asp Val Glu Val Asp Pro Lys Ser Pro Leu Val Lys Ser Leu
    210                 215                 220
Ser Arg Ser Asp Ser Gly Tyr Tyr Ala Asn Leu Phe Leu His Ile Gly
225                 230                 235                 240
Leu Met Ser Thr Val Asp Lys Arg Gly Lys Lys Val Thr Phe Asp Gln
                245                 250                 255
Leu Glu Arg Lys Ile Arg Arg Leu Asp Leu Ser Val Gly Leu Ser Asp
            260                 265                 270
Val Leu Gly Pro Ser Val Leu Val Lys Ala Arg Gly Ala Arg Thr Arg
        275                 280                 285
Leu Leu Ala Pro Phe Phe Ser Asn Ser Gly Thr Ala Cys Tyr Pro Ile
    290                 295                 300
Ala Asn Ala Ser Pro Gln Val Ala Lys Ile Leu Trp Ser Gln Thr Ala
305                 310                 315                 320
Cys Leu Arg Ser Val Lys Ile Ile Ile Gln Ala Gly Thr Gln Arg Ala
                325                 330                 335
Val Ala Val Thr Ala Asp His Glu Val Thr Ser Thr Lys Ile Glu Lys
            340                 345                 350
Arg His Thr Ile Ala Lys Tyr Asn Pro Phe Lys Lys
        355                 360

<210> 11
<211> 553
<212> PRT
<213> Newcastle disease virus

<220>
<223> F protein

<400> 11
Met Gly Pro Arg Ser Ser Thr Arg Ile Pro Val Pro Leu Met Leu Thr
1               5                   10                  15
Ile Arg Ile Thr Leu Ala Leu Ser Tyr Val Arg Leu Thr Ser Ser Leu
            20                  25                  30
Asp Gly Arg Pro Leu Ala Ala Ala Gly Ile Val Val Thr Gly Asp Lys
        35                  40                  45
Ala Val Asn Ile Tyr Thr Ser Ser Gln Thr Gly Ser Ile Ile Val Lys
    50                  55                  60
Leu Leu Pro Asn Met Pro Lys Asp Lys Glu Ala Cys Ala Lys Ala Pro
65                  70                  75                  80
Leu Glu Ala Tyr Asn Arg Thr Leu Thr Thr Leu Leu Thr Pro Leu Gly
                85                  90                  95
Asp Ser Ile Arg Arg Ile Gln Glu Ser Val Thr Thr Ser Gly Gly Arg
            100                 105                 110
Arg Gln Arg Arg Ser Ile Gly Ala Ile Ile Gly Ser Val Ala Leu Gly
        115                 120                 125
Val Ala Thr Ala Ala Gln Ile Thr Ala Ala Ser Ala Leu Ile Gln Ala
    130                 135                 140
Asn Gln Asn Ala Ala Asn Ile Leu Arg Leu Lys Glu Ser Ile Ala Ala
145                 150                 155                 160
Thr Asn Glu Ala Val His Glu Val Thr Gly Gly Leu Ser Gln Leu Ala
                165                 170                 175
Val Ala Val Gly Lys Met Gln Gln Phe Val Asn Asp Gln Leu Asn Asn
            180                 185                 190
Thr Ala Gln Glu Leu Asp Cys Ile Lys Ile Thr Gln Gln Val Gly Val
        195                 200                 205
Glu Leu Asn Leu Tyr Leu Thr Glu Leu Thr Thr Val Phe Gly Pro Gln
    210                 215                 220
Ile Thr Ser Pro Ala Leu Thr Gln Leu Thr Val Gln Ala Leu Tyr Asn
225                 230                 235                 240
Leu Ala Gly Gly Asn Val Asp Tyr Leu Leu Thr Lys Leu Gly Val Gly
                245                 250                 255
Asn Asn Gln Leu Ser Ser Leu Ile Gly Ser Gly Leu Ile Thr Gly Asn
            260                 265                 270
Pro Ile Phe Tyr Asp Ser Gln Thr Gln Leu Leu Gly Ile Gln Val Thr
        275                 280                 285
Leu Pro Ser Val Gly Asn Leu Asn Asn Met Arg Ala Thr Tyr Leu Glu
    290                 295                 300
Thr Leu Ser Val Ser Thr Thr Lys Gly Phe Ala Ser Ala Leu Val Pro
305                 310                 315                 320
Lys Val Val Thr Gln Val Gly Ser Val Ile Glu Glu Leu Asp Thr Ser
                325                 330                 335
Tyr Cys Ile Glu Ala Asp Leu Asp Leu Tyr Cys Thr Arg Ile Val Thr
            340                 345                 350
Phe Pro Met Ser Pro Gly Ile Tyr Ser Cys Leu Ser Gly Asn Thr Ser
        355                 360                 365
Ala Cys Met Tyr Ser Arg Thr Glu Gly Ala Leu Thr Thr Pro Tyr Met
    370                 375                 380
Thr Leu Lys Gly Ser Val Val Ala Asn Cys Gln Met Thr Thr Cys Arg
385                 390                 395                 400
Cys Ala Asp Pro Pro Gly Ile Ile Ser Gln Asn Tyr Gly Glu Ala Val
                405                 410                 415
Ser Leu Ile Asp Lys His Ser Cys Asn Val Val Ser Leu Asp Gly Ile
            420                 425                 430
Thr Leu Arg Leu Ser Gly Glu Phe Asp Ala Thr Tyr Gln Lys Asn Ile
        435                 440                 445
Ser Ile Leu Asp Ser Gln Val Leu Val Thr Gly Asn Leu Asp Ile Ser
    450                 455                 460
Thr Glu Leu Gly Asn Val Asn His Ser Ile Ser Asn Ala Leu Asp Lys
465                 470                 475                 480
Leu Glu Glu Ser Asn Ser Lys Leu Asp Lys Val Asn Val Lys Leu Thr

485                 490                 495
Ser Thr Ser Ala Leu Ile Thr Tyr Ile Val Leu Thr Val Ile Ser Leu
            500                 505                 510
Val Leu Gly Met Leu Ser Leu Val Leu Ala Cys Tyr Leu Met Tyr Lys
        515                 520                 525
Gln Lys Ala Gln Arg Lys Thr Leu Leu Trp Leu Gly Asn Asn Thr Leu
    530                 535                 540
Asp Gln Met Arg Ala Thr Thr Lys Met
545                 550

<210> 12
<211> 2204
<212> PRT
<213> Newcastle disease virus

<220>
<223> L protein

<400> 12
Met Ala Gly Ser Gly Pro Glu Arg Ala Glu His Gln Ile Ile Leu Pro
1               5                   10                  15
Glu Ser His Leu Ser Ser Pro Leu Val Lys His Lys Leu Leu Tyr Tyr
            20                  25                  30
Trp Lys Leu Thr Gly Leu Pro Leu Pro Asp Glu Cys Asp Phe Asp His
        35                  40                  45
Leu Ile Ile Ser Arg Gln Trp Lys Lys Val Leu Glu Ser Ala Thr Pro
    50                  55                  60
Asp Ile Glu Arg Met Ile Lys Leu Gly Arg Ala Val His Gln Thr Leu
65                  70                  75                  80
Asn His Asn Ser Arg Ile Thr Gly Val Leu His Pro Arg Cys Leu Glu
                85                  90                  95
Glu Leu Ala Ser Ile Glu Val Pro Asp Ser Thr Asn Lys Phe Arg Lys
            100                 105                 110
Ile Glu Lys Lys Ile Gln Ile His Asn Thr Arg Tyr Gly Glu Leu Phe
        115                 120                 125
Thr Arg Leu Cys Thr His Val Glu Lys Lys Leu Leu Gly Ser Ser Trp
    130                 135                 140
Ser Asn Asn Val Pro Arg Ser Glu Glu Phe Asn Ser Ile Arg Thr Asp
145                 150                 155                 160
Pro Ala Phe Trp Phe His Ser Lys Trp Ser Thr Ala Lys Phe Ala Trp
                165                 170                 175
Leu His Ile Lys Gln Val Gln Arg His Leu Ile Val Ala Ala Arg Thr
            180                 185                 190
Arg Ser Ala Val Asn Lys Leu Val Thr Leu Thr His Lys Val Gly Gln
        195                 200                 205
Ile Phe Val Thr Pro Glu Leu Val Ile Val Thr His Thr Asp Glu Asn
    210                 215                 220
Lys Phe Thr Cys Leu Thr Gln Glu Leu Val Leu Met Tyr Ala Asp Met
225                 230                 235                 240
Met Glu Gly Arg Asp Met Val Ser Ile Ile Ser Ser Thr Ala Ala His
                245                 250                 255
Leu Arg Ser Leu Ser Glu Lys Ile Asp Asp Ile Leu Arg Leu Val Asp
            260                 265                 270
Ala Leu Ala Arg Asp Leu Gly Asn Gln Val Tyr Asp Val Val Ala Leu
        275                 280                 285
Met Glu Gly Phe Ala Tyr Gly Ala Val Gln Leu Leu Glu Pro Ser Gly
    290                 295                 300
Thr Phe Ala Gly Asp Phe Phe Ala Phe Asn Leu Gln Glu Leu Lys Asp
305                 310                 315                 320
Thr Leu Thr Gly Leu Leu Pro Lys Asp Ile Ala Glu Ser Val Thr His

                325                 330                 335
Ala Ile Ala Thr Ile Phe Ser Gly Leu Glu Gln Asn Gln Ala Ala Glu
            340                 345                 350
Met Leu Cys Leu Leu Arg Leu Trp Gly His Pro Leu Leu Glu Ser Arg
        355                 360                 365
Ile Ala Ala Lys Ala Val Arg Ser Gln Met Cys Ala Pro Lys Met Val
    370                 375                 380
Asp Phe Asp Met Ile Leu Gln Val Leu Ser Phe Phe Lys Gly Thr Ile
385                 390                 395                 400
Ile Asn Gly Tyr Arg Lys Lys Asn Ala Gly Val Trp Pro Arg Val Lys
                405                 410                 415
Ile Asp Thr Ile Tyr Gly Lys Val Ile Gly Gln Leu His Ala Asp Ser
            420                 425                 430
Ala Glu Ile Ser His Asp Val Met Leu Arg Glu Tyr Lys Ser Leu Ser
        435                 440                 445
Ala Leu Glu Phe Glu Pro Cys Ile Glu Tyr Asp Pro Val Thr Asn Leu
    450                 455                 460
Ser Met Phe Leu Lys Asp Lys Ala Ile Ala His Pro Lys Asp Asn Trp
465                 470                 475                 480
Leu Ala Ser Phe Arg Arg Asn Leu Leu Ser Glu Asp Gln Lys Lys His
                485                 490                 495
Val Arg Glu Ala Thr Ser Thr Asn Arg Leu Leu Ile Glu Phe Leu Glu
            500                 505                 510
Ser Asn Asp Phe Asp Pro Tyr Lys Glu Met Glu Tyr Leu Thr Thr Leu
        515                 520                 525
Glu Tyr Leu Arg Asp Asp Asn Val Ala Val Ser Tyr Ser Leu Lys Glu
    530                 535                 540
Lys Glu Val Lys Val Asn Gly Arg Ile Phe Ala Lys Leu Thr Asn Arg
545                 550                 555                 560
Leu Arg Asn Cys Gln Val Met Ala Glu Gly Ile Leu Ala Asp Gln Ile
                565                 570                 575
Ala Pro Phe Phe Gln Gly Asn Gly Val Ile Gln Asp Ser Ile Ser Leu
            580                 585                 590
Thr Lys Ser Met Leu Ala Met Ser Gln Leu Ser Phe Asn Ser Asn Lys
        595                 600                 605
Lys Arg Ile Thr Asp Cys Lys Glu Arg Val Ser Ser Asn Arg Asn His
    610                 615                 620
Asp Pro Lys Ser Lys Asn Arg Arg Arg Val Ala Thr Phe Val Thr Thr
625                 630                 635                 640
Asp Leu Gln Lys Tyr Cys Leu Asn Trp Arg Tyr Gln Thr Val Lys Leu
                645                 650                 655
Phe Ala His Ala Ile Asn Gln Leu Met Gly Leu Pro His Phe Phe Glu
            660                 665                 670
Trp Ile His Leu Arg Leu Met Asp Thr Thr Met Phe Val Gly Asp Pro
        675                 680                 685
Phe Asn Pro Pro Ser Asp Pro Thr Asp Cys Asp Leu Ser Arg Val Pro
    690                 695                 700
Asn Asp Asp Ile Tyr Ile Val Ser Ala Arg Gly Gly Ile Glu Gly Leu
705                 710                 715                 720
Cys Gln Lys Leu Trp Thr Met Ile Ser Ile Ala Ala Ile Gln Leu Ala
                725                 730                 735
Ala Ala Arg Ser His Cys Arg Val Ala Cys Met Val Gln Gly Asp Asn
            740                 745                 750
Gln Val Ile Ala Ile Thr Arg Glu Val Arg Ser Asp Asp Ser Pro Glu
        755                 760                 765
Met Val Leu Thr Gln Leu His Gln Ala Ser Asp Asn Phe Phe Lys Glu
    770                 775                 780
Leu Ile His Val Asn His Leu Ile Gly His Asn Leu Lys Asp Arg Glu
785                 790                 795                 800
Thr Ile Arg Ser Asp Thr Phe Phe Ile Tyr Ser Lys Arg Ile Phe Lys
                805                 810                 815
Asp Gly Ala Ile Leu Ser Gln Val Leu Lys Asn Ser Ser Lys Leu Val
            820                 825                 830

Leu Ile Ser Gly Asp Leu Ser Glu Asn Thr Val Met Ser Cys Ala Asn
        835                 840                 845
Ile Ala Ser Thr Val Ala Arg Leu Cys Glu Asn Gly Leu Pro Lys Asp
    850                 855                 860
Phe Cys Tyr Tyr Leu Asn Tyr Leu Met Ser Cys Val Gln Thr Tyr Phe
865                 870                 875                 880
Asp Ser Glu Phe Ser Ile Thr Asn Asn Ser Gln Leu Asp Ser Asn Gln
                885                 890                 895
Ser Trp Ile Glu Asp Ile Ser Phe Val His Ser Tyr Val Leu Thr Pro
            900                 905                 910
Ala Gln Leu Gly Gly Leu Ser Asn Leu Gln Tyr Ser Arg Leu Tyr Thr
        915                 920                 925
Arg Asn Ile Gly Asp Pro Gly Thr Thr Ala Phe Ala Glu Ile Lys Arg
    930                 935                 940
Leu Glu Ala Val Gly Leu Leu Ser Pro Ser Ile Met Thr Asn Ile Leu
945                 950                 955                 960
Thr Arg Pro Pro Gly Asn Gly Asp Trp Ala Ser Leu Cys Asn Asp Pro
                965                 970                 975
Tyr Ser Phe Asn Phe Glu Thr Val Ala Ser Pro Asn Ile Val Leu Lys
            980                 985                 990
Lys His Thr Gln Arg Val Leu Phe Glu Thr Cys Ser Asn Pro Leu Leu
        995                 1000                1005
Ser Gly Val His Thr Glu Asp Asn Glu Ala Glu Glu Lys Ala Leu Ala
    1010                1015                1020
Glu Phe Leu Leu Asn Gln Glu Met Ile His Pro Arg Val Ala His Ala
1025                1030                1035                1040
Ile Met Glu Ala Ser Ser Val Gly Arg Arg Lys Gln Ile Gln Gly Leu
                1045                1050                1055
Val Asp Thr Thr Asn Thr Val Ile Lys Ile Ala Leu Thr Arg Arg Pro
            1060                1065                1070
Leu Gly Ile Lys Arg Leu Met Arg Ile Val Asn Tyr Ser Ser Met His
        1075                1080                1085
Ala Met Leu Phe Arg Asp Asp Val Phe Ser Ser Asn Arg Ser Asn His
    1090                1095                1100
Pro Leu Val Ser Ser Asp Met Cys Ser Gln Thr Leu Ala Asp Tyr Ala
1105                1110                1115                1120
Arg Asn Arg Ser Trp Ser Pro Leu Thr Gly Gly Arg Lys Ile Leu Gly
                1125                1130                1135
Val Ser Asn Pro Asp Thr Ile Glu Leu Val Glu Gly Glu Ile Leu Ser
            1140                1145                1150
Val Ser Gly Gly Cys Thr Arg Cys Asp Ser Gly Asp Glu Gln Phe Thr
        1155                1160                1165
Trp Phe His Leu Pro Ser Asn Ile Glu Leu Asn Asp Asp Thr Ser Lys
    1170                1175                1180
Asn Pro Pro Met Arg Val Pro Tyr Leu Gly Ser Lys Thr Gln Glu Arg
1185                1190                1195                1200
Arg Ala Ala Ser Leu Ala Lys Ile Ala His Met Ser Pro His Val Lys
                1205                1210                1215
Ala Ala Leu Arg Ala Ser Ser Val Leu Ile Trp Ala Tyr Gly Asp Asn
            1220                1225                1230
Glu Ile Asn Trp Thr Ala Ala Leu Lys Ile Ala Arg Ser Arg Cys Asn
        1235                1240                1245
Ile Ser Ser Glu Tyr Leu Arg Leu Leu Ser Pro Leu Pro Thr Ala Gly
    1250                1255                1260
Asn Leu Gln His Arg Leu Asp Asp Gly Ile Thr Gln Met Thr Phe Thr
1265                1270                1275                1280
Pro Ala Ser Leu Tyr Arg Val Ser Pro Tyr Ile His Ile Ser Asn Asp
                1285                1290                1295
Ser Gln Arg Leu Phe Thr Glu Glu Gly Val Lys Glu Gly Asn Val Val
            1300                1305                1310
Tyr Gln Gln Ile Met Leu Leu Gly Leu Ser Leu Ile Glu Ser Leu Phe
        1315                1320                1325
Pro Met Thr Thr Thr Lys Thr Tyr Asp Glu Ile Thr Leu His Leu His

1330                1335                1340
Ser Lys Phe Ser Cys Cys Ile Arg Glu Ala Pro Val Ala Val Pro Phe
1345                1350                1355                1360
Glu Leu Leu Gly Val Ala Pro Glu Leu Arg Ala Val Thr Ser Asn Lys
                1365                1370                1375
Phe Met Tyr Asp Pro Ser Pro Val Ser Glu Gly Asp Phe Ala Arg Leu
            1380                1385                1390
Asp Leu Ala Ile Phe Lys Ser Tyr Glu Leu Asn Leu Glu Ser Tyr Ser
        1395                1400                1405
Thr Ile Glu Leu Met Asn Val Leu Ser Ile Ser Ser Gly Lys Leu Ile
    1410                1415                1420
Gly Gln Ser Val Val Ser Tyr Asp Glu Asp Thr Ser Ile Lys Asn Asp
1425                1430                1435                1440
Ala Ile Ile Val Tyr Asp Asn Thr Arg Asn Trp Ile Ser Glu Ala Gln
                1445                1450                1455
Asn Ser Asp Val Val Arg Leu Phe Glu Tyr Ala Ala Leu Glu Val Leu
            1460                1465                1470
Leu Asp Cys Ser Tyr Gln Leu Tyr Tyr Leu Arg Val Arg Gly Leu Asn
        1475                1480                1485
Asn Ile Val Leu Tyr Met Ser Asp Leu Tyr Lys Asn Met Pro Gly Ile
    1490                1495                1500
Leu Leu Ser Asn Ile Ala Ala Thr Ile Ser His Pro Val Ile His Ser
1505                1510                1515                1520
Arg Leu Asn Ala Val Gly Leu Val Asn His Asp Gly Ser His Gln Leu
                1525                1530                1535
Ala Asp Thr Asp Phe Ile Glu Met Ser Ala Lys Leu Leu Val Ser Cys
            1540                1545                1550
Thr Arg Arg Val Val Ser Gly Leu His Ala Gly Asn Lys Tyr Asp Leu
        1555                1560                1565
Leu Phe Pro Ser Val Leu Asp Asp Asn Leu Ser Glu Lys Met Leu Gln
    1570                1575                1580
Leu Ile Ser Gln Leu Cys Cys Leu Tyr Thr Val Leu Phe Ala Thr Thr
1585                1590                1595                1600
Arg Glu Ile Pro Lys Ile Arg Gly Leu Ser Ala Glu Glu Lys Cys Ser
                1605                1610                1615
Val Leu Thr Glu Tyr Leu Leu Ser Asp Ala Val Lys Pro Leu Leu Gly
            1620                1625                1630
Ser Glu Gln Val Ser Ser Ile Met Ser Pro Asn Ile Val Thr Phe Pro
        1635                1640                1645
Ala Asn Leu Tyr Tyr Met Ser Arg Lys Ser Leu Asn Leu Ile Arg Glu
    1650                1655                1660
Arg Glu Asp Arg Asp Thr Ile Leu Ala Leu Leu Phe Pro Gln Glu Pro
1665                1670                1675                1680
Leu Leu Glu Phe Pro Leu Val Arg Asp Ile Gly Ala Arg Val Lys Asp
                1685                1690                1695
Pro Phe Thr Leu Gln Pro Ala Ala Phe Leu Gln Glu Leu Asp Leu Ser
            1700                1705                1710
Ala Pro Ala Arg Tyr Asp Ala Phe Thr Ile Ser Gln Ala His Ser Glu
        1715                1720                1725
His Ile Leu Pro Asn Pro Glu Glu Asp His Leu Val Arg Tyr Leu Phe
    1730                1735                1740
Arg Gly Ile Gly Thr Ala Ser Ser Ser Trp Tyr Lys Ala Ser His Leu
1745                1750                1755                1760
Leu Ser Val Pro Glu Val Arg Cys Ala Arg His Gly Asn Ser Leu Tyr
                1765                1770                1775
Leu Ala Glu Gly Ser Gly Ala Ile Met Ser Leu Leu Glu Leu His Ile
            1780                1785                1790
Pro His Glu Thr Ile Tyr Tyr Asn Thr Leu Phe Ser Asn Glu Met Asn
        1795                1800                1805
Pro Pro Gln Arg His Phe Gly Pro Thr Pro Thr Gln Phe Leu Asn Ser
    1810                1815                1820
Val Val Phe Arg Asn Leu Gln Ala Glu Val Pro Cys Lys Asp Gly Phe
1825                1830                1835                1840

Val Gln Glu Phe Arg Pro Leu Trp Arg Glu Asn Thr Glu Glu Ser Asp
                1845                1850                1855
Leu Thr Ser Asp Lys Ala Val Gly Tyr Ile Thr Ser Val Val Pro Tyr
            1860                1865                1870
Arg Ser Val Ser Leu Leu His Cys Asp Ile Glu Ile Pro Pro Gly Ser
        1875                1880                1885
Asn Gln Ser Leu Leu Asp Gln Leu Ala Thr Asn Leu Ser Leu Ile Ala
    1890                1895                1900
Met His Ser Val Lys Glu Gly Gly Val Val Ile Ile Lys Val Leu Tyr
1905                1910                1915                1920
Ala Met Gly Tyr Tyr Phe His Leu Leu Met Asn Leu Phe Thr Pro Cys
                1925                1930                1935
Ser Thr Lys Gly Tyr Ile Leu Ser Asn Gly Tyr Ala Cys Arg Gly Asp
            1940                1945                1950
Met Glu Cys Tyr Leu Ile Phe Val Met Gly Tyr Leu Gly Gly Pro Thr
        1955                1960                1965
Phe Val His Glu Val Val Arg Met Ala Lys Thr Leu Val Gln Arg His
    1970                1975                1980
Gly Thr Leu Leu Ser Lys Ser Asp Glu Ile Thr Leu Thr Arg Leu Phe
1985                1990                1995                2000
Thr Ser Gln Gln Gln Arg Val Thr Asp Ile Leu Ser Ser Pro Leu Pro
                2005                2010                2015
Arg Leu Met Lys Tyr Leu Arg Glu Asn Ile Asp Thr Ala Leu Ile Glu
            2020                2025                2030
Ala Gly Gly Gln Pro Val Arg Pro Phe Cys Ala Glu Ser Leu Val Ser
        2035                2040                2045
Thr Leu Thr Asp Met Thr Gln Thr Thr Gln Ile Ile Ala Ser His Ile
    2050                2055                2060
Asp Thr Val Ile Arg Ser Val Ile Tyr Met Glu Ala Glu Gly Asp Leu
2065                2070                2075                2080
Ala Asp Thr Val Phe Leu Phe Thr Pro Tyr Asn Leu Ser Met Asp Gly
                2085                2090                2095
Lys Lys Arg Thr Ser Leu Lys Gln Cys Thr Arg Gln Ile Leu Glu Val
            2100                2105                2110
Thr Ile Leu Gly Leu Arg Val Lys Asn Leu Asn Lys Val Gly Asp Val
        2115                2120                2125
Ile Ser Leu Val Leu Arg Gly Met Ile Ser Leu Glu Asp Leu Leu Pro
    2130                2135                2140
Leu Arg Thr Tyr Leu Lys Cys Ser Thr Cys Pro Lys Tyr Leu Lys Ala
2145                2150                2155                2160
Val Leu Gly Ile Thr Lys Leu Lys Glu Met Phe Thr Asp Ala Ser Leu
                2165                2170                2175
Leu Tyr Leu Thr Arg Ala Gln Gln Lys Phe Tyr Met Lys Thr Ile Gly
            2180                2185                2190
Asn Ala Val Lys Gly Tyr Tyr Ser Asn Cys Asp Ser
        2195                2200

<210> 13
<211> 36
<212> DNA
<213> Artificial Sequence

<220>
<223> Primer

<400> 13
acgactcact ataggaccaa acagagaatc cgtgag                                36

<210> 14
<211> 35
<212> DNA
<213> Artificial Sequence

<220>
<223> Primer

<400> 14
ccgggaagat ccagggcact cttcttgcat gttac                                35

<210> 15
<211> 35
<212> DNA
<213> Artificial Sequence

<220>
<223> Primer

<400> 15
gggcctgcct cactatggtg gtaacatgca agaag                                35

<210> 16
<211> 35
<212> DNA
<213> Artificial Sequence

<220>
<223> Primer

<400> 16
tgcatgttac caccaatgtg tcattgtatc gcttg                                35

<210> 17
<211> 37
<212> DNA
<213> Artificial Sequence

<220>
<223> Primer

<400> 17
caagaaggga gatacgtaat atacaagcga tacaatg                              37

<210> 18
<211> 35
<212> DNA
<213> Artificial Sequence

<220>

<223> Primer

<400> 18
tcgcttgtat attacttgtt gtagcaaaga gcacc 35

<210> 19
<211> 38
<212> DNA
<213> Artificial Sequence

<220>
<223> Primer

<400> 19
ggcctggatc ttcccattat gctgtctgta tacggtgc 38

<210> 20
<211> 38
<212> DNA
<213> Artificial Sequence

<220>
<223> Primer

<400> 20
atgccatgcc gacccaccaa acaaagactt ggtgaatg 38

<210> 21
<211> 24
<212> DNA
<213> Artificial Sequence

<220>
<223> Primer

<400> 21
cctatagtga gtcgtattaa tttc 24

<210> 22
<211> 20
<212> DNA
<213> Artificial Sequence

<220>
<223> Primer

<400> 22
cctggatctt cccgggtcgg 20

<210> 23
<211> 22
<212> DNA
<213> Artificial Sequence

<220>
<223> Primer

<400> 23
gggtcggcat ggcatctcca cc                                                22

<210> 24
<211> 22
<212> DNA
<213> Artificial Sequence

<220>
<223> Primer

<400> 24
gggaagatcc aggcctatag tg                                                22

<210> 25
<211> 35
<212> DNA
<213> Artificial Sequence

<220>
<223> Primer

<400> 25
ctctagagtc gacccttctg ccaacatgtc ttccg                                   35

<210> 26
<211> 35
<212> DNA
<213> Artificial Sequence

<220>
<223> Primer

<400> 26
gggaagcggc cgcccgtcgg tcagtatccc cagtc                                   35

<210> 27
<211> 37
<212> DNA
<213> Artificial Sequence

<220>

<223> Primer

<400> 27
ctctagagtc gaccccagag tgaagatggc caccttc                                37

<210> 28
<211> 35
<212> DNA
<213> Artificial Sequence

<220>
<223> Primer

<400> 28
gggaagcggc cgcccgtagt agtgatcagc cattc                                  35

<210> 29
<211> 35
<212> DNA
<213> Artificial Sequence

<220>
<223> Primer

<400> 29
ctctagagtc gacccgggta ggacatggcg ggctc                                  35

<210> 30
<211> 37
<212> DNA
<213> Artificial Sequence

<220>
<223> Primer

<400> 30
gggaagcggc cgccctgcct ttaagagtca cagttac                                37

## Description

This invention relates to recombinant Newcastle Disease Viruses (NDV), which have been demonstrated to possess significant oncolytic activity against mammalian cancers, especially selected from the specific cancers as described herein and/or mentioned in the claims, and which provided improved viral safety. The invention provides the development of novel and improved oncolytic agents through the use of genetic engineering, including the transfer of genes across species boundaries in order to produce improved genetically modified viruses carrying these transgenes.

### Background

NDV is known as an oncolytic virus that is a virus for use in oncological treatment, preferably in the treatment of human subjects in need thereof. A number of RNA viruses, including NDV, reovirus, measles virus, and vesicular stomatitis virus (VSV), are members of this novel class of viruses being exploited as potential oncolytic agents. These oncolytic viruses are characterized in inherently replicating selectively within and killing tumor cells while leaving non-tumor cells unharmed. Accordingly these oncolytic viruses offer an attractive new tool for cancer therapy.

NDV derives its name from the site of the original outbreak in chickens at a farm near Newcastle-upon-Tyne in England in 1926. The virus is an economically important pathogen in multiple avian species and it is endemic in many countries. NDV is a member of the Avulavirus genus in the Paramyxoviridae family and is a non-segmented, negative-strand RNA virus, whose natural host range is limited to avian species; however, it is known to enter cells by binding to sialic acid residues present on a wide range of human and rodent cancer cells. The oncolytic property of NDV, i.e. to selectively replicate in and destroy tumor cells, while sparing normal cells, is believed to be based in part on defective antiviral responses in tumor cells. Normal cells, which are competent in launching an efficient antiviral response rapidly after infection, are able to inhibit viral replication before viral-mediated cell damage can be initiated. The sensitivity of NDV to interferon (IFN), coupled with defective IFN signalling pathways in tumor cells, provides one plausible mechanism whereby NDV can replicate exclusively within neoplastic tissue. The selective replication of NDV in human tumor cells may also be caused by several other mechanisms, including defects in activation of anti-viral signalling pathways, and activation of Ras signalling and/or expression of Rac1 (Schirrmacher, 2015, Expert Opin. Biol. Ther. 15:17 57-71).

Due to its tumor-specifity NDV is already under investigation as a safe clinical oncolytic virotherapy agent (Cassel et al., 1965, Cancer 1:863-888; Steiner et al., 2004, J. Clin. Oncol. 22:4272-4281; Schulze et al., 2009, Cancer Immunol. Immunother. 58:61-69; Csatary et al., 1999, Anticancer Res. 19:635-638; Pecora et al., 2002, J. Clin. Oncol. 20:2251-2266; Lorence et al., 2003, Curr. Opin. Mol. Ther. 5:618-624; Freeman et al., 2006, Mol. Ther. 13:221-228). The safety margin of NDV is a function of its inability to replicate and kill normal human cells.

Typically, an oncolytic NDV strain is defined as an NDV for use in oncological virotherapy, preferably in the treatment of human subjects in need thereof. Multiple preclinical model studies have shown significant anti-tumor activity of natural and recombinant oncolytic NDV strains after varying treatment modalities. Promising results were noted in preclinical models of glioblastoma multiforme, anaplastic astrocytoma, leukemia, lymphoma, melanoma, neuroblastoma, osteosarcoma, rhabdomyosarcoma, Ewing's sarcoma, fibrosarcoma, pheochromocytoma, colon carcinoma, lung carcinoma, prostate carcinoma, breast carcinoma, ovary carcinoma, gastric carcinoma, mesothelioma, renal cell carcinoma, and head and neck carcinoma. One typically useful oncolytic NDV that has been extensively explored for use in the treatment of humans, and for which an advantageous safety and efficacy profile has been established in human trials, is a strain named MTH-68/H (Csatary LK, et al., J Neurooncol. 2004 Mar-Apr;67(1-2):83-93). MTH-68/H therapy has been employed in a range of different tumors with success. MTH-68/H has been developed into a highly purified, lyophilized product, containing live, replication competent viral particles, grown to standardized titers. A Phase II clinical trial in humans was completed where the inhalatory mode of administration was used on patients suffering from a variety of advanced malignancies no longer responding to conventional cancer therapies. The study demonstrated relative efficacy, an overall improved quality of life, and a relatively benign side effect profile (Csatary LK, et al., Cancer detection and Prevention, 1993, 17(6):619-627). Success of treatment with MTH-68/H has also been shown in glioblastomas and anaplastic astrocytomas. Glioblastoma multiforme (GBM) is the most common primary brain tumor and by far the most aggressive form of glial tumors. This neoplasm has a poor prognosis, averaging six months to a year without therapy or about one-and-one-half years with conventional therapy. Four cases of advanced GBM and anaplastic astrocytoma were treated with the NDV viral product MTH-68/H after the conventional modalities of cancer therapy had failed. Results included survival rates of twelve and half to six years post-viral treatment for the surviving patients, and six years for one patient who has since died, after having discontinued treatment. Against all odds, these surviving patients returned to good quality of life and to a lifestyle that resemble their pre-morbid daily routines including giving birth to a healthy child and full professional activity. They have been able to enjoy good clinical health. These patients regularly received the MTH-68/H viral therapy for a number of years without interruption as a form of monotherapy once the classical modalities failed. The MRI and CT results have revealed an objective decrease in size of the tumors, in some cases the near total disappearance of the tumor mass.

As reviewed by Zamarin and Palese (Future Microbiol. 2012 Mar; 7(3): 347-367), when compared to other oncolytic agents in development such as poxvirus, HSV-1, adenovirus, measles, and reovirus vectors, NDV as an oncolytic virus exhibits several advantages over other viruses for use in oncological treatment.

The first advantage is that NDV is an avian pathogen. This avoids the problems of preexisting immunity that can neutralize virus infectivity and pathogenicity of the virus in humans. These potential problems exist when using vaccinia, HSV-1, adenovirus, and measles vectors. Based on serological studies it seems that about 96% of the human population is seronegative for NDV, which avoids the problem of pre-existing immunity. The actual level of seropositivity in today's population may be even lower due to low human exposure to NDV, as the outbreaks of which are primarily limited to farm settings. To attest for viral safety, administration of virulent strains to humans has been shown to result in only mild to moderate adverse effects, with mild conjunctivitis, laryngitis, and flu-like symptoms as the only reported side effects. Natural human infections with highly virulent (avian) strains have been reported in the literature in farmers and laboratory workers and have been limited to mild symptoms such as conjunctivitis and laryngitis.

The second advantage offered by using NDV as an oncolytic agent is that NDV, similarly to other oncolytic viruses, possesses strong immunostimulatory properties that are the basis for oncolytic therapy being considered an immunotherapy. These properties include the induction of type I IFN and chemokines, upregulation of MHC and cell adhesion molecules, and facilitation of adhesion of lymphocytes and antigen-presenting cells (APCs) through expression of viral glycoproteins on the surface of infected cells. These properties have been shown to generate effective anti-tumor immune responses, which may persist long after clearance of the primary viral infection.

A third advantage is that the NDV genome has the plasticity to enable the incorporation and stable expression of foreign genes of relatively large size. Since these viruses replicate in the cytoplasm of the cell and not in the nucleus, an unintended integration of the foreign gene into the host genome is avoided. Such an unintended integration of foreign genes carried by an oncolytic agent into the host genome could be a safety problem with some DNA oncolytic vectors. Moreover, the absence of homologous RNA recombination ensures that foreign genes incorporarted in and expressed from the NDV genome are stable for many serial passages in cell culture and in tumor cells.

Lastly, the ubiquitous nature of the NDV receptor allows for utilization of the virus against a wide variety of tumor types. The specificity of NDV for cancer cells due to their defects in antiviral pathways ensures viral safety.

Nevertheless, the full view of all clinical data generated to date in different trials of different NDV strains shows that there is much need for improvement in terms of percentage responders and magnitude of therapeutic effect. Also it is desired to further improve the viral safety. This need for improvement has spurred efforts to design improved recombinant NDV strains, which is the subject of the present patent application.

Similar to other paramyxoviruses in its family, the 15186, 15192 or 15198 nucleotide(nt)-long negative single-strand RNA genome of NDV encodes six genes including the nucleocapsid protein (NP), phosphoprotein (P), matrix protein (M), fusion protein (F), hemagglutinin-neuraminidase (HN), and RNA-dependent RNA polymerase (L) (Lamb R, Paramyxoviridae: the viruses and their replication. In: Knipe D, editor. Fields Virology, Lippincott Williams & Wilkins; 2007, pp. 1449-1496). The genes are separated by junction sequences that consist of three elements, known as gene start (GS), intergenic (IG), and gene-end (GE) motifs, which regulate mRNA transcription. In the P gene, a unique RNA editing mechanism adds non-templated G residues, resulting in the expression of V and W proteins that are colinear to the P protein in the amino-terminal end. The genomic RNA is bound in a ribonucleotide protein complex (RNP) consisting of NP, P, and L proteins and is surrounded by a lipid envelope containing three virus glycoprotein spikes, HN, M and F. An important factor that influences the extent of virulence of NDV in birds is the cleavage site of the F protein.

Pathogenic classification and virulence of NDV strains in birds generally correlates with their oncolytic properties in human cancer cells. Velogenic (high virulence) strains produce severe respiratory and nervous system signs, spread rapidly through chicken flocks, and can cause up to 90% mortality. Mesogenic (intermediate virulence) strains cause coughing, affect egg production and quality, and can result in up to 10% mortality. Lentogenic (low virulence) strains produce only mild symptoms with little if any mortality. Correspondingly, velogenic strains can efficiently carry out multicycle replication in multiple human cancer cells with effective and efficient cell lysis, lentogenic strains are more attenuated due to lack of activation of the F0 protein, and mesogenic strains convey intermediate effects. On the basis of these observations in human cancers, NDV strains have been classified as either lytic or non-lytic, with velogenic and mesogenic viruses being lytic (high and low respectively), and lentogenic viruses in general being non-lytic. Early studies demonstrated that the lytic abilities of lentogenic NDV strains could be enhanced by the introduction of a polybasic cleavage site into their F proteins (Peeters et al, J. Virol. 1999; 73:5001-5009). Other NDV proteins including NP, P, V, HN, and L have also been shown to be implicated in virulence in birds. A deletion (18nt) introduced in the NP gene (Mebatsion T et al., J Virol. 2002 Oct; 76 (20):10138-46) resulted in the deletion of NP residues 443-460. The resulting mutant NDV propagated in embryonated specific-pathogen-free (SPF) chicken eggs to a level fully comparable to that of the parent virus.

In addition, a B-cell epitope of the S2 glycoprotein of murine hepatitis virus (MHV) was inserted in-frame. Recombinant NDV viruses properly expressing the introduced MHV epitope were successfully generated, demonstrating that the NP can be used as the insertion point in the NDV genome to insert foreign or transgenic sequences to be co-expressed with NDV during virus infection.

Several studies showed that type I IFN antagonist activity of NDV V protein is associated with pathogenicity in birds. In recombinant viruses possessing attenuating mutations or deletions in the V protein or in viruses expressing no or lower levels of V protein decreased pathogenicity in birds was observed (Huang et al., 2003, J Virol. 77:8676-8685; Mebatsion et al., 2001, J Virol. 75:420-428; Qiu et al., 2016, PLoS ONE 11(2): e0148560. doi: 10.1371/journal.pone.0148560; Elankumaran et al., 2010, J Virol. 84:3835-3844; Jang et al., 2010, Appl Microbiol Biotechnol. 85:1509-1520; Alamares et al., 2010, Virus Res. 147:153-157; Park et al., 2003, J Virol.77:9522-9532.). It was found to be sufficient to introduce a small (6 nt) deletion in the P gene to abolish expression of the V protein (Mebatsion et al., 2001).

Recently, two studies showed the role of the polymerase complex in viral pathogenesis, with L protein contributing to pathogenicity of the mesogenic Beaudette C strain, and NP, P, and L proteins contributing to pathogenicity of the velogenic Herts strain (Rout & Samal, 2008, J Virol. 82:7828-7836; Dortmans et al., 2010, J Virol. 84:10113-10120). In addition, several studies noted the contribution of the HN protein to virulence in birds (Römer-Oberdörfer et al., 2006, Avian Dis. 50:259-263; Paldurai et al., 2014, J Virol. 88: 8579-8596.) The HN (hemagglutinin-neuraminidase) protein of NDV is a multifunctional protein with receptor-recognition, hemagglutinin (HA) and receptor-destroying neuraminidase (NA) activities associated with the virus. HN is thought to possess both the receptor recognition of sialic acid at the termini of host glycoconjugates and the neuraminidase activity to hydrolyze sialic acid from progeny virion particles in order to prevent viral self-aggregation. It also recognizes sialic acid-containing receptors on cell surfaces and promotes the fusion activity of the F protein, thereby allowing the virus to penetrate the cell surface. Thus, the HN protein plays a critical role in viral infection of birds.

A recombinant NDV vector virus can be provided by a reverse genetics (rg) system, which has been known since 1999 (Peeters et al., 1999, J. Virol. 73:5001-5009). This system allows desired modification and engineering of NDV genomes. Indeed, various reports have shown the successful use of recombinant NDV vectors engineered to express various transgenes, i.e. foreign genes incorporated in the genome of the virus, with the goal of improving viral oncolytic efficacy (Vigil et al. Cancer Res. 2007, 67:8285-8292; Janke et al., 2007, Gene Ther. 14:1639-1649). However, it should be noted that the replication capability of a recombinant NDV expressing a transgene may be hampered in comparison with its parental NDV strain not expressing the transgene. As discussed above, the viral F protein of NDV is responsible for viral fusion with the cell membrane and for viral spread from host cell to host cell via formation of syncytia. The presence of the multi-basic amino acid cleavage site in the F protein enables protein cleavage and activation by a broad range or proteases and is known to be a determinant of virulence in velogenic NDV strains. In order to increase the oncolytic potency of a highly attenuated lentogenic Hitchner B1 NDV strain, a polybasic cleavage site was introduced into the F protein to generate rNDV/F3aa with mutated F protein. Altomonte et al. (Mol Ther. 2010 18:275-84) reported an oncolytic NDV vector virus with the mutated F-protein rNDV/F(L289A), harboring an L289A mutation within the F protein, which is required for virus entry and cell-cell fusion. This rNDV/F3aa(L289A) virus with mutated F protein showed further enhanced fusion and cytotoxicity of hepatocellular carcinoma (HCC) cells in vitro, as compared with a rNDV/F3aa control virus. In vivo administration of rNDV/F3aa(L289A), via hepatic arterial infusion in immune-competent Buffalo rats bearing multifocal, orthotopic liver tumors resulted in tumor-specific syncytia formation and necrosis, with no evidence of toxicity to the neighboring hepatic parenchyma, which translated to a 20% prolongation of survival time relative to treatment with the original rNDV/F3aa virus.

Due to compelling preclinical data implicating oncolytic NDV as an ideal candidate for cancer therapy, phase I and II clinical trials have been initiated. Several trials have shown that NDV is a safe and potentially therapeutically useful therapeutic agent, with no reports of significant adverse effects in patients beyond conjunctivitis or mild flu-like symptoms (Csatary et al., 1999, Anticancer Res. 19:635-638; Pecora et al., 2002, J. Clin. Oncol. 20:2251-2266; Lorence et al., 2003, Curr. Opin. Mol. Ther. 5, 618-624; Freeman et al., 2006, Mol. Ther. 13:221-228). Although the results of these clinical trials have been encouraging, the extent of clinical responses has not been strong and robust enough to consider bringing one of these initial NDV strains into advanced clinical development. Thus, strategies for improving therapeutic effectiveness while maintaining an acceptable safety profile of oncolytic NDV strains are needed. Thus there remains a clear need for improvement in therapeutic outcome by providing improved NDV strains, in particular NDV strains with enhanced killing effect without affecting - or even improving - its safety toward normal cells.

### Description of the present invention

In a first aspect the present invention provides recombinant Newcastle disease virus or NDV strains. A recombinant NDV according to the present invention carries as a foreign gene at least one gene selected from:
- A gene encoding an antibody capable of blocking checkpoint inhibition, such as an antibody directed against checkpoint inhibition protein PD-1 (programmed cell death protein 1, also known as CD279) or an antigen-binding part directed to protein PD-1 (anti-PD-1).
- A gene encoding an antibody capable of blocking checkpoint inhibition, such as an antibody directed to the surface protein CTLA-4 or an antigen-binding part directed to protein CTLA-4 (anti-CTLA-4).
- A gene encoding a protein which improves the cellular immune response and improves the ability of T cells to enter tumor cells, or a part thereof which improves the cellular immune response and improves the ability of T cells to enter tumor cells.
- A gene encoding a protein with the ability to modulate the virus replication cycle, or a part thereof with the ability to modulate the virus replication cycle.
- A gene encoding a protein with the ability to selectively induce apoptosis in human tumor cells, but not in normal human cells, or a part thereof with the ability to selectively induce apoptosis in human tumor cells, but not in normal human cells.
- A gene encoding a protein that reduces or inhibits IFN expression such as an IFN-beta receptor, or a part thereof that reduces or inhibits IFN expression such as an IFN-beta receptor.
- Any combination of these genes, parts or variants of these genes or parts thereof.

The foreign gene or part or the respective variant is/are expressed in the recombinant NDV strain of the present invention, when the virus is replicating in a suitable host or host cell. An expression of a gene means that the nucleic acid information encoded by the gene is translated in a respective amino acid sequence, which is the primary sequence for building up the respective protein or gene product. By way of example, the gene product of the HN gene is the HN protein.

Within the present invention the foreign gene carried by the recombinant NDV encodes in one embodiment an antibody capable of blocking checkpoint inhibition, such as an antibody directed against checkpoint inhibition protein PD-1 (programmed cell death protein 1, also known as CD279) or an antigen-binding part directed to protein PD-1 (anti-PD-1). According to the present invention the antiboby or antigen-binding part thereof is in a particularly preferred embodiment Nivolumab, an antigen-binding part of Nivolumab, a variant of Nivolumab or a variant of an antigen-binding part of Nivolumab. They work by blocking a signal that prevents activated T cells from attacking the cancer upon expression in rNDV-expresser cells, thus allowing the immune system to clear the cancer.

According to another embodiment of the present invention the foreign gene carried by the recombinant NDV encodes an antibody capable of blocking checkpoint inhibition, such as an antibody directed to the surface protein CTLA-4 or an antigen-binding part directed to protein CTLA-4 (anti-CTLA-4). According to the present invention the antiboby or antigen-binding part thereof is in a particularly preferred embodiment Ipilimumab, an antigen-binding part of Ipilimumab or a variant of Ipilimumab or a variant of an antigen-binding part of Ipilimumab.

According to another embodiment of the present invention the foreign gene carried by the recombinant NDV encodes a protein which improves the cellular immune response and improves the ability of T cells to enter tumor cells, or a part thereof which improves the cellular immune response and improves the ability of T cells to enter tumor cells. According to the present invention the protein is in a particularly preferred embodiment interleukin-12 (IL-12) or a part of interleukin-12 or a variant of interleukin-12 or a variant of a part of interleukin-12.

According to another embodiment of the present invention the foreign gene carried by the recombinant NDV encodes a protein with the ability to modulate the virus replication cycle, or a part thereof with the ability to modulate the virus replication cycle. According to the present invention the protein is in a particularly preferred embodiment the non-structural protein NS1 of influenza A virus or a part of the non-structural protein NS1 of influenza A virus or a variant of the non-structural protein NS1 of influenza A virus or a variant of a part of the non-structural protein NS1 of influenza A virus.

According to another embodiment of the present invention the foreign gene carried by the recombinant NDV encodes a protein with the ability to selectively induce apoptosis in human tumor cells, but not in normal human cells, or a part thereof with the ability to selectively induce apoptosis in human tumor cells, but not in normal human cells. According to the present invention the protein is in a particularly preferred embodiment Apoptin (VP3 from chicken anemia virus) or a part of Apoptin or a variant of Apoptin or a variant of a part of Apoptin.

According to another embodiment of the present invention the foreign gene carried by the recombinant NDV encodes a protein that reduces or inhibits IFN expression such as an IFN-beta receptor, or a part thereof that reduces or inhibits IFN expression such as an IFN-beta receptor. According to the present invention the protein is in a particularly preferred embodiment the viral protein B18R from vaccinia virus or a part of the viral protein B18R from vaccinia virus or a variant of the viral protein B18R from vaccinia virus or a variant of a part of the viral protein B18R from vaccinia virus. B18R is a homologue of the human IFN-β receptor. B18R acts as a decoy for IFN-β, thereby inhibiting activation by cell signaling of the antiviral host response in native cells, resulting in increased lytic activity in cancer cells.

In another embodiment of the present invention a recombinant NDV comprises in its viral genome at least two foreign genes or parts or variants thereof, which foreign genes or parts or variants are selected from the above mentioned foreign genes.

A particulary preferred embodiment of the present invention is a recombinant NDV strain carrying as a foreign gene Nivolumab, an antigen-binding part of Nivolumab or a variant of Nivolumab or its antigen-binding part. Therefore, in the following the present invention will be described in particular with reference to that foreign gene, bit it should be understood that the respective teaching and all discloed preferred embodiments of such Nivolumab carrying NDV strains also apply to the other foreign genes, parts or variants, in particular Ipilimumab, interleukin-12 (IL-12), the non-structural protein NS1 of influenza A virus, Apoptin, and the viral protein B18R from vaccinia virus.

A "variant" of a starting nucleic acid is a nucleic acid that comprises a nucleic acid sequence different from that of the starting nucleic acid. Generally, a variant will possess at least 75 % sequence identity, preferably at least 90 % sequence identity, more preferably at least 95 % sequence identity, and most preferably at least 98 % sequence identity with the native nucleic acid. Variants of a nucleic acid may be prepared by introducing appropriate nucleotide changes into the nucleic acid. Such variants include, for example, deletions from, and/or insertions into and/or substitutions of residues within the nucleic acid sequence of the gene of interest. Any combination of deletion, insertion, and substitution is possibly made to arrive at the final construct, provided that the final construct possesses the desired characteristics. Methods for generating nucleic acid sequence variants are known to the skilled person.

A "variant" of a starting polypeptide or protein, e.g. an antibody or another protein, is a polypeptide or protein that comprises an amino acid sequence different from that of the starting polypeptide or protein. Generally, a variant will possess at least 75 % sequence identity, preferably at least 90 % sequence identity, more preferably at least 95 % sequence identity, and most preferably at least 98 % sequence identity with the native polypeptide or protein. Variants of a polypeptide or a proteine may be prepared by introducing appropriate nucleotide changes into the nucleic acid encoding the polypeptide or protein. Such variants include, for example, deletions from, and/or insertions into and/or substitutions of residues within the amino acid sequence of the polypeptide/proteine of interest. Any combination of deletion, insertion, and substitution is possibly made to arrive at the final construct, provided that the final construct possesses the desired characteristics. The amino acid changes also may alter post-translational processes of the polypeptide, such as changing the number or position of glycosylation sites. Methods for generating amino acid sequence variants of polypeptides are known to the skilled person.

Percentage sequence identity is determined after the best alignment of the sequence of interest with the respective reference sequence. The best alignment of the sequences may for example be produced by means of the Fitchet al., Proc. Natl. Acad. Sci. USA 80:1382-1386 (1983), version of the algorithm described by Needleman et al., J. Mol. Biol.48:443-453 (1970), after aligning the sequences to provide for best homology, by means of the similarity search method of Pearson and Lipman, Proc. Natl Acad. Sci. USA 85, 2444 (1988), or by means of computer programs which use these algorithms (in particular BLASTN in Wisconsin Genetics Software Package, Genetics Computer Group, 575 Science Drive, Madison, Wis.). The percentage identity is calculated by determining the number of identical positions between the two sequences being compared, dividing this number by the number of positions compared and multiplying the result obtained by 100 so as to obtain the percentage identity between these two sequences. Preferably, sequence identity of a nucleotide sequence is determined using BLASTN, preferably BLASTN in standard settings as provided by the website of the U.S. National Library of Medicine "https://blast.ncbi.nlm.nih.gov". Also preferably, sequence identity of an amino acid sequence is determined using BLASTP, preferably BLASTP in standard settings as provided by the website of the U.S. National Library of Medicine "https://blast.ncbi.nlm.nih.gov". It is further preferred that the sequence identity is calculated over the entire length of the respective sequence.

As used herein, the term "part" means a fragment of a starting nucleic acid or amino acid sequence, which fragment is a made up of a consecutive sequence of the nucleotides or amino acids of the starting sequence. The part or fragment preferably comprises at least 10, more preferably at least 20 and preferably up to 500 nucleotides or amino acids. In any case a part according to the present invention is a functional part. That is nucleic acid or proteine encoded by that nucleic acid must provide the desired function, e.g. an antigen-binding function or expression inhibiting function as specified herein, of the starting gene or protein.

In addition to carrying at least one foreign gene or a part or variant thereof as specified supra, the recombinant NDV strains according to the present invention further comprise a mutation in the viral HN gene leading to a change in the amino acid sequence of the HN protein and providing the NDV with an increased replication capability in a cancer cell, preferably in a human cancer cell, as compared to an otherwise identical NDV not having the said mutation in the HN gene.

Preferably the mutation in the HN gene results in an unexpected beneficially increased replication capability of the recombinant NDV strain in cancer cells, preferably in human cancer cells, and more preferably results in a replication of said oncolytic NDV in a human cancer cell which is at least 2-fold higher, more preferably 2- to 10-fold higher, still more preferably 5- to 10-fold higher than an NDV parent strain not having said mutation in its viral HN gene. Preferably, said parent strain comprises or is an oncolytic NDV already having an advantageous safety and efficacy profile in humans, such as the NDV strain MTH-68/H.

According to a preferred embodiment the recombinant NDV strain comprises a mutated HN gene and the encoded hemagglutinin-neuramidase (HN proteine) with an amino acid substitution at position 277 to an amino acid with a hydrophobic side chain other than phenylalanine. Still more preferred is the amino acid phenylalanine (F) substituted to leucine (L) at amino acid position 277 of the HN gene. In a more preferred embodiment, the invention provides an oncolytic NDV derived from NDV strain MTH-68/H having this mutation in the HN gene and carrying at least one of the foreign genes or parts or functional analogs thereof as specified supra, said oncolytic NDV according to the invention having an unexpected beneficially increased replication capability allowing replication of said oncolytic NDV in a human cancer cell preferably up to at least 10-fold higher levels than the oncolytic NDV parent strain MTH-68/H, thereby maintaining the useful oncological safety and efficacy specifications of the parent virus and advantageously supplementing it with the increased replication capability of an oncolytic NDV and the ability to express within infected cancer cells at least one of the before mentioned foreign genes, which beneficialls influence the immunological response towards the cancer cells. Herein an oncolytic NDV having such a mutation is also identified as NDV^{F277L} to discern it from a parent NDV having a phenylalanine at amino acid position 277 of the HN gene.

According to the present invention the recombinant and mutated NDV strains comprise in addition to the above described mutation in the HN gene, at least one mutation in the F gene and the thus encoded fusion protein. Still more preferred the mutated F gene encodes a fusion protein with an amino acid substitution at amino acid position 117 to an amino acid with a hydroxylated side chain, preferably where phenylalanine (F) is substituted to serine (S) at position 117 of the F gene product, and/or with an amino acid substitution at amino acid position 190 to an amino acid with an aliphatic amino acid side chain, preferably where phenylalanine (F) is substituted to leucine (L) at position 190 of the F gene product. The combination of both mutations at amino acid position 117 and 190 of the F gene is particularly preferred.

A NDV strain derived from NDV strain MTH-68/H having a mutation in the HN gene product or HN protein at position 277, wherein the amino acid phenylalanine (F) is substituted to leucine (L) at position 277, and having a mutation in the F gene resulting in a substitution of the amino acid phenylalanine (F) to the amino acid serine (S) at position 117 of the F gene product is referred to herein also as NDV-Mut HN(F277L)/F(F117S). A NDV strain derived from NDV strain MTH-68/H having a mutation in the HN gene product or HN protein at position 277, wherein the amino acid phenylalanine (F) is substituted to leucine (L) at position 277, and having a mutation in the F gene resulting in a substitution of the amino acid phenylalanine (F) to the amino acid leucine (L) at position 190 of the F gene product is referred to herein also as NDV-Mut HN(F277L)/F(F190L). A NDV strain having the mutation in the HN gene and both mutations in the F gene is referred to herein also as NDV-Mut HN(F277L)/F(F117S)/F(F190L). The foreign gene or part or respective variant thereof carried by this strain is referred to as Nivolumab, Ipilimumab, IL-12, NS1, Apoptin, or B18R in this shortcut for the strain. Within the shortcut for the recombinant NDV strains as used herein a reference to e.g. Nivolumab is intended to comprise the complete antibody, antigen-binding parts, and variants of the antibody or of antigen-binding parts, but also encompasses the more general form of an anti-PD-1-antibody and antigen-binding parts of this antibody. A reference to Ipilimumab is again intended to comprise the complete antibody, antigen-binding parts and variants of the antibody or antigen-binding parts, but also the more general form of an anti-CTLA-4-antibody, and antigen-binding parts of this antibody. A reference to IL-12 is intended to comprise the complete protein, parts and variants of the protein, as well as the more general form of a protein which improves the cellular immune response and the ability of T cells to enter tumor cells, or a respective part thereof. A reference to NS1 is intended to comprise the complete protein, parts and variants of the protein, as well as the more general form of a protein with the ability to modulate the virus replication cycle, or a respective part thereof. A reference to Apoptin is intended to comprise the complete protein, parts and variants of the protein, as well as the more general form of a protein with the ability to selectively induce apoptosis in human tumor cells, but not in normal human cells, or a respective part thereof. Finally, a reference to B18R in this shortcut is intented to comprise the complete protein, parts and variants of the protein, as well as the more general form of a protein that reduces or inhibits IFN expression such as an IFN-beta receptor, or a respective part thereof. The following NDV strains are provided by the present invention in a preferred embodiment:
- NDV-Mut HN(F277L)/F(F117S)/F(F190L)-Nivolumab
- NDV-Mut HN(F277L)/F(F117S)/F(F190L)-Ipilimumab
- NDV-Mut HN(F277L)/F(F117S)/F(F190L)-IL-12
- NDV-Mut HN(F277L)/F(F117S)/F(F190L)-NS 1
- NDV-Mut HN(F277L)/F(F117S)/F(F190L)-Apoptin
- NDV-Mut HN(F277L)/F(F117S)/F(F190L)-B18R

Within the meaning of the present invention the term "Newcastle disease virus" or "NDV" includes all known velogenic, mesogenic and lentogenic NDV strains. According to the present invention mesogenic and lentogenic strains are preferred. Still more preferred is the NDV derived from NDV strain MTH-68/H.

Within the meaning of the present invention "derived from NDV strain MTH-68/H" is intended to mean that the derived NDV strain is encoded by a viral genome or comprises a viral genome having a nucleic acid sequence with a sequence identity of at least 75 %, particularly at least 90 %, more particularly at least 95 % to the nucleic acid sequence of the NDV strain MTH-68/H. In one embodiment the sequence identity is at least 99 % or 100 %. Any foreign genes comprised in the recombinant NDV strains are not considered in the sequence alignment, and the reference sequence for the sequence alignment is that of SEQ ID No. 1 of the sequence listing. A sequence variation is for example the substitution, insertion or deletion of at least one nucleotide, preferably the substitution, insertion or deletion of up to 20 nucleotides, still more preferably of up to 15 nucleotides. Any combination of substitution, insertion and deletion is also comprised by that definition, provided that the final construct possesses the desired characteristics. The variation may result in at least one amino acid substitution, wherein each amino acid substitution can be a conservative or a non-conservative amino acid substitution.

It has been surprisingly shown that the mutation in the HN gene results in a beneficially improved replication capability of the respective strain as compared to an otherwise identical NDV not having said mutation in the HN gene. The beneficially increased replication rate of the NDV not only results in higher number of virus particles capable of infecting and destroying cancer cells, but also results in a higher expression of the foreign gene carried by the said recombinant NDV strains. As explained earlier, the respective gene products of the foreign gene results in an improved immunological response and thus an improved destruction of cancer cells.

Also it has been surprisingly shown that the at least one mutation, preferably both mutations (also referred to as "F2" herein), in the F gene at position 117 and 190 of the amino acid sequence of the resulting F protein beneficially improves the oncolytic potential of the respective NDV strains by improving the safety profile of the viruses in humans. The respective amino acid substitutions are as specified supra.

With regard to the viral safety profile, this in particular means that the intracerebral pathogenicity index (ICPI) is reduced by these mutations in the F gene, preferably F2, The ICPI value indicates the the pathogenicity of a Newcastle disease virus. Preferably, the ICPI is reduced by at least 10 %, more preferably by at least 20 % and still more preferred by at least 30 %, as compared to the identically determined ICPI value of a NDV strain not having the respective mutation(s) in the F gene. The ICPI can be determined according to the method set forth in World Organization for Animal Health (OIE) 2009, Chapter 2.3.14. Newcastle disease, pp. 576-589, in: Manual of Diagnostic Tests and Vaccines for Terrestrial Animals 2009, World Organization for Animal Health (OIE), Paris.

In a preferred embodiment of the present invention the recombinant and mutated NDV strains comprise in addition to the above described mutation in the HN gene, and the at least one mutation in the F gene at amino acid position 117 and/or 190, a mutation in the M gene and the thus encoded matrix protein. Still more preferred the mutated M gene encodes a matrix protein with an amino acid substitution at position 165 to an amino acid with an aromatic side chain, wherein still more preferred the amino acid glycine (G) at amino acid position 165 of the M gene is substituted to the amino acid tryptophane (W).

NDV strains comprising both a mutation in the HN gene and the M gene, as described supra, have been shown as being particularly suitable to infect cancer cells and to provide an enhanced replication rate within the cancer cells. Thus, according to one embodiment of the present invention a recombinant NDV strain comprises as a foreign gene at least one gene selected from the group consisting of a gene encoding an antibody directed to protein PD-1 or an antigen-binding part directed to protein PD-1 (anti-PD-1), preferably a gene encoding Nivolumab, an antigen-binding part or a variant of Nivolumab or its antigen-binding part, a gene encoding an antibody directed to the surface protein CTLA-4 or an antigen-binding part directed to protein CTLA-4 (anti-CTLA-4), preferably a gene encoding Ipilimumab, an antigen-binding part or a variant of Ipilimumab or its antigen-binding part, a gene encoding a protein which improves the cellular immune response and the ability of T cells to enter tumor cells, or a part thereof which improves the cellular immune response and the ability of T cells to enter tumor cells, preferably a gene encoding interleukin-12 (IL-12), a part of interleukin-12 or a variant of interleukin-12 or a part thereof, a gene encoding a protein with the ability to modulate the virus replication cycle, or a part thereof with the ability to modulate the virus replication cycle, preferably a gene encoding the non-structural protein NS1 of influenza A virus, a part of the non-structural protein NS1 of influenza A virus or a variant of the non-structural protein NS1 of influenza A virus or a part thereof, a gene encoding a protein with the ability to selectively induce apoptosis in human tumor cells, but not in normal human cells, or a part thereof with the ability to selectively induce apoptosis in human tumor cells, but not in normal human cells, preferably a gene encoding Apoptin (VP3 from chicken anemia virus), a part of Apoptin or a variant of Apoptin or a part thereof, a gene encoding a protein that reduces or inhibits IFN expression such as an IFN-beta receptor, or a part thereof that reduces or inhibits IFN expression such as an IFN-beta receptor, preferably a gene encoding the viral protein B18R from vaccinia virus, a part of the viral protein B18R from vaccinia virus or a variant of the viral protein B18R from vaccinia virus or a part thereof, and any combination thereof, and has a mutation in the HN gene, preferably resulting in a substitution of the amino acid phenylalanine (F) to leucine (L) at position 277 of the HN gene product, a mutation in the M gene, preferably resulting in a substitution of the amino acid glycine (G) to the amino acid tryptophane (W) at position 165 of the M gene product, and at least one mutation in the F gene, preferably resulting in a substitution of the amino acid phenylalanine (F) to the amino acid serine (S) at position 117 of the F gene product, and/or resulting in a substitution of the amino acid phenylalanine (F) to the amino acid leucine (L) at position 190 of the F gene product. The combination of both mutations at amino acid position 117 and 190 of the F gene is particularly preferred. As explained supra, other substitutions at these amino acid positions are also comprised within the present invention.

In a preferred embodiment of the present invention the recombinant and mutated NDV strains comprise in addition to the above described mutation in the HN gene and the at least one mutation in the F gene at amino acid positions 117 and/or 190 as specified above, at least one mutation in the L gene and the thus encoded RNA-dependent RNA polymerase protein, also referred to as L protein or L gene product herein. Still more preferred the mutated L gene encodes a RNA-dependent RNA polymerase protein with an amino acid substitution at position 757 to an amino acid with an aliphatic amino acid side chain other than valine (V), preferably where valine (V) is substituted to isoleucine (I) at position 757 of the L gene product, and/or with an amino acid substitution at position 1551 to an amino acid with a hydroxylated side chain, preferably where phenylalanine (F) is substituted to serine (S) at position 1551 of the L gene product, and/or with an amino acid substitution at position 1700 to an amino acid with an aliphatic side chain, preferably where arginine (R) is substituted to leucine (L) at position 1700 of the L gene product, preferably wherein the NDV comprises a mutated L gene and the encoded L protein with the amino acid substitution at position 757, position 1551 and position 1700. These recombinant NDV strains may in addition also comprise the above described mutation in the M gene.

Thus, according to one embodiment of the present invention a recombinant NDV strain comprises as a foreign gene at least one gene selected from the group consisting of a gene encoding an antibody directed to protein PD-1 or an antigen-binding part directed to protein PD-1 (anti-PD-1), preferably a gene encoding Nivolumab, an antigen-binding part or a variant of Nivolumab or its antigen-binding part, a gene encoding an antibody directed to the surface protein CTLA-4 or an antigen-binding part directed to protein CTLA-4 (anti-CTLA-4), preferably a gene encoding Ipilimumab, an antigen-binding part or a variant of Ipilimumab or its antigen-binding part, a gene encoding a protein which improves the cellular immune response and the ability of T cells to enter tumor cells, or a part thereof which improves the cellular immune response and the ability of T cells to enter tumor cells, preferably a gene encoding interleukin-12 (IL-12), a part of interleukin-12 or a variant of interleukin-12 or a part thereof, a gene encoding a protein with the ability to modulate the virus replication cycle, or a part thereof with the ability to modulate the virus replication cycle, preferably a gene encoding the non-structural protein NS1 of influenza A virus, a part of the non-structural protein NS1 of influenza A virus or a variant of the non-structural protein NS1 of influenza A virus or a part thereof, a gene encoding a protein with the ability to selectively induce apoptosis in human tumor cells, but not in normal human cells, or a part thereof with the ability to selectively induce apoptosis in human tumor cells, but not in normal human cells, preferably a gene encoding Apoptin (VP3 from chicken anemia virus), a part of Apoptin or a variant of Apoptin or a part thereof, a gene encoding a protein that reduces or inhibits IFN expression such as an IFN-beta receptor, or a part thereof that reduces or inhibits IFN expression such as an IFN-beta receptor, preferably a gene encoding the viral protein B18R from vaccinia virus, a part of the viral protein B18R from vaccinia virus or a variant of the viral protein B18R from vaccinia virus or a part thereof, and any combination thereof. The said recombinant NDV strain also comprises a mutation in the HN gene, preferably resulting in a substitution of the amino acid phenylalanine (F) to leucine (L) at amino acid position 277 of the HN gene, one or more of the above described mutations in the F gene, preferably resulting in a substitution of the amino acid phenylalanine (F) to the amino acid serine (S) at position 117 of the gene product and/or resulting in a substitution of the amino acid phenylalanine (F) to the amino acid leucine (L) at position 190 of the F gene product, and at least one mutation in the L gene, preferably one resulting at amino acid position 757 to an amino acid with an aliphatic amino acid side chain other than valine (V), preferably where valine (V) is substituted to isoleucine (I) at position 757 of the L gene product, and/or with an amino acid substitution at position 1551 to an amino acid with a hydroxylated side chain, preferably where phenylalanine (F) is substituted to serine (S) at position 1551 of the L gene product, and/or with an amino acid substitution at position 1700 to an amino acid with an aliphatic side chain, preferably where arginine (R) is substituted to leucine (L) at position 1700 of the L gene product, preferably wherein the NDV comprises a mutated L gene and the encoded L protein with the amino acid substitution at position 757, position 1551 and position 1700. These recombinant NDV strains may in addition also comprise the above described mutation in the M gene, preferably resulting in a substitution of the amino acid glycine (G) to the amino acid tryptophane (W) at position 165 of the M gene product. As explained supra, other substitutions at these amino acid positions are also comprised within the present invention.

A NDV strain derived from NDV strain MTH-68/H having a mutation in the HN gene, wherein the amino acid phenylalanine (F) is substituted to leucine (L) at amino acid position 277, having a mutation in the M gene, wherein the amino acid glycine (G) is substituted to the amino acid tryptophane (W) at position 165 of the M gene product, having two mutations in the F gene, wherein the amino acid phenylalanine (F) is substituted to the amino acid serine (W) at position 117 and the amino acid phenylalanine (F) is substituted to the amino acid leucine (L) at position 190 of the F gene product, and having three mutations in the L gene, wherein the amino acid valine (V) is substituted to the amino acid isoleucine (I) at position 757, the amino acid phenylalanine (F) is substituted to the amino acid serine (S) at position 1551, and the amino acid arginine (R) is substituted to the amino acid leucine (L) at position 1700 of the L gene product is herein referred to also as NDV-Mut HN(F277L)/M(G165W)/F(F117S)/F(F190L)/L(V757I)/L(F1551S)/L(R1700L) or MutHu2 or NDV-NoThaBene-2. The foreign gene or part or variant thereof carried by this strain is referred to as Nivolumab, Ipilimumab, IL-12, NS1, Apoptin, or B18R in this shortcut for the strain. Within these shortcuts for the recombinant NDV strains a reference to the respective foreign gene is intended to have the meaning as already defined above. The following NDV strains are provided by the present invention in a particularly preferred embodiment:
- NDV-Mut HN(F277L)/M(G165W)/F(F117S)/F(F190L)/L(V757I)/L(F1551S)/ L(R1700L)-Nivolumab,
- NDV-Mut HN(F277L)/M(G165W)/F(F117S)/F(F190L)/L(V757I)/L(F1551S)/ L(R1700L)-Ipilimumab,
- NDV-Mut HN(F277L)/M(G165W)/F(F117S)/F(F190L)/L(V757I)/L(F1551S)/ L(R1700L)-IL-12,
- NDV-Mut HN(F277L)/M(G165W)/F(F117S)/F(F190L)/L(V757I)/L(F1551S)/ L(R1700L)-NS1,
- NDV-Mut HN(F277L)/M(G165W)/F(F117S)/F(F190L)/L(V757I)/L(F1551S)/ L(R1700L)-Apoptin,
- NDV-Mut HN(F277L)/M(G165W)/F(F117S)/F(F190L)/L(V757I)/L(F1551S)/ L(R1700L)-B18R.

A recombinant NDV strain referred to as "NDV-Mut HN(F277L)/M(G165W)/F(F117S)/F(F190L)/L(V757I)/L(F1551S)/L(R1700L)-Nivolumab " is a NDV strain derived from NDV strain MTH-68/H having a mutation in the HN gene product at position 277, wherein the amino acid phenylalanine (F) is substituted to leucine (L) at position 277, and having a mutation in the M gene, wherein the amino acid glycine (G) is substituted to the amino acid tryptophane (W) at position 165 of the M gene product, having two mutations in the F gene, wherein the amino acid phenylalanine (F) is substituted to the amino acid serine (S) at position 117 and the amino acid phenylalanine (F) is substituted to the amino acid leucine (L) at position 190 of the F gene product, and having three mutations in the L gene, wherein the amino acid valine (V) is substituted to the amino acid isoleucine (I) at position 757, the amino acid phenylalanine (F) is substituted to the amino acid serine (S) at position 1551, and the amino acid arginine (R) is substituted to the amino acid leucine (L) at position 1700 of the L gene product, and carrying as a foreign gene a gene encoding an antibody directed to protein PD-1 or an antigen-binding part directed to protein PD-1 (anti-PD-1), preferably a gene encoding Nivolumab, an antigen-binding part or a variant of Nivolumab or its antigen-binding part.

The two mutations in the F gene at position 117 and 190 of the amino acid sequence of the resulting F protein and the three mutations in the L gene at position 757, 1551 and 1700 of the amino acid sequence of the resulting L protein, either alone or in combination, have been shown to beneficially improve the oncolytic potential of the respective NDV strains by improving the safety profile of the viruses in humans. The combinations include any combination of the referenced mutations in the F gene and L gene, and the F protein and L protein, respectively. That is any combination from two to five mutations. Particularly preferred is a combination of both mutations at amino acid position 117 and 190 of the F gene (also referred to as "F2" herein) and a combination of all five mutations, that is mutations at positions 117 und 190 of the F gene product and at positions 757, 1551 and 1700 of the L gene product (also referred to as "F2L3" herein). The respective amino acid substitutions are as specified supra. The F2 and F2L3 mutations result in a reduced intracerebral pathogenicity index (ICPI). Preferably, the ICPI is reduced by at least 10 %, more preferably by at least 20 % and still more preferred by at least 30 %, as compared to the identically determined ICPI value of a NDV strain not having the respective mutations in the F gene and the L gene.

According to another embodiment of the present invention, in addition to the mutation at position 277 of the HN gene product, the at least one mutation at positions 117 and/or 190 of F gene product, and preferably one or more of the above specified mutations at position 165 of the M gene product, and at positions 757 and/or 1551 and/or 1700 of the L gene product, a recombinant NDV according to the present invention may also comprise a further mutation in the F gene, which mutation is a mutation which encodes a fusion protein having an amino acid substitution at position 289, and which mutation is capable of improving the oncolytic potential of the NDV strain. Still more preferred, the amino acid at position 289 of the F gene product is substituted from leucine (L) to alanine (A). According to another embodiment of the present invention, alternatively or in addition to the mutation at position 289 of the F protein, a recombinant NDV according to the present invention may also comprise at least one further mutation in the L gene and the encoded L protein having an amino acid substitution at position 1717 to an amino acid with aromatic side chain other than tyrosine (Y), preferably where tyrosine (Y) is substituted to histidine (H) at position 1717 of the L gene product, and/or at position 1910 to an amino acid with a basic side chain, preferably where glutamic acid (E) is substituted to lysine (K) at position 1910 of the L gene product.

Alternatively or in addition a recombinant NDV according to the present invention may comprise a mutation in the RNA-editing sequence of the P gene. More preferably, the mutation abolishes and/or decreases the expression of the V protein.

Table 1a shows a list of mutations for obtaining NDV variants with increased titer production and/or good or improved safety profile according to the present invention. The mutation in the HN gene and at least one mutation in the F gene at amino acid position 117 or 190 is comprised in all NDV strains according to the present invention, while the mentioned mutations in the M gene, in the F gene at amino acid position 289, and L gene may or may not be present. If present, any possible combination of these mutations is encompassed by the present invention.

**Table 1a:**

| Gene | Relevant amino acid |
|---|---|
| HN | F277L |
| M | G165W |
| F | F117S |
| F | F190L |
| F | L289A |
| L | V757I |
| L | F1551S |
| L | R1700L |
| L | Y1717H |
| L | E1910K |

Table 1b shows particulary preferred NDV variants with increased titer production and/or improved safety profile, and the parent NDV strain MTH-68/H. "+" means that at the mentioned amino acid position the original (first mentioned) amino acid is substituted by the last mentioned amino acid, while "-" means that there is no amino acid substitution at the mentioned position, i.e. the first mentioned amino acid is present. For example and with regard to the HN gene "+" means that at amino acid position 277 phenylalanine (F) is substituted to leucine (L).

**Table 1b:**

| Gene | Relevant amino acid | MTH-68/H | | | | | | | | |
|---|---|---|---|---|---|---|---|---|---|---|
| HN | F277L | - | + | + | + | + | + | + | + | + |
| M | G165W | - | - | + | - | + | - | + | - | + |
| F | F117S | - | + | + | + | + | + | + | + | + |
| F | F190L | - | + | + | + | + | + | + | + | + |
| F | L289A | - | - | - | + | + | - | - | + | + |
| L | V757I | - | - | - | - | - | + | + | + | + |
| L | F1551S | - | - | - | - | - | + | + | + | + |
| L | R1700L | - | - | - | - | - | + | + | + | + |
| L | Y1717H | - | - | - | - | - | - | - | + | + |
| L | E1910K | - | - | - | - | - | - | - | + | + |

Table 1c shows all mutations in the F gene encompassed by the present invention. "+" means that at the mentioned amino acid position the original (first mentioned) amino acid is substituted by the last mentioned amino acid, while "-" means that there is no amino acid substitution at the mentioned position, i.e. the first mentioned amino acid is present. According to the present invention at least one mutation at amino acid position 117 or 190 is mandatory. The combination of both mutations is particularly preferred.

**Table 1c:**

| Gene | Relevant amino acid | 1 | 1 | 2 | 2 | 2 | 3 |
|---|---|---|---|---|---|---|---|
| F | F117S | + | - | + | + | - | + |
| F | F190L | - | + | + | - | + | + |
| F | L289A | - | - | - | + | + | + |

Table Id shows preferred mutations in the L gene encompassed by the present invention. "+" means that at the mentioned amino acid position the original (first mentioned) amino acid is substituted by the last mentioned amino acid, while "-" means that there is no amino acid substitution at the mentioned position, i.e. the first mentioned amino acid is present.

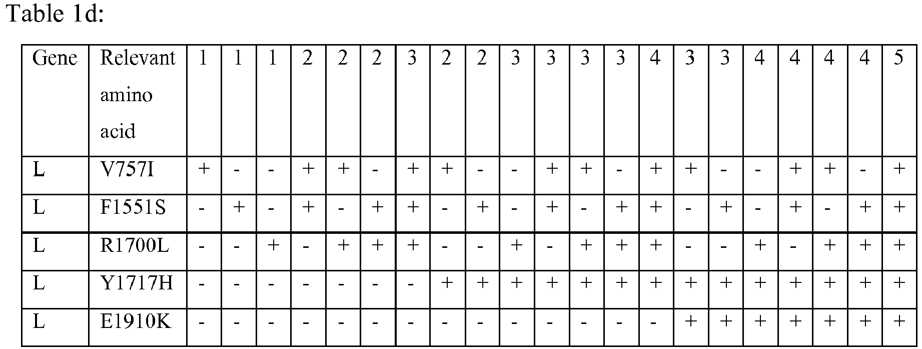

The NDV variants mentioned in tables 1a to Id carry as a foreign gene at least one gene selected from the group consisting of a gene encoding an antibody directed to protein PD-1 or an antigen-binding part directed to protein PD-1 (anti-PD-1), preferably a gene encoding Nivolumab, an antigen-binding part or a variant of Nivolumab or its antigen-binding part, a gene encoding an antibody directed to the surface protein CTLA-4 or an antigen-binding part directed to protein CTLA-4 (anti-CTLA-4), preferably a gene encoding Ipilimumab, an antigen-binding part or a variant of Ipilimumab or its antigen-binding part, a gene encoding a protein which improves the cellular immune response and the ability of T cells to enter tumor cells, or a part thereof which improves the cellular immune response and the ability of T cells to enter tumor cells, preferably a gene encoding interleukin-12 (IL-12), a part of interleukin-12 or a variant of interleukin-12 or a part thereof, a gene encoding a protein with the ability to modulate the virus replication cycle, or a part thereof with the ability to modulate the virus replication cycle, preferably a gene encoding the non-structural protein NS1 of influenza A virus, a part of the non-structural protein NS1 of influenza A virus or a variant of the non-structural protein NS1 of influenza A virus or a part thereof, a gene encoding a protein with the ability to selectively induce apoptosis in human tumor cells, but not in normal human cells, or a part thereof with the ability to selectively induce apoptosis in human tumor cells, but not in normal human cells, preferably a gene encoding Apoptin (VP3 from chicken anemia virus), a part of Apoptin or a variant of Apoptin or a part thereof, a gene encoding a protein that reduces or inhibits IFN expression such as an IFN-beta receptor, or a part thereof that reduces or inhibits IFN expression such as an IFN-beta receptor, preferably a gene encoding the viral protein B18R from vaccinia virus, a part of the viral protein B18R from vaccinia virus or a variant of the viral protein B18R from vaccinia virus or a part thereof, and any combination thereof. According to a particularly preferred embodiment the NDV strain carries as a foreign gene a gene encoding Nivolumab, an antigen-binding part or a variant of Nivolumab or its antigen-binding part.

In describing a protein or peptide formulation, structure and function herein, reference is made to amino acids. In the present specification, amino acid residues are expressed by using the following abbreviations. Also, unless explicitly otherwise indicated, the amino acid sequences of peptides and proteins are identified from N-terminal to C-terminal (left terminal to right terminal), the N-terminal being identified as a first residue. Amino acids are designated by their 3-letter abbreviation, 1-letter abbreviation, or full name, as follows. Ala : A : alanine; Asp : D : aspartic acid; Glu : E : glutamic acid ; Phe : F : phenylalanine; Gly : G : glycine; His : H : histidine; Ile : I : isoleucine; Lys : K : lysine; Leu : L : leucine; Met: M : methionine; Asn : N : asparagine; Pro : P: proline; Gln : Q : glutamine; Arg : R : arginine; Ser : S : serine; Thr : T : threonine; Val : V : valine; Trp : W : tryptophan; Tyr : Y : tyrosine; Cys : C : cysteine.

In a preferred embodiment the NDV and the recombinant NDV resulting thereof is encoded by and/or comprises at least one of the nucleic acids according to SEQ ID No. 1 to 7 or parts thereof. According to another preferred the recombinant NDV strains of the present invention have a sequence identity of at least 75 %, particularly of at least 90 %, more particularly of at least 95 % to any one of SEQ ID No. 1 to 8. The sequences given by these SEQ ID numbers are as follows:

| | |
|---|---|
| SEQ ID No. 1: | Nucleic acid (cDNA) sequence of the Newcastle disease virus (NDV) strain MTH-68/H genome; |
| SEQ ID No. 2: | Nucleic acid (cDNA) sequence of Newcastle disease virus strain Mut HN(F277L)/M(G165W)/F(F117S)/F(F190L)/L(V757I)/L(F1551S)/L(R1700L) genome having a mutation in the HN gene, a mutation in the M gene, two mutations in the F gene (F2) and three mutations in the L gene (L3) (= NDV-NoThaBene-2 genome); |
| SEQ ID No. 3: | Nucleic acid (cDNA) sequence of Newcastle disease virus strain Mut HN(F277L)/M(G165W)/F(F117S)/F(F190L)/L(V757I)/L(F1551S)/L(R1700L)-Nivolumab genome created by reverse genetics as disclosed herein, wherein the nucleic acid comprises a mutation in the HN gene, a mutation in the M gene, two mutations in the F gene (F2) and three mutations in the L gene (L3), and additionally encodes Nivolumab; |
| SEQ ID No. 4: | Nucleic acid (cDNA) sequence of Newcastle disease virus strain Mut HN(F277L)/M(G165W)/F(F117S)/F(F190L)/L(V757I)/L(F1551S)/L(R1700L)-Ipilimumab genome created by reverse genetics as disclosed herein, wherein the nucleic acid comprises a mutation in the HN gene, a mutation in the M gene, two mutations in the F gene (F2) and three mutations in the L gene (L3), and additionally encodes Ipilimumab; |
| SEQ ID No. 5: | Nucleic acid (cDNA) sequence of Newcastle disease virus strain Mut HN(F277L)/M(G165W)/F(F117S)/F(F190L)/L(V757I)/L(F1551S)/L(R1700L)-IL-12 genome created by reverse genetics as disclosed herein, wherein the nucleic acid comprises a mutation in the HN gene, a mutation in the M gene, two mutations in the F gene (F2) and three mutations in the L gene (L3), and additionally encodes IL-12; |
| SEQ ID No. 6: | Nucleic acid (cDNA) sequence of Newcastle disease virus strain Mut HN(F277L)/M(G165W)/F(F117S)/F(F190L)/L(V757I)/L(F1551S)/L(R1700L)-NS1 genome created by reverse genetics as disclosed herein, wherein the nucleic acid comprises a mutation in the HN gene, a mutation in the M gene, two mutations in the F gene (F2) and three mutations in the L gene (L3), and additionally encodes NS1; |
| SEQ ID No. 7: | Nucleic acid (cDNA) sequence of Newcastle disease virus strain Mut HN(F277L)/M(G165W)/F(F117S)/F(F190L)/L(V757I)/L(F1551S)/L(R1700L)-Apoptin genome created by reverse genetics as disclosed herein, wherein the nucleic acid comprises a mutation in the HN gene, a mutation in the M gene, two mutations in the F gene (F2) and three mutations in the L gene (L3), and additionally encodes Apoptin; |
| SEQ ID No. 8: | Nucleic acid (cDNA) sequence of Newcastle disease virus strain Mut HN(F277L)/M(G165W)/F(F117S)/F(F190L)/L(V757I)/L(F1551S)/L(R1700L)-B18R genome created by reverse genetics as disclosed herein, wherein the nucleic acid comprises a mutation in the HN gene, a mutation in the M gene, two mutations in the F gene (F2) and three mutations in the L gene (L3), and additionally encodes B18R. |

A person skilled in the art is well aware of the fact that the nucleobase thymine in the nucleic acid sequence of DNA is replaced by the nucleobase uracil in the nucleic acid sequence of RNA.

That is in a preferred embodiment of the present invention the viral HN gene product has an amino acid sequence as identified in SEQ ID No. 9, and/or the viral M gene product preferably has an amino acid sequence as identified in SEQ ID No. 10. In addition it is preferred that a NDV strain according to the present invention comprises beside the viral HN gene product having an amino acid sequence as identified in SEQ ID No. 9, and/or the viral M gene product preferably having an amino acid sequence as identified in SEQ ID No. 10, a viral F gene product having an amino acid sequence as identified in SEQ ID No. 11 and/or a viral L gene product having an amino acid sequence as identified in SEQ ID No. 12. Also encompassed by the present invention are NDV strains comprising variants of the gene products according to SEQ ID No. 9 to 12.

In another aspect the present invention also provides a recombinant nucleic acid comprising the nucleic acid sequence of the recombinant NDV strains of the present invention.

In describing nucleic acid formulation, structure and function herein, reference is made to any of a group of polymeric nucleotides such as DNA or RNA. Nucleic acid is composed of either one or two chains of repeating units called nucleotides, which nucleotides consist of a nitrogen base (a purine or pyrimidine) attached to a sugar phosphate. In the present specification, nucleotide residues are identified by using the following abbreviations. Adenine residue: A; guanine residue: G; thymine residue: T; cytosine residue: C; uracil residue: U. Also, unless explicitly otherwise indicated, the nucleotide sequences of nucleic acid are identified from 5'-terminal to 3'-terminal (left to right terminal), the 5'-terminal being identified as a first residue.

A nucleic acid according to the present invention comprises a transgenic construct. According to one embodiment the transgenic construct encodes an antibody directed to protein PD-1 or an antigen-binding part directed to protein PD-1 (anti-PD-1), preferably encoding Nivolumab, an antigen-binding part or a variant of Nivolumab or its antigen-binding part. According to another embodiment the transgenic construct encodes an antibody directed to the surface protein CTLA-4 or an antigen-binding part directed to protein CTLA-4 (anti-CTLA-4), preferably encoding Ipilimumab, an antigen-binding part or a variant of Ipilimumab or its antigen-binding part. According to another embodiment the transgenic construct encodes a protein which improves the cellular immune response and the ability of T cells to enter tumor cells, or a part thereof which improves the cellular immune response and the ability of T cells to enter tumor cells, preferably encoding interleukin-12 (IL-12), a part of interleukin-12 or a variant of interleukin-12 or a part thereof. According to another embodiment the transgenic construct encodes a protein with the ability to modulate the virus replication cycle, or a part thereof with the ability to modulate the virus replication cycle, preferably a gene encoding the non-structural protein NS1 of influenza A virus, a part of the non-structural protein NS1 of influenza A virus or a variant of the non-structural protein NS1 of influenza A virus or a part thereof. According to another embodiment the transgenic construct encodes a gene encoding a protein with the ability to selectively induce apoptosis in human tumor cells, but not in normal human cells, or a part thereof with the ability to selectively induce apoptosis in human tumor cells, but not in normal human cells, preferably encoding Apoptin (VP3 from chicken anemia virus), a part of Apoptin or a variant of Apoptin or a part thereof. According to another embodiment the transgenic construct encodes a protein that reduces or inhibits IFN expression such as an IFN-beta receptor, or a part thereof that reduces or inhibits IFN expression such as an IFN-beta receptor, preferably a gene encoding the viral protein B18R from vaccinia virus, a part of the viral protein B18R from vaccinia virus or a variant of the viral protein B18R from vaccinia virus or a part thereof.

According to a further embodiment of the present invention, the transgenic construct comprised in the nucleic acid may be a combination of two or more of the foreign genes or parts or variants thereof mentioned supra.

In addition to the transgenic construct a nucleic acid according to the present invention comprises a mutation in the HN gene, said mutation allowing replication of the NDV in a cancer cell to a higher level than replication of an otherwise identical NDV not having said the mutation in the HN gene.

In a preferred embodiment of the present invention the sequence encoding the recombinant NDV comprises a mutated HN gene encoding hemagglutinin-neuramidase with an amino acid substitution at position 277 to an amino acid with a hydrophobic side chain other than phenylalanine. Still more preferred the mutated HN gene encodes for a hemagglutinin-neuramidase referred to as HN^{F277L}. That is the amino acid phenylalanine (F) at position 277 of the hemagglutinin-neuramidase is substituted to leucine (L).

In addition a nucleic acid according to the present invention comprises at least one mutation in the F gene. Preferably the mutated F gene encodes a fusion protein with an amino acid substitution at position 117 and/or position 190. With regard to amino acid position 117 the amino acid is substituted to an amino acid with a hydroxylated side chain. Most preferred the mutated F gene encodes F^{F117S}. That is the amino acid phenylalanine (F) at position 117 of the F gene product is substituted to the amino acid serine (S) at the respective position. With regard to the amino acid substitution at position 190 the amino acid is changed to an amino acid with an aliphatic side chain.

Most preferred the mutated F gene encodes F^{F190L}. That is the amino acid phenylalanine (F) at position 190 of the F gene product is substituted to the amino acid leucine (L) at the respective position. The combination of boths mutations at amino acid position 117 (F^{F117S}) and 190 (F^{F190L}) is particularly preferred.

In a particular preferred embodiment the nucleic acid is derived from the NDV strain MTH-68/H.

In another embodiment of the present invention the sequence encoding the recombinant NDV may also comprises a mutation in the M gene. Preferably the mutated M gene encodes a matrix protein with an amino acid substitution at position 165 to an amino acid with an aromatic side chain. Most preferred the mutated M gene encodes M^{G165W}. That is the amino acid glycine (G) at position 165 of the M gene product is substituted to the amino acid tryptophane (W) at the respective position. In one embodiment of the present invention the mutation in the M gene is comprised in combination with the mutation in the HN gene as specified supra.

In a particular preferred embodiment the nucleic acid is derived from the NDV strain MTH-68/H and comprises a mutation in the HN gene resulting in an amino acid with a hydrophobic side chain other than phenylalanine at amino acid position 277, a mutation in the M gene resulting in a matrix protein with an amino acid substitution at position 165 to an amino acid with an aromatic side chain, and at least one mutation in the F gene resulting in a fusion protein with an amino acid substitution at position 117 to an amino acid with a hydroxylated side chain and/or an amino acid substitution at position 190 to an amino acid with an aliphatic side chain. Still more preferred the mutated HN gene encodes for a hemagglutinin-neuramidase in which the amino acid phenylalanine (F) at position 277 of the hemagglutinin-neuramidase is substituted to leucine (L), the optionally mutated M gene encodes for a matrix protein in which the amino acid glycine (G) at position 165 is substituted to the amino acid tryptophane (W), and the mutated F gene encodes for a fusion protein in which the amino acid phenylalanine (F) at position 117 is substituted to the amino acid serine (S) and/or the amino acid phenylalanine (F) at position 190 is substituted to the amino acid leucine (L). The combination of boths mutations at amino acid position 117 (F^{F117S}) and 190 (F^{F190L}) of the F gene is particularly preferred.

According to a further preferred embodiment of the present invention the sequence encoding the recombinant NDV comprises, in addition to the above described mutation in the HN gene, particulary the mutation resulting in an amino acid substitution at position 277 of the HN gene product as specified supra, and in addition to the above described at least one mutation in the F gene, particulary the mutation resulting in an amino acid substitution at position 117 (F^{F117S}) and/or at position 190 (F^{F190L}) of the F gene product as specified supra, optionally in combination with the above described mutation on the M gene, particulary the mutation resulting in an amino acid exchange at position 165 of the M gene product as specified supra, also comprises at least one mutation in the L gene. Preferably the mutated L gene encodes for a L protein with at least one amino acid substitution at amino acid position 757, 1551 and/or 1700. At position 757 the amino acid may be changed to an amino acid with an aliphatic side chain other than valine (V). Most preferred the mutated L gene encodes L^{V757I}. That is the amino acid valine (V) at position 757 of the L gene product is substituted to the amino acid isoleucine (I) at the respective position. At position 1551 the amino acid may be changed to an amino acid with a hydroxylated side chain. Most preferred the mutated L gene encodes L^{F1551S}. That is the amino acid phenylalanine (F) at position 1551 of the L gene product is substituted to the amino acid serine (S) at the respective position. At position 1700 the amino acid may be changed to an amino acid with an aliphatic side chain. Most preferred the mutated L gene encodes L^{R1700L}. That is the amino acid arginine (R) at position 1700 of the L gene product is substituted to the amino acid leucine (L) at the respective position. The mutations at amino acid positions 757, 1551 and 1700 of the L gene may be present alone or in each possible combination, i.e. position 757 and/or position 1551 and/or position 1700. The combination of all three mutations in the L gene (L3) is particularly preferred. In one embodiment of the present invention the at least one mutation in the L gene, preferably all three mutation at the respective amino acid positions 757, 1551 and 1700, is/are comprised in combination with the mutation in the HN gene as specified supra, the one or two mutations in the F gene as specified supra, and optionally, also in combination with the mutation in the M gene as specified supra.

In a particular preferred embodiment the nucleic acid is derived from the NDV strain MTH-68/H and comprises a mutation in the HN gene resulting in an amino acid with a hydrophobic side chain other than phenylalanine at position 277 of the respective amino acid sequence, a mutation in the M gene resulting in a matrix protein with an amino acid substitution at position 165 to an amino acid with an aromatic side chain, at least one mutation of the F gene resulting in a fusion protein with an amino acid substitution at position 117 to an amino acid with a hydroxylated side chain and/or an amino acid substitution at position 190 to an amino acid with an aliphatic side chain, and at least one mutation in the L gene resulting in a L protein with an amino acid substitution at position 757 to an amino acid with an aliphatic side chain other than valine (V), and/or an amino acid substitution at position 1551 to an amino acid with a hydroxylated side chain, and/or an amino acid substitution at position 1700 to an amino acid with an aliphatic side chain. More preferred, the mutated L gene results in a L protein comprising the three amino acid substitutions at positions 757, 1551 and 1700. Still more preferred the mutated HN gene encodes for a hemagglutinin-neuramidase in which the amino acid phenylalanine (F) at position 277 of the hemagglutinin-neuramidase is substituted to leucine (L), the optionally mutated M gene encodes for a matrix protein in which the amino acid glycine (G) at position 165 is substituted to the amino acid tryptophane (W), the mutated F gene encodes for a fusion protein in which the amino acid phenylalanine (F) at position 117 is substituted to the amino acid serine (S) and/or the amino acid phenylalanine (F) at position 190 is substituted to the amino acid leucine (L), and the mutated L gene encodes for a L protein in which the amino acid valine (V) at position 757 is substituted to the amino acid isoleucine (I), and/or the amino acid phenylalanine (F) at position 1551 is substituted to the amino acid serine (S), and/or the amino acid arginine (R) at position 1700 is substituted to the amino acid leucine (L). Still more preferred the L protein comprises all three mutations at positions 757, 1551 and 1700.

Also within the scope of the present invention are those nucleic acids comprising, in addition to the mutation in the HN gene and to the at least one mutation in the F gene at amino acid position 117 or 190, preferably in combination with one or more mutation in the M gene, and the L gene, as specified supra, a mutation in the F gene resulting in an amino acid substitution at position 289 of the amino acid sequence, and/or at least one mutation in the L gene resulting in an amino acid substitution at position 1717 and/or at position 1910 of the L proteine, and/or a mutation in the P gene as specified supra.

In one embodiment of the present invention the nucleic acid consists of or comprises at least one of the nucleic acids according to SEQ ID No. 2 to 8 or parts thereof. In another preferred embodiment the nucleic acid of the present invention has a sequence identity of at least 75 %, particularly of at least 90 %, more particularly of at least 95 % to any of the sequences according to SEQ ID No. 2 to 8.

In one embodiment of the present invention the nucleic acid is derived from a recombinant NDV as described supra.

The skilled artisan knows how to use the disclosed sequences to produce synthetic DNAs (e.g. vectors, in particular in rg-NDV vectors), and how to use the synthetic DNAs to reconstruct and express the viral genome. Thus, the skilled artisan is enabled to produce virus particles by using the information disclosed herein.

Recombinant viral genomes, which can be used to rescue virus particles, can for example be obtained by a method called 'reverse genetics'. Therefore, in another aspect the present invention also provides a method (herein also called 'reverse genetics') for providing cloned full-length cDNA of the recombinant NDV strains according to the present invention, and, in a further embodiment, the invention also provides infectious virus particles obtained from said full-length cDNA of the recombinant NDV strains according to the present invention. These recombinant NDV strains have an unexpected beneficial replication capability in cell cultures, eggs or animals that are used to propagate the virus for pharmaceutical purposes, and have good or even an improved viral safety profile, giving production advantages to such improved rgNDV strains. Furthermore, the higher replication rate is also particularly useful to obtain the desired oncolytic effects in vivo in that more virus particles will be produced for subsequent rounds of infection of cancer cells that were missed in the first round of infection. What is more, higher levels (yields) of the foreign gene or the foreign genes encoded by the transgenic construct will be achieved for providing the desired enhanced oncolytic effects in vivo. Thus, by modifying the HN protein within a transgene-expressing NDV higher yields of the transgenic construct protein can be obtained, which is very useful in anti-tumor immunotherapy. By modifying the F protein, and preferably also the L protein, as specified supra, also the viral safety can be improved.

The recombinant NDV strains according to the present invention can be obtained by a reverse genetic method for preparing an rgNDV encoding at least one foreign gene and having improved replication in a cancer cell, and good or even improved viral safety, over a parent NDV.

The method according to the present invention comprises providing a nucleic acid construct encoding a HN gene with a mutation, particularly wherein the mutation in the HN gene leads to a change in the expression of the hemagglutinin-neuraminidase, wherein the amino acid, particularly phenylalanine (F), in position 277 is substituted, particularly wherein the amino acid in position 277 is substituted to an amino acid with a hydrophobic side chain, more particularly wherein the amino acid in position 277 is substituted to leucine (L) at position 277 of the HN gene product / HN protein, and encoding a F gene with at least one mutation, particularly wherein the at least one mutation in the F gene encodes a fusion protein / F protein with an amino acid substitution at position 117 to an amino acid with a hydroxylated side chain and/or with an amino acid substitution at position 190 to an amino acid with an aliphatic side chain. Still more preferred is a substitution of the amino acid phenylalanine (F) at position 117 of the F gene product to the amino acid serine (S) and/or a substitution of the amino acid phenylalanine (F) at position 190 of the F gene product to the amino acid leucine (L). The combination of both mutations in the F gene is particularly preferred.

The method further comprises a step of providing a nucleic acid encoding a rgNDV further comprising a transgenic construct encoding an antibody directed to protein PD-1 or an antigen-binding part directed to protein PD-1 (anti-PD-1), preferably encoding Nivolumab, an antigen-binding part or a variant of Nivolumab or its antigen-binding part, an antibody directed to the surface protein CTLA-4 or an antigen-binding part directed to protein CTLA-4 (anti-CTLA-4), preferably a gene encoding Ipilimumab, an antigen-binding part or a variant of Ipilimumab or its antigen-binding part, a protein which improves the cellular immune response and the ability of T cells to enter tumor cells, or a part thereof which improves the cellular immune response and the ability of T cells to enter tumor cells, preferably a gene encoding interleukin-12 (IL-12), a part of interleukin-12 or a variant of interleukin-12 or a part thereof, a protein with the ability to modulate the virus replication cycle, or a part thereof with the ability to modulate the virus replication cycle, preferably a gene encoding the non-structural protein NS1 of influenza A virus, a part of the non-structural protein NS1 of influenza A virus or a variant of the non-structural protein NS1 of influenza A virus or a part thereof, a protein with the ability to selectively induce apoptosis in human tumor cells, but not in normal human cells, or a part thereof with the ability to selectively induce apoptosis in human tumor cells, but not in normal human cells, preferably a gene encoding Apoptin (VP3 from chicken anemia virus), a part of Apoptin or a variant of Apoptin or a part thereof, a protein that reduces or inhibits IFN expression such as an IFN-beta receptor, or a part thereof that reduces or inhibits IFN expression such as an IFN-beta receptor, preferably a gene encoding the viral protein B18R from vaccinia virus, a part of the viral protein B18R from vaccinia virus or a variant of the viral protein B18R from vaccinia virus or a part thereof, or any combination thereof.

In the next step of the claimed method the nucleic acid construct with said mutations in the HN gene and the at least one mutation in the F gene at amino acid position 117 or 190 (optionally with further mutations, in particular those as specified herein) is incorporated in the nucleic acid encoding a rgNDV comprising the transgenic construct in order to obtain a full length cDNA of the NDV, which additionally comprises a transgenic construct as specified supra. In a preferred embodiment this method step results in a nucleic acid as disclosed supra.

The nucleic acid encoding a rgNDV further comprising a transgenic construct as specified supra can be obtained by generating sub-genomic cDNA fragments and assembling the full length cDNA of the recombinant NDV from these fragments.

Following these method steps the thus obtained nucleic acid encoding a recombinant and mutated rgNDV according to the present invention is used to produce infectious rgNDV particles, which replication characteristics and expression rates of the encoded foreign protein(s) in cancer cells are compared with the replication characteristics and expressions rates of the parent NDV used for designing the recombinant and mutated NDV strain. Also the ICPI values of the produced infectious rgNDV particles are compared with the parent NDV.

Finally, said rgNDV is selected for further use, when it shows an improved replication characteristic over the parent NDV and good or even an improved viral safety, as well as a sufficient expression of the gene product or gene products of the at least one foreign genes.

If it is intended to introduce further to the mutation in the HN gene and the at least one mutation in the F gene at amino acid position 117 or 190, at least one further mutation in a viral gene, preferably the M gene, the F gene, the L gene and/or the P gene, the selected recombinant and mutated rgNDV comprising a transgenic construct as specified supra and a mutated HN gene is used for incorporating a nucleic acid construct encoding a mutated M gene, F gene, L gene or P gene, and the method for introducing the mutations in the HN gene and F gene is repeated respectively. Alternatively there is already provided a nucleic acid encoding a rgNDV with the transgenic construct and carrying a mutation in the M gene, F gene, L gene or P gene, particularly wherein said mutation is capable of improving oncolytic potential of said rgNDV and/or viral safety.

According to a preferred embodiment of the present invention the mutation in the M gene comprises or is a mutation which encodes a matrix protein with an amino acid substitution at position 165 to an amino acid with an aromatic side chain. Still more preferred is a substitution of the amino acid glycine (G) at position 165 of the M gene product to the amino acid tryptophane (W).

According to a further preferred embodiment of the present invention the F gene may also comprise a mutation which encodes a fusion protein with an amino acid substitution at position 289 to an amino acid with an aliphatic side chain other than leucine, preferably a substitution of the amino acid leucine (L) at position 289 of the F gene product to the amino acid alanine (A).

According to a still further preferred embodiment of the present invention the at least one mutation in the L gene comprises or is a mutation which encodes a L protein with an amino acid substitution at position 757 to an amino acid with an aliphatic side chain other than valine, and/or with an amino acid substitution at position 1551 to an amino acid with a hydroxylated side chain, and/or with an amino acid substitution at position 1700 to an amino acid with an aliphatic side chain. Still more preferred is a substitution of the amino acid valine (V) at position 757 of the L gene product to the amino acid isoleucine (I) and/or a substitution of the amino acid phenylalanine (F) at position 1551 of the L gene product to the amino acid serine (S), and/or a substitution of the amino acid arginine (R) at position 1700 of the L gene product to the amino acid leucine (L). Optionally, the L gene may also comprise a mutation which encodes a L protein with an amino acid substitution at position 1717 to an amino acid with an aromatic side chain other than tyrosine, preferably a substitution of the amino acid tyrosine (Y) at position 1717 of the L gene product to the amino acid histidine (H), and/or a mutation which encodes a L protein with an amino acid substitution at position 1910 to an amino acid with a basic side chain, preferably a substitution of the amino acid glutamic acid (E) at position 1910 of the L gene product to the amino acid lysine (K).

According to another preferred embodiment of the present invention the mutation is in the RNA-editing sequence of the P gene. More preferably, the mutation abolishes and/or decreases the expression of the V protein.

It is also within the scope of the present invention that the viral genome comprises a combination of two or more mutations, which are selected from a mutation in the HN gene, a mutation in the M gene, at least one mutation in the F gene, at least one mutation in the L gene, and a mutation in the P gene. For the details of the respective mutations, reference to the detailed disclosure of each of these mutations given supra and in tables 1 is made.

The invention also provides an isolated, recombinant nucleic acid encoding a rgNDV obtainable by a method for preparing an rgNDV having improved replication in a cancer cell in comparison to that of a parent NDV, as described supra.

In one preferred embodiment the isolated and recombinant nucleic acid encodes a rgNDV provided with a mutation in the HN gene resulting in a change of the amino acid phenylalanine (F) at amino acid position 277 of the hemagglutinin-neuraminidase into a leucine (L), said mutation (a F277L mutation) allowing replication of said rgNDV in a human cancer cell to a higher level than replication of an otherwise identical rgNDV having the amino acid phenylalanine (F) at position 277 in the HN gene product, and with at least one mutation in the F gene resulting in a change of the amino acid phenylalanine (F) at amino acid position 117 of the fusion protein into serine (S), and/or resulting in a change of the amino acid phenylalanine (F) at amino acid position 190 of the fusion protein into leucine (L), said at least one mutation providing a decreased ICPI value than the ICPI of an otherwise identical rgNDV strain not having the at least one mutation in the F gene. These mutations are provided in combination with a transgenic construct encoding any of the foreign genes or parts or variants as specified supra, more preferably Nivolumab or a part or variant thereof. The rgNDV may further comprise at least one or more mutation(s) selected from a mutation in the M gene, preferably G165W, a mutation at amino acid position 289 in the F gene, preferably L289A, a mutation at amino acid position 757, 1551, 1700, 1717 and/or 1910 in the L gene, preferably V757I, F1551S, R1700L, Y1717H and/or E1910K, and a mutation in the P gene, preferably the mutation in the P gene abolishes and/or decreases the expression of the V protein.

According to a particularly preferred embodiment the nucleic acid used to engineer a recombinant NDV genome is derived from the NDV strain MTH-68/H. Thus the present invention provides in one embodiment nucleic acids, preferably obtainable by the above specified method, which are
- rgNDV-Mut HN(F277L)/F(F117S)/F(F190L)-Nivolumab,
- rgNDV-Mut HN(F277L)/F(F117S)/F(F190L)-Ipilimumab,
- rgNDV-Mut HN(F277L)/F(F117S)/F(F190L)-IL-12,
- rgNDV-Mut HN(F277L)/F(F117S)/F(F190L)-NS1,
- rgNDV-Mut HN(F277L)/F(F117S)/F(F190L)-Apoptin,
- rgNDV-Mut HN(F277L)/F(F117S)/F(F190L)-B18R.

In another enmbodiment the present invention provides nucleic acids, preferably obtainable by the above specified method, which are
- rgNDV-Mut HN(F277L)/M(G165W)/F(F117S)/F(F190L)/L(V757I)/ L(F1551S)/L(R1700L)-Nivolumab (= rgNDV-NoThaBene-2-Nivolumab),
- rgNDV-Mut HN(F277L)/M(G165W)/F(F117S)/F(F190L)/L(V757I)/ L(F1551S)/L(R1700L)-Ipilimumab (= rgNDV-NoThaBene-2-Ipilimumab),
- rgNDV-Mut HN(F277L)/M(G165W)/F(F117S)/F(F190L)/L(V757I)/ L(F1551S)/L(R1700L)-IL-12 (= rgNDV-NoThaBene-2-IL-12),
- rgNDV-Mut HN(F277L)/M(G165W)/F(F117S)/F(F190L)/L(V757I)/ L(F1551S)/L(R1700L)-NS1 (= rgNDV-NoThaBene-2-NS1),
- rgNDV-Mut HN(F277L)/M(G165W)/F(F117S)/F(F190L)/L(V757I)/ L(F1551S)/L(R1700L)-Apoptin (= rgNDV-NoThaBene-2-Apoptin),
- rgNDV-Mut HN(F277L)/M(G165W)/F(F117S)/F(F190L)/L(V757I)/ L(F1551S)/L(R1700L)-B18R (= rgNDV-NoThaBene-2-B18R).

A NDV or rgNDV according to the present invention can be provided in a suitable cell or cell line, for example a HeLa cell line or in cancer cells, or an embryonated egg that is susceptible to a NDV infection.

In these infected cells the virus is grown to sufficient quantities, and preferably under sufficient conditions such that the virus is free from exogenous contamination, and the progeny virus particles are collected by suitable methods well known to those skilled in the art.

In a further aspect the present invention is concerned with the medical use of the recombinant NDV strains (the virus particles) of the present invention. In this regard the invention provides an oncolytic NDV for use in oncological treatment (this term is used herein also replaceable with "for use as a medicament, especially in a method of treatment of cancer"; or the use of said oncolytic NDV in the preparation of a pharmaceutical formulation for use in said method of treatment) in humans (or more generically animals, such as mammalian animals).

In this regard the present invention is first concerned with a recombinant NDV according to the present invention for use in medicine. Secondly, the present invention is more particulary concerned with a recombinant NDV according to the present invention for use in a method of treating cancer in a subject considered in need thereof.

In particular one or more of the recombinant and mutated NDV strains of the present invention are used in a method for treating one or more indications selected from the group consisting of brain tumors, like glioblastoma, bone tumors, like osteosarcoma and/or Ewing's sarcoma, soft tissue tumors, like rhabdomyosarcoma, gynecological tumors, like breast cancer, ovary cancer and/or cervix cancer, gastrointestinal tumors, like esophageal tumors, stomach tumors, colon tumors, pancreas tumors, prostate tumors, lung tumors, ear, nose, throat tumors, tongue tumors, and skin tumors, like melanoma.

In a preferred embodiment of the present invention the subject to be treated is a mammal, particularly a mammalian animal or a human subject. Still more preferred the one or more oncolytic NDV strains of the present invention may be used for the treatment of adults and/or children, preferably human adults and/or human children.

In one embodiment only one recombinant and mutated NDV or rgNDV strain according to the present invention is used in a method for treating cancer, in particular a cancer selected from the specific cancers as described and/or mentioned in the claims herein. Alternatively in another preferred embodiment of the present invention a combination of at least two of the recombinant and mutated NDV or rgNDV strains according to the present invention is used in a method for treating cancer, in particular a cancer selected from the specific cancers as described and/or mentioned in the claims herein. In particular it is preferred to use a combination of virus particles, wherein each virus particle encodes and expresses another foreign gene, part or variant thereof selected from the group of the above specified foreign genes or parts or variants of the present invention. The use of such a combination of foreign genes results in an active combination of proteins beneficially improving the immunological response with the cancer.

Also within the scope of the present invention is the use of one or more NDV or rgNDV strains according to the present invention in combination with one or more other therapies suitable for the treatment of cancer. In a preferred embodiment the one or more NDV or rgNDV strains or a pharmaceutical formulation comprising the same is used in a method for treating cancer, especially a cancer selected from the specific cancers as described and/or mentioned in the claims herein.

The one or more NDV or rgNDV strains and one or more other therapies can be used concurrently or sequentially. In certain embodiments, the one or more NDV or rgNDV and the one or more other therapies are administered in the same ("fixed") pharmaceutical formulation. In other embodiments, the one or more NDV or rgNDV strains and the one or more other therapies are administered in different formulations. The one or more NDV or rgNDV strains of the present invention and the one or more other therapies can be administered by the same or different routes of administration to the subject considered in need thereof. Another therapy within the meaning of the present invention also comprises an administration of other oncolytical virus strains, for example recombinant NDV or rgNDV strains encoding and expressing foreign genes other than those as disclosed within the present invention. Another foreign gene expressed by the NDV or rgNDV may be selected from the group consisiting of a gene encoding an antibody directed to protein PD-L1 (anti-PD-Ll), preferably a gene encoding Atezolizumab, a gene encoding an antibody directed to the growth factor protein VEGF-A (anti-VEGF-A), preferably a gene encoding Bevacizumab, a gene encoding an antibody directed to killer-cell immunoglobulin-like receptors (anti-KIR), preferably a gene encoding Lirilumab, a gene encoding an antibody directed to and inhibiting LAG-3 (anti-LAG-3), preferably a gene encoding Relatlimab, a gene encoding an antibody directed to NKG2A (anti-NKG2A), preferably a gene encoding Monalizumab, a gene encoding an antibody directed to the glucocorticoid-induced TNF-superfamily receptor (anti-GITR), preferably a gene encoding TRX518, a gene encoding an antibody directed to and activating the protein OX40 (anti-OX40), preferably a gene encoding BMS 986178, a gene encoding a membrane protein which is the receptor for the proteins CD28 and CTL-4, and which membrane protein is involved in the costimulatory signal essential for T-lymphocyte activation, preferably a gene encoding CD80, a gene encoding a human interleukin 12 (hIL-12), preferably a gene encoding (ns)hIL-12, a gene encoding a green fluorescent protein, preferably a gene encoding eGFP, any antigen-binding part of these antibodies, any parts of these other proteins, variants of these antibodies and these other proteins, and variants of these antigen-binding parts or parts. Particularly preferred NDV strains and rgNDV which may be used in combination are disclosed in the copending European patent application no. 18166400.4 and the copending European patent application no. 19154204.2. For details of these strains reference to these patent applications is made.

Particulary preferred is the combined use of a recombinant NDV or rgNDV strain encoding and expressing as foreign genes a gene encoding an antibody directed to protein PD-1 or an antigen-binding part directed to protein PD-1 (anti-PD-1), preferably a gene encoding Nivolumab, an antigen-binding part of Nivolumab, a variant of Nivolumab or a variant of an antigen-binding part of Nivolumab, a recombinant NDV or rgNDV strain encoding and expressing as foreign genes a gene encoding an antibody directed to the surface protein CTLA-4 or an antigen-binding part directed to protein CTLA-4 (anti-CTLA-4), preferably a gene encoding Ipilimumab, an antigen-binding part of Ipilimumab, a variant of Ipilimumab or a variant of an antigen-binding part of Ipilimumab, and a recombinant NDV or rgNDV strain encoding and expressing as foreign genes a gene encoding an antibody directed to protein PD-L1 or an antigen-binding part directed to protein PD-L1 (anti-PD-Ll), preferably a gene encoding Atezolizumab, an antigen-binding part of Atezolizumab, a variant of Atezolizumab or a variant of an antigen-binding part of Atezolizumab. Such a combination results in decreased side effects, as compared to directly administering the respective proteins to a patient in need thereof. For example the combination of the following NDV strains may be used in accordance with the present invention:
- NDV-Mut HN(F277L)/M(G165W)-Nivolumab,
- NDV-Mut HN(F277L)/M(G165W)-Ipilimumab, and
- NDV-Mut HN(F277L)/M(G165W)/F(F117S)/F(F190L)/L(V757I)/ L(F1551S)/L(R1700L)-Atezolizumab
or
- NDV-Mut HN(F277L)/M(G165W)/F(F117S)/F(F190L)/L(V757I)/ L(F 1551 S)/L(R1700L)-Nivolumab,
- NDV-Mut HN(F277L)/M(G165W)/F(F117S)/F(F190L)/L(V757I)/ L(F1551S)/L(R1700L)-Ipilimumab, and
- NDV-Mut HN(F277L)/M(G165W)/F(F117S)/F(F190L)/L(V757I)/ L(F 1551 S)/L(R1700L)-Atezolizumab

In a further aspect the present invention also provides a pharmaceutical formulation comprising a recombinant and mutated NDV strain, preferably an rgNDV, as described herein, without or preferably with at least one pharmaceutically acceptable carrier material.

For the indications mentioned herein, the appropriate dosage will, of course, vary depending upon, for example, the particular molecule of the invention to be employed, the mode of administration and the nature and severity of the condition being treated. In general, the dosage, in one embodiment of the invention, can preferably be in the range of 10⁷ to 10⁹ pfu per dose.

The recombinant and mutated NDV strains according to the present invention provide on the one hand an improved replication capacity of the NDV particles in cancer cells and a good or even an improved safety profile, preferably in humans, which is associated with increased cancer cell lysis and increased anti-cancer activitity, because the recombinant and mutated NDV strains of the present invention are still selective for cancer cells. The low to sero activity towards normal cells is associated with an increased therapeutic window or safety margin, as is commonly determined for cancer therapeutics. Beside the virological activity the recombinant mutated NDV strains of the present invention are in addition beneficial for the treatment of cancer from an immunological point of view. This is because the foreign gene carried by NDV strains of the present invention beneficially influences the ability of the immune system of the subject to be treated to attack and destroy cancer cells. Advantageuously the foreign gene is expressed inside the tumor to be treated, that is directly in the side of need.

The pharmaceutical formulations or pharmaceutical compositions of the present invention may be manufactured in any conventional manner, which are known to those skilled in the art. For example a pharmaceutical composition according to the present invention comprising a recombinant virus particle as decribed within this description can be provided in a dried, preferably in a lyophilized form and can be complemented with a suitable solvent, e.g. an aqueous carrier for injection at the time when used for administration. The dried form is very stable. Thus, in one embodiment the pharmaceutical composition is present as a dried form, still more preferred in a lyophilized form, and is reconstituted before administration by adding a suitable solvent. That is the present invention also relates to the above described dried form of the pharmaceutical composition for use as a medicament, preferably for use in a method of treating cancer. The process parameters for drying, preferably lyophilisation must be chosen such that the dried/lyophilized product contains live, replication competent viral particles, which can be grown to standardized titers, when needed. A person skilled in the art knows how to set these parameters.

For administration of a dried form, the dried form is directly dissolved in a suitable solvent or pharmaceutically acceptable carrier for injection and/or stabilization of the NDV or rg NDV and/or for improving its delivery to cancer cells. The solvent or carrier is preferably an aqueous carrier, for example sterile water for injection or sterile buffered physiological saline or another, preferably isotonic buffer system having a pH in the range of 7.2 to 7.6. The reconstitution can be performed ideally at or close to the intended time of administration in order to avoid any contaminations with microbes, etc. The skilled person is familiar with the handling of pharmaceutical compositions for reconstitution and reconstituted solutions.

In another embodiment, the present invention is concerned with pharmaceutical formulations comprising cancer cells infected with a transgene-expressing NDV or transgene-expressing rgNDV strain as described herein, and a pharmaceutically acceptable carrier. In specific embodiments, the cancer cells have been treated with gamma radiation prior to incorporation into the pharmaceutical formulation. In specific embodiments, the cancer cells have been treated with gamma radiation before infection with the NDV strain (e.g., rgNDV). In other specific embodiments, the cancer cells have been treated with gamma radiation after infection with the NDV (e.g., rgNDV). In another embodiment, presented herein are pharmaceutical formulations comprising a protein concentrate from lysed NDV-infected cancer cells (e.g., rgNDV infected cancer cells), and a pharmaceutically acceptable carrier.

The pharmaceutical formulation of the present invention can be prepared by a method comprising or consisting of the steps of:
- propagating an NDV, preferably an rgNDV, according to the present invention in at least one cell or embryonated egg that is susceptible to a NDV infection;
- collecting the progeny virus particles, wherein the virus is grown to sufficient quantities and under sufficient conditions that the virus is free from exogenous contamination, such that the progeny virus is suitable for formulation into a pharmaceutical formulation; and
- preferably manufacturing a pharmaceutical formulation comprising said progeny virus particles in the absence of or after addition of at least one pharmaceutically acceptable carrier material.

Thus in a further aspect the present invention also provides a method for producing the above mentioned pharmaceutical formulation. It is also within the scope of the present invention that for manufacturing the said pharmaceutical formulation a nucleic acid according to the present invention is used, from which virus particles can be rescued which are then propagated in at least one cell or embryonated egg that is susceptible to a NDV infection.

The step of collecting progeny virus particles may in one embodiment also comprise a step of processing the collected virus-containing material to enrich virus particles and/or to eliminate host cell DNA.

In a further aspect the present invention also provides a cell or cell line or an embryonated egg comprising a NDV, preferably a rgNDV, according to the present invention.

In still a further aspect the present invention is also concerned with a method for treating cancer, especially a cancer selected from the specific cancers as described and/or mentioned in the claims herein, in a subject considered in need thereof. The method for treating cancer utilizes a transgene-expressing NDV or transgene-expressing rgNDV described herein or any combination thereof or a pharmaceutical formulation comprising such a NDV or rgNDV or any combination thereof, especially in a therapeutically effective amount. Within the meaning of the present patent application a therapeutically effective amount also includes an effective amount for preventing a cancer, in particular a cancer selected from the specific cancers as described and/or mentioned in the claims herein.

The method of treatment comprises or consists of administering to a subject in need thereof a NDV or an rgNDV according to the present invention or any combination thereof in a sufficient amount for infecting and destroying some or all of the cancer cells. In a specific embodiment the method for treating cancer comprises or consists of infecting a cancer cell in a subject with a NDV or rgNDV of the present invention or any combination thereof or with a pharmaceutical composition of the present invention. In another embodiment the one or more NDV or rgNDV or a pharmaceutical composition comprising the same is administered to a subject in need thereof by intravenous, intra-arterial, intratumoral, intramuscular, intradermal, subcutaneous, or any other medically relevant route of administration. According to another embodiment of the present invention the NDV or rgNDV according to the present invention or any combination thereof is administering to a subject in need thereof by administering cancer cells infected with a NDV or rgNDV according to the present invention, especially in a therapeutically effective amount, or pharmaceutical formulation thereof by intravenous, intra-arterial, intratumoral, intramuscular, intradermal, subcutaneous, or any other medically relevant route of administration. In specific embodiments, the cancer cells have been treated with gamma radiation prior to administration to the subject or incorporation into the pharmaceutical formulation. In still another embodiment, a method for treating cancer comprises or consists of administering to a subject in need thereof protein concentrates or plasma membrane fragments from cancer cells infected with a NDV or rgNDV or a pharmaceutical formulation according to the present invention.

In a particular preferred embodiment the method for treating a cancer, in particular a cancer selected from the specific cancers as described and/or mentioned in the claims herein, comprises administering a mixture of at least two recombinant and mutated NDV or rgNDV strains according to the present invention. In particular it is preferred to use a combination of virus particles, wherein each virus particle encodes and expresses another foreign gene selected from the group consisting of a gene encoding an antibody directed to protein PD-1 or an antigen-binding part directed to protein PD-1 (anti-PD-1), preferably a gene encoding Nivolumab, an antigen-binding part or a variant of Nivolumab or its antigen-binding part, encoding an antibody directed to the surface protein CTLA-4 or an antigen-binding part directed to protein CTLA-4 (anti-CTLA-4), preferably a gene encoding Ipilimumab, an antigen-binding part or a variant of Ipilimumab or its antigen-binding part, encoding a protein which improves the cellular immune response and the ability of T cells to enter tumor cells, or a part thereof which improves the cellular immune response and the ability of T cells to enter tumor cells, preferably a gene encoding interleukin-12 (IL-12), a part of interleukin-12 or a variant of interleukin-12 or a part thereof, encoding a protein with the ability to modulate the virus replication cycle, or a part thereof with the ability to modulate the virus replication cycle, preferably a gene encoding the non-structural protein NS1 of influenza A virus, a part of the non-structural protein NS1 of influenza A virus or a variant of the non-structural protein NS1 of influenza A virus or a part thereof, encoding a protein with the ability to selectively induce apoptosis in human tumor cells, but not in normal human cells, or a part thereof with the ability to selectively induce apoptosis in human tumor cells, but not in normal human cells, preferably a gene encoding Apoptin (VP3 from chicken anemia virus), a part of Apoptin or a variant of Apoptin or a part thereof, encoding a protein that reduces or inhibits IFN expression such as an IFN-beta receptor, or a part thereof that reduces or inhibits IFN expression such as an IFN-beta receptor, preferably a gene encoding the viral protein B18R from vaccinia virus, a part of the viral protein B18R from vaccinia virus or a variant of the viral protein B18R from vaccinia virus or a part thereof, and any combination thereof. The administration of such a combination of foreign genes results in an active combination of proteins beneficially improving the immunological response with the cancer. The administration of this mixture of NDVs or rgNDVs according to the present invention can be achieved as specified supra.

Also within the scope of the present invention is a method of treating cancer, especially a cancer selected from the specific cancers as described and/or mentioned in the claims herein, which utilizes a NDV or rgNDV according to the present invention in combination with one or more other therapies. In a preferred embodiment the NDV or rgNDV or a pharmaceutical formulation comprising the same is administered to a subject in need thereof in a therapeutically effective amount. The NDV or rgNDV and one or more other therapies can be administered concurrently or sequentially to the subject (meaning they are jointly therapeutically active). In certain embodiments, the NDV or rgNDV and one or more other therapies are administered in the same ("fixed") pharmaceutical formulation. In other embodiments, the NDV or rgNDV and one or more other therapies are administered in different formulations. The NDV or rgNDV of the present invention and one or more other therapies can be administered by the same or different routes of administration to the subject considered in need thereof. Another therapy within the meaning of the present invention also comprises an administration of other oncolytical virus strains, for example recombinant NDV or rgNDV strains encoding and expressing foreign genes other than those as disclosed within the present invention. Another foreign gene expressed by the NDV or rgNDV may be selected from the group consisiting of a gene encoding an antibody directed to protein PD-L1 (anti-PD-Ll), preferably a gene encoding Atezolizumab, a gene encoding an antibody directed to the growth factor protein VEGF-A (anti-VEGF-A), preferably a gene encoding Bevacizumab, a gene encoding an antibody directed to killer-cell immunoglobulin-like receptors (anti-KIR), preferably a gene encoding Lirilumab, a gene encoding an antibody directed to and inhibiting LAG-3 (anti-LAG-3), preferably a gene encoding Relatlimab, a gene encoding an antibody directed to NKG2A (anti-NKG2A), preferably a gene encoding Monalizumab, a gene encoding an antibody directed to the glucocorticoid-induced TNF-superfamily receptor (anti-GITR), preferably a gene encoding TRX518, a gene encoding an antibody directed to and activating the protein OX40 (anti-OX40), preferably a gene encoding BMS 986178, a gene encoding a membrane protein which is the receptor for the proteins CD28 and CTL-4, and which membrane protein is involved in the costimulatory signal essential for T-lymphocyte activation, preferably a gene encoding CD80, a gene encoding a human interleukin 12 (hIL-12), preferably a gene encoding (ns)hIL-12, a gene encoding a green fluorescent protein, preferably a gene encoding eGFP, any antigen-binding part of these antibodies, any parts of these other proteins, variants of these antibodies and these other proteins, and variants of these antigen-binding parts or parts. Particularly preferred NDV strains and rgNDV which may be used in combination are disclosed in the copending European patent application no. 18166400.4 and the copending European patent application no. 19154204.2. For details of these strains reference to these patent applications is made. Particulary preferred is the combined use of recombinant NDV or rgNDV strains encoding and expressing as foreign genes anti-PD-1, preferably Nivolumab, anti-CTLA-4, preferably Ipilimumab, anti-PD-Ll, preferably Atezolizumab, or parts or variants thereof. For example the following combinations are in accordance with the present invention:
- NDV-Mut HN(F277L)/M(G165W)-Nivolumab,
- NDV-Mut HN(F277L)/M(G165W)-Ipilimumab, and
- NDV-Mut HN(F277L)/M(G165W)/F(F117S)/F(F190L)/L(V757I)/ L(F1551S)/L(R1700L)-Atezolizumab
or
- NDV-Mut HN(F277L)/M(G165W)/F(F117S)/F(F190L)/L(V757I)/ L(F 1551 S)/L(R1700L)-Nivolumab,
- NDV-Mut HN(F277L)/M(G165W)/F(F117S)/F(F190L)/L(V757I)/ L(F1551S)/L(R1700L)-Ipilimumab, and
- NDV-Mut HN(F277L)/M(G165W)/F(F117S)/F(F190L)/L(V757I)/ L(F 1551 S)/L(R1700L)-Atezolizumab

Another therapy may also comprise or may be radiotherapy for cancer.

### Figures

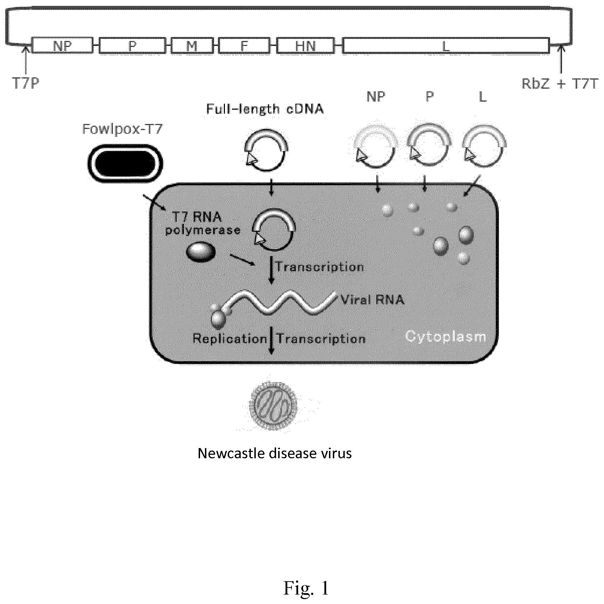

Figure 1 is a schematic presentation of the NDV reverse genetics system. The upper part shows the composition of the full-length cDNA plasmid which contains the full-length NDV cDNA (encoding the nucleocapsid protein (NP), the phosphoprotein (P), the matrix protein (M), the fusion protein (F), the hemagglutinin-neuraminidase (HN), and the RNA-dependent RNA polymerase (L)) cloned behind the bacteriophage T7 RNA Polymerase promoter (T7P; yellow triangle) and followed by a ribozyme sequence (Rz) and T7 transcription termination signal (T7T).
   A suitable host cell (shaded round-cornered box) is infected with a recombinant Fowlpox virus that expresses T7 DNA-dependent RNA polymerase (Fowlpox-T7) and subsequently co-transfected with the full-length cDNA plasmid and three helper plasmids containing the genes encoding the NDV NP, P and L proteins, respectively. Transcription of the full-length cDNA results in the generation of the NDV antigenome RNA which is encapsidated by NP protein then transcribed and replicated by the RNA-leading to the generation of infectious NDV.

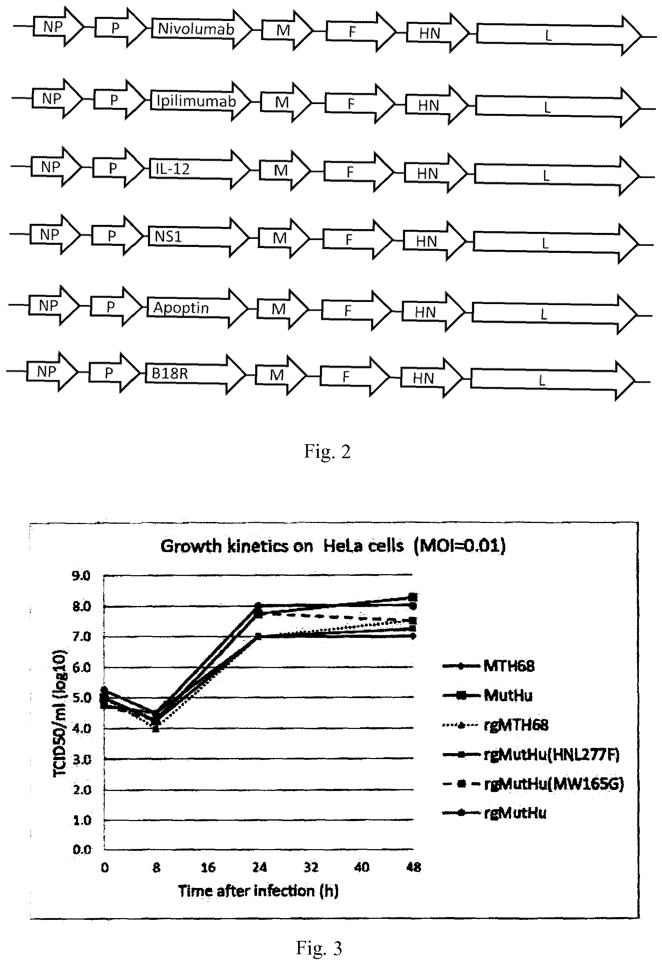

Figure 2 is a schematic presentation of the genome of the rgNDV-mutants according to the present invention. The figure shows the position of the respective foreign gene which is inserted as extra transcription unit into the rgNDV-mutant genome between the P and M genes.
Figure 3 shows growth kinetics in HeLa cells. HeLa cells were infected at a multiplicity of infection (MOI) of 0.01 with the indicated viruses. At 0h, 8h, 24h and 48h after infection, the amount of infectious virus in the supernatant was determined by end-point titration on QM5 cells. MTH68: NDV strain MTH-68/H; MutHu: NDV-Mut HN(F277L)/M(G165W); rgMTH68: NDV strain MTH-68/H derived by reverse genetics from cloned full-length cDNA; rgMutHu: NDV strain NDV-Mut HN(F277L)/M(G165W) derived by reverse genetics from cloned full-length cDNA; rgMutHu(HNL277F): rgMutHu in which the amino acid mutation at position 277 in the HN gene was converted from L back to F; rgMutHu(MW165G): rgMutHu in which the amino acid mutation at position 165 in the M gene was converted from W back to G.

### Examples

### Example 1. Nucleotide sequence analysis of mutant NDV-Mut HN(F277L)/M(G165W).

We identified a spontaneous mutant of an oncolytic NDV strain MTH-68/H (Csatary et al., 1999, Anticancer Res. 19:635-638.; further called MTH68). The replication capacity of the mutant strain (designated NDV-Mut HN(F277L)/M(G165W) in a variety of human neoplastic cell lines, as well as autologous primary tumors, is greatly enhanced as compared to the original MTH-68/H strain (also referred to as MTH68 strain). We analyzed its nucleotide sequence and found that, compared to MTH68, NDV-HN(F277L)/M(G165W) has two nucleotide mutations, one leading to an amino acid substitution in the M protein (G165W) and the other in the HN protein (F277L).

### Example 2. A reverse genetics system that allows genetic modification of NDV-strains.

### 2.1 Reverse genetics

In order to be able to genetically modify the genome of an RNA virus such as NDV, a manipulatable genetic system must be developed that uses a copy of the full viral RNA (vRNA) genome in the form of DNA. This full-length cDNA is amenable to genetic modification by using recombinant DNA techniques. The authentic or modified cDNA can be converted back into vRNA in cells, which in the presence of the viral replication proteins results in the production of a new modified infectious virus. Such 'reverse genetics systems' have been developed in the last few decades for different classes of RNA viruses. This system enables the rapid and facile introduction of mutations and deletions and the insertion of a transgene transcriptional unit, thereby enabling the changing of the biological properties of the virus.

Reverse genetics systems for several NDV strains, including lentogenic as well as velogenic strains, were developed by the Central Veterinary Institute (CVI), part of Wageningen University and Research, currently Wageningen Bioveterinary Research (WBVR) under the supervision of Dr. Ben Peeters (Peeters et al., 1999, J. Virol. 73:5001-9; de Leeuw et al., 2005, J. Gen. Virol. 86:1759-69; Dortmans et al., 2009, J. Gen. Virol. 90:2746-50). In order to generate a reverse genetics system for providing the NDV nucleic acids and strains according to the present invention, a similar approach was used. Details of the procedure can be found in the above cited papers and in the paragraphs below. Briefly, the system consists of 4 components, i.e., a transcription plasmid containing the full-length (either authentic or genetically modified) cDNA of the virus, which is used to generate the vRNA, and 3 expression plasmids ('helper plasmids') containing the NP, P and L genes of NDV respectively, which are used to generate the vRNA-replication complex (consisting of NP, P and L proteins). Transcription of the cDNA (i.e. conversion of the cDNA into vRNA) and expression of the NP, P and L genes by the helper plasmids is driven by a T7 promoter. The corresponding T7 DNA-dependent RNA polymerase (T7-RNAPol) is provided by a helper-virus (Fowlpox-T7).

In order to rescue virus, the 4 plasmids are co-transfected into Fowlpox-T7 infected cells (Fig. 1). Three to five days after transfection, the supernatant is inoculated into specific-pathogen-free embryonated chicken eggs (ECE) and incubated for 3 days. Infectious virus that is produced by transfected cells will replicate in the ECE and progeny virus can be harvested from the allantois fluid.

In order to develop a reverse genetics system for NDV the following steps were followed:
- Generation of sub-genomic NDV and foreign gene cDNA's by RT-PCR
- Assembly of full-length cDNA in a transcription vector
- Cloning of each of the NP, P and L genes into an expression vector
- Verify nucleotide sequence of full-length cDNA and helper-plasmids
- Repair nucleotide differences resulting from the cloning procedure, if necessary
- Rescue of infectious virus from cDNA using co-transfection (Fig. 1)

### 2.2 Construction of full-length NDV-Mut HN(F277L)/M(G165W) cDNA, NDV-Mut HN(F277L)/M(G165W)/ F(F117S)/F(F190L)/ L(V757I)/ L(F1551S)/ L(R1700L) and helper plasmids

NDV-Mut HN(F277L)/M(G165W) and NDV-Mut HN(F277L)/M(G165W)/F(F117S)/ F(F190L)/L(V757I)/L(F1551S)/L(R1700L) (passage 28 HeLa cells) were used for the isolation of vRNA using standard procedures. The vRNA was used to generate first-strand cDNA by means of Reverse Transcriptase followed by PCR to generate 4 sub-genomic cDNA fragments (designated C1, C2, C3 and C8). The full-length cDNA of NDV-MutHu was assembled from these fragments and cloned in the transcription vector pOLTV5 (Peeters et al., 1999, J. Virol. 73:5001-5009) by a combination of In-Fusion® cloning and classical cloning using restriction enzymes. An overview of the procedure is shown in Fig. 2, and further details can be found in Appendix 1 of this reference. The NP, P and L-genes of NDV-MutHu were obtained by RT-PCR (Appendix 1) and cloned in the expression plasmid pCVI which was derived by deletion of a ClaI restriction fragment from pCI-neo (Promega).

### 2.3 Nucleotide sequence analysis

Nucleotide sequence analysis was used to verify that the sequence of pFL-NDV Mut HN(F277L)/M(G165W) and pFL-NDV Mut HN(F277L)/M(G165W)/F(F117S)/ F(F190L)/L(V757I)/L(F1551S)/L(R1700L) were correct. A few nucleotides which differed from the Reference sequence were repaired. Silent mutations (i.e., not leading to an amino acid change) may be left unchanged.

### 2.4 Rescue of infectious virus from pFL-NDV Mut HN(F277L)/M(G165W)

In order to generate infectious virus, we used the co-transfection system described above (and illustrated in Fig. 1) to transfect QM5 cells (derived from Quail) using plasmid pFL-NDV_Mut HN(F277L)/M(G165W) and the helper plasmids pCVI-NP^{Mut HN(F277L)/M(G165W)}, pCVI-P^{Mut HN(F277L)/M(G165W)} and pCVI-L^{Mut HN(F277L)/M(G165W)}. Rescue of infectious virus was successful as demonstrated by the presence of infectious virus in the allantoic fluid of ECE that were inoculated with the transfection supernatant (data not shown). The identity of the rescued virus was determined by RT-PCR followed by sequencing. The rescued virus was designated rgMut HN(F277L)/ M(G165W). NDV-Mut HN(F277L)/M(G165W) and rgMut HN(F277L)/M(G165W) have similar growth kinetics in HeLa cells.

### 2.5 Rescue of infectious virus from pFL-NDV Mut HN(F277L)/M(G165W)/ F(F117S)/F(F190L)/L(V757I)/L(F1551S)/L(R1700L)

In order to generate infectious virus, we used the co-transfection system described above (and illustrated in Fig. 1) to transfect QM5 cells (derived from Quail) using plasmid plasmid pFL-NDV_Mut HN(F277L)/M(G165W)/F(F117S)/F(F190L)/ L(V757I)/ L(F1551S)/ L(R1700L) and the helper plasmids pCVI-NP^{Mut HN(F277L)/M(G165W)/F(F117S)/F(F190L)/L(V757I)/L(F1551S)/L(R1700L)}, pCVI-P^{Mut HN(F277L)/M(G165W)/ F(F117S)/F(F190L)/L(V757I)/L(F1551S)/L(R1700L)} and pCVI-L^{Mut HN(F277L)/M(G165W)/F(F117S)/F(F190L)/L(V757I)/ L(FI55IS)/L(R1700L)}. Rescue of infectious virus was successful as demonstrated by the presence of infectious virus in the allantoic fluid of ECE that were inoculated with the transfection supernatant (data not shown). The identity of the rescued virus was determined by RT-PCR followed by sequencing. The rescued virus was designated rgMut HN(F277L)/M(G165W)/F(F117S)/F(F190L)/L(V757I)/L(F1551S)/L(R1700L).

### Example 3. Identify whether one or both of the amino acid substitutions in Mut HN(F277L)/M(G165W) are responsible for the difference in growth kinetics between Mut HN(F277L)/M(G165W) and the parent strain MTH68.

### 3.1 Growth kinetics in HeLa cells

The rescued rg-viruses (Table 1) as well as the original Mut HN(F277L)/M(G165W) and MTH68 viruses were used to determine their growth-kinetics in HeLa cells. Briefly, 4x10⁶ HeLa cells were seeded in 25 cm² cell culture flasks and grown overnight. The cells were infected using a MOI of 0.01 (i.e., 1 infectious virus particle per 100 cells), and at 8, 24 and 48 hours after infection the virus titer in the supernatant was determined by end-point titration on QM5 cells.

The data (Fig. 3) indicate that strains Mut HN(F277L)/M(G165W) and rgMut HN(F277L)/M(G165W) yield at least 10-fold higher virus titers than MTH68. Furthermore, the data indicate that the mutation at amino acid position 277 in the HN gene is responsible for this effect. The M mutation does not seem to have an effect. This can be best seen when looking at the virus titers 24h after infection, or even better when comparing the increase in virus titer between 8h and 24h (the exponential growth phase). The virus titer shows an increase of 3.5 (log10) for Mut HN(F277L)/M(G165W), rgMut HN(F277L)/M(G165W) and rgMut HN(F277L)/M(W165G), whereas this is 2.5 for MTH68, 2.7 for rgMut HN(L277F) and 3.0 for rgMTH68 (Table 3).

rgMut HN(F277L)/M(W165G) is a strain in which the mutation in the M gene has been restored in accordance with the NDV MTH-68/H.

**Table 1. Virus titers (log10 TCID50/ml)**

| | Time after infection (h) | | | |
|---|---|---|---|---|
| Virus | 0 h | 8 h | 24 h | 48 h |
| MTH68 | 4.8 | 4.5 | 7.0 | 7.0 |
| Mut HN(F277L)/M(G165W) | 5.0 | 4.3 | 7.8 | 8.3 |
| rgMTH68 | 5.0 | 4.0 | 7.0 | 7.5 |
| rgMut HN(L277F) | 5.0 | 4.3 | 7.0 | 7.3 |
| rgMut M(W165G) | 4.8 | 4.3 | 7.8 | 7.5 |
| rgMutHu | 5.3 | 4.5 | 8.0 | 8.0 |

### Example 4. Generation of recombinant NDV-strains with enhanced oncolytic and immune stimulating properties due to the expression of different therapeutic proteins.

The recombinant NDV-strains expressing one of the foreign genes of the present invention can be generated by means of the previously established reverse genetics system described above. The respective gene is to be inserted into the full-length cDNA of e.g. NDV-Mut HN(F277L)/M(G165W)/F(F117S)/ F(F190L)/L(V757I)/ L(F1551S)/L(R1700L) between the P and the M genes. To this end the open reading frames of the foreign genes may be fused via a 2A sequence. The genes were provided with the necessary NDV gene-start and gene-end sequences in order to allow transcription by the vRNA polymerase. Figure 2 shows the final constellation of the recombinant viruses.

Infectious virus can be rescued, and virus stocks can be prepared by two passages in HeLa cells. Expression of the respective foreign gene can be determined and quantified by using a total human IgG ELISA (Invitrogen).

### References:

- Blach-Olszewska et al., (1977) Why HeLa cells do not produce interferon? Arch Immunol Ther Exp (Warsz) 25:683-91.
- Chng et al., (2015) Cleavage efficient 2A peptides for high level monoclonal antibody expression in CHO cells, mAbs, 7:2, 403-412; http://dx.doi.org/10.1080/19420862.2015.1008351.
- Enoch et al., (1986) Activation of the Human beta-Interferon Gene Requires an Interferon-Inducible Factor, Mol. Cell. Biol. 6:801-10.
- Haryadi et al., (2015) Optimization of Heavy Chain and Light Chain Signal Peptides for High Level Expression of Therapeutic Antibodies in CHO Cells. PLoS ONE 10(2): e0116878. doi:10.1371/journal.pone.0116878.
- Schirrmacher, (2015) Oncolytic Newcastle disease virus as a prospective anti-cancer therapy. A biologic agent with potential to break therapy resistance. Expert Opin. Biol. Ther. 15:17 57-71
- Zamarin et al., (2014) Localized oncolytic virotherapy overcomes systemic tumor resistance to immune checkpoint blockade immunotherapy. Sci. Transl. Med. 6(226).
- Zamarin & Palese, (2017) Oncolytic Newcastle Disease Virus for cancer therapy: old challenges and new directions. Future Microbiol. 7: 347-67.

### Appendix 1: primers used for the generation of cDNA fragments and helper-plasmids

**cDNA fragments**

| Fragment | Size | Primer | Sequence (5'-3') |
|---|---|---|---|
| C1 | 3.6 kb | Noss-09 (SEQ ID No. 13) | ACGACTCACTATAGGaccaaacagagaatccgtgag |
| | | Noss-121 (SEQ ID No. 14) | CCGGGAAGATCCAGGgcactcttcttgcatgttac |
| C2 | 3.7 kb | Noss-122 (SEQ ID No. 15) | GGGCCTGCCTCACTAtggtggtaacatgcaagaag |
| | | Noss-123 (SEQ ID No. 16) | TGCATGTTACCACCAatgtgtcattgtatcgcttg |
| C3 | 5.7 kb | Noss-125 (SEQ ID No. 17) | CAAGAAGGGAGATACgtaatatacaagcgatacaatg |
| | | N oss-126 (SEQ ID No. 18) | TCGCTTG TAT ATT ACttgttgtagcaaagagcacc |
| C8 | 2.0 kb | Noss-133 (SEQ ID No. 19) | GGCCTGGATCTTCCCattatgctgtctgtatacggtgc |
| | | Noss-10 (SEQ ID No. 20) | ATGCCATGCCGACCCaccaaacaaagacttggtgaatg |
| iPCR pOLTV5 (StuI) | 2.5 kb | Noss-17 (SEQ ID No. 21) | CCTATAGTGAGTCGTATTAATTTC |
| | | Noss-128 (SEQ ID No. 22) | CCTGGATCTTCCCGGGTCGG |
| iPCR pOLTV5 (SmaI) | 2.5 kb | Noss-137 (SEQ ID No. 23) | GGGTCGGCATGGCATCTCCACC |
| | | Noss-138 (SEQ ID No. 24) | GGGAAGATCCAGGCCTATAGTG |

**Helper-plasmids (generated by In-Fusion® cloning in pCVI)**

| Gene | primer | sequence |
|---|---|---|
| NP | Noss-22 (SEQ ID No. 25) | CTCTAGAGTCGACCCttctgccaacatgtcttccg |
| | Noss-23 (SEQ ID No. 26) | GGGAAGCGGCCGCCCgtcggtcagtatccccagtc |
| P | Noss-24 (SEQ ID No. 27) | CTCTAGAGTCGACCCcagagtgaagatggccaccttc |
| | Noss-25n (SEQ ID No. 28) | GGGAAGCGGCCGCCCgtagtagtgatcagccattc |
| L | Noss-26 (SEQ ID No. 29) | CTCTAGAGTCGACCCgggtaggacatggcgggctc |
| | Noss-27 (SEQ ID No. 30) | GGGAAGCGGCCGCCCtgcctttaagagtcacagttac |

## Claims

1. A recombinant Newcastle Disease Virus (NDV), wherein the recombinant NDV carries as a foreign gene at least one gene selected from the group consisting of
- a gene encoding an antibody directed to protein PD-1 or an antigen-binding part directed to protein PD-1 (anti-PD-1), preferably a gene encoding Nivolumab, an antigen-binding part of Nivolumab, a variant of Nivolumab or a variant of an antigen-binding part of Nivolumab,
- a gene encoding an antibody directed to the surface protein CTLA-4 or an antigen-binding part directed to protein CTLA-4 (anti-CTLA-4), preferably a gene encoding Ipilimumab, an antigen-binding part of Ipilimumab, a variant of Ipilimumab or a variant of an antigen-binding part of Ipilimumab,
- a gene encoding a protein which improves the cellular immune response and the ability of T cells to enter tumor cells, or a part thereof which improves the cellular immune response and the ability of T cells to enter tumor cells, preferably a gene encoding interleukin-12 (IL-12), a part of interleukin-12, a variant of interleukin-12 or a variant of a part of interleukin-12,
- a gene encoding a protein with the ability to modulate the virus replication cycle, or a part thereof with the ability to modulate the virus replication cycle, preferably a gene encoding the non-structural protein NS1 of influenza A virus, a part of the non-structural protein NS1 of influenza A virus, a variant of the non-structural protein NS1 of influenza A virus or a variant of a part of the non-structural protein NS1 of influenza A virus,
- a gene encoding a protein with the ability to selectively induce apoptosis in human tumor cells, but not in normal human cells, or a part thereof with the ability to selectively induce apoptosis in human tumor cells, but not in normal human cells, preferably a gene encoding Apoptin, a part of Apoptin, a variant of Apoptin or a variant of a part of Apoptin,
- a gene encoding a protein that reduces or inhibits IFN expression such as an IFN-beta receptor, or a part thereof that reduces or inhibits IFN expression such as an IFN-beta receptor, preferably a gene encoding the viral protein B18R from vaccinia virus, a part of the viral protein B18R from vaccinia virus, a variant of the viral protein B18R from vaccinia virus or a variant of a part of the viral protein B18R from vaccinia virus, and
- and any combination of these genes, parts or variants, wherein
said recombinant NDV comprises a mutated HN gene and the encoded hemagglutinin-neuramidase with an amino acid substitution at position 277 to an amino acid with a hydrophobic side chain other than phenylalanine, preferably where phenylalanine (F) is substituted to leucine (L) at position 277 of the HN gene, and
said recombinant NDV comprises a mutated F gene and the encoded fusion protein with an amino acid substitution at position 117 to an amino acid with a hydroxylated amino acid side chain, preferably where phenylalanine (F) is substituted to serine (S) at position 117 of the F protein, and/or with an amino acid substitution at position 190 to an amino acid with an aliphatic amino acid side chain, preferably where phenylalanine (F) is substituted to leucine (L) at position 190 of the F protein.

2. The recombinant NDV according to claim 1, wherein the NDV further comprises a mutation in the M gene and the thus encoded matrix protein, preferably wherein the mutated M gene encodes a matrix protein with an amino acid substitution at position 165 to an amino acid with an aromatic side chain, particulary where glycine (G) is substituted to tryptophane (W) at position 165 of the M protein.

3. The recombinant NDV according to claim 1 or claim 2, wherein the NDV comprises a mutated L gene and the encoded L protein with an amino acid substitution at position 757 to an amino acid with an aliphatic amino acid side chain other than valine (V), preferably where valine (V) is substituted to isoleucine (I) at position 757 of the L protein, and/or with an amino acid substitution at position 1551 to an amino acid with a hydroxylated side chain, preferably where phenylalanine (F) is substituted to serine (S) at position 1551 of the L protein, and/or with an amino acid substitution at position 1700 to an amino acid with an aliphatic side chain, preferably where arginine (R) is substituted to leucine (L) at position 1700 of the L protein, preferably wherein the NDV comprises a mutated L gene and the encoded L protein with the amino acid substitution at position 757, position 1551 and position 1700.

4. The recombinant NDV according to any one of claims 1 to 3, wherein the recombinant NDV carries as a foreign gene a gene encoding Nivolumab, an antigen-binding part of Nivolumab, a variant of Nivolumab or a variant of a antigen-binding part of Nivolumab, or a gene encoding Ipilimumab, an antigen-binding part of Ipilimumab, a variant of Ipilimumab or a variant of a antigen-binding part of Ipilimumab.

5. The recombinant NDV according to any one of claims 1 to 4, wherein the recombinant NDV is encoded by and/or comprises at least one of the nucleic acids according to SEQ ID No. 2 to 8 or variants thereof, preferably the variants having a sequence identity of at least 75 %, particularly of at least 90 %, more particularly of at least 95 % to the respective sequence.

6. The recombinant NDV according to any one of claims 1 to 5, wherein the NDV comprises a mutated F gene and the encoded fusion protein with a amino acid substitution at position 289 to an amino acid with a aliphatic side chain other than leucine (L), preferably where leucine (L) is substituted to alanine (A) at position 289 of the F protein, and/or wherein the NDV comprises a mutated L gene and the encoded large protein with a amino acid substitution at position 1717 to an amino acid with aromatic side chain other than tyrosine (Y), preferably where tyrosine (Y) is substituted to histidine (H) at position 1717 of the L protein, and/or at position 1910 to an amino acid with a basic side chain, preferably where glutamic acid (E) is substituted to lysine (K) at position 1910 of the L protein.

7. A recombinant NDV according to any one of claims 1 to 6 for use in medicine.

8. A recombinant NDV according to any one of claims 1 to 7 for use in a method of treating cancer in a subject, preferably in a mammal, more preferably a mammalian animal or a human subject, considered in need thereof, in particular for the treatment of one or more indications selected from the group consisting of brain tumors, like glioblastoma, bone tumors, like osteosarcoma and/or Ewing's sarcoma, soft tissue tumors, like rhabdomyosarcoma, gynecological tumors, like breast cancer, ovary cancer and/or cervix cancer, gastrointestinal tumors, like esophageal tumors, stomach tumors, colon tumors, pancreas tumors, prostate tumors, lung tumors, ear, nose, throat tumors, tongue tumors, and skin tumors, like melanoma.

9. A nucleic acid encoding a recombinant NDV, preferably a rgNDV, the nucleic acid comprising a transgenic construct, wherein said transgenic construct encodes a protein selected from the group consisting of
- an antibody directed to protein PD-1 or an antigen-binding part directed to protein PD-1 (anti-PD-1), preferably Nivolumab, an antigen-binding part of Nivolumab, a variant of Nivolumab or a variant of an antigen-binding part of Nivolumab,
- an antibody directed to the surface protein CTLA-4 or an antigen-binding part directed to protein CTLA-4 (anti-CTLA-4), preferably Ipilimumab, an antigen-binding part of Ipilimumab, a variant of Ipilimumab or a variant of an antigen-binding part of Ipilimumab,
- a protein which improves the cellular immune response and the ability of T cells to enter tumor cells, or a part thereof which improves the cellular immune response and the ability of T cells to enter tumor cells, preferably interleukin-12 (IL-12), a part of interleukin-12, a variant of interleukin-12 or a variant of a part of interleukin-12,
- a protein with the ability to modulate the virus replication cycle, or a part thereof with the ability to modulate the virus replication cycle, preferably the non-structural protein NS1 of influenza A virus, a part of the non-structural protein NS1 of influenza A virus, a variant of the non-structural protein NS1 of influenza A virus or a variant of a part of the non-structural protein NS1 of influenza A virus,
- a protein with the ability to selectively induce apoptosis in human tumor cells, but not in normal human cells, or a part thereof with the ability to selectively induce apoptosis in human tumor cells, but not in normal human cells, preferably Apoptin, a part of Apoptin, a variant of Apoptin or a variant of a part of Apoptin,
- a protein that reduces or inhibits IFN expression such as an IFN-beta receptor, or a part thereof that reduces or inhibits IFN expression such as an IFN-beta receptor, preferably the viral protein B18R from vaccinia virus, a part of the viral protein B18R from vaccinia virus, a variant of the viral protein B18R from vaccinia virus or a variant of a part of the viral protein B18R from vaccinia virus, and
- any combination of these genes, parts or variants, wherein
wherein the nucleic acid in addition comprises a mutation in the HN gene, the sequence of the mutated HN gene encoding hemagglutinin-neuramidase with an amino acid substitution at position 277 to an amino acid with a hydrophobic side chain other than phenylalanine, particulary where the mutated HN gene encodes HN^{F277L}, and
wherein the nucleic acid in addition comprises at least one mutation in the F gene, preferably wherein the encoded mutated F gene encodes a fusion protein with an amino acid substitution at position 117 to an amino acid with a hydroxylated side chain, preferably where the encoded mutated F gene encodes F^{F117S}, and/or with an amino acid substitution at position 190 to an amino acid with an aliphatic side chain, preferably where the mutated F gene encodes F^{F190L}.

10. The nucleic acid according to claim 9, wherein the sequence encoding the recombinant NDV further comprises a mutation in the M gene, preferably wherein the mutated M gene encodes a matrix protein with an amino acid substitution at position 165 to an amino acid with an aromatic side chain, particulary where the mutated M gene encodes M^{G165W}.

11. The nucleic acid according to claim 9 or claim 10, wherein the sequence encoding the recombinant NDV further comprises at least one mutation in the L gene, preferably wherein the encoded mutated L gene encodes a large protein with an amino acid substitution at position 757 to an amino acid with an aliphatic side chain, preferably where the mutated L gene encodes L^{V757I}, and/or with an amino acid amino acid substitution at position 1551 to an amino acid with an aliphatic side chain, preferably where the encoded mutated L gene encodes L^{F1551S}, and/or with an amino acid amino acid substitution at position 1700 to an amino acid with an aliphatic side chain, preferably where the encoded mutated L gene encodes L^{R1700L}, more preferably wherein the encoded mutated L gene encodes a large protein with the amino acid substitution at position 757, position 1551 and position 1700.

12. The nucleic acid according to any one of claims 9 to 11, wherein the transgenic construct encodes for Nivolumab, an antigen-binding part of Nivolumab, a variant of Nivolumab or a variant of an antigen-binding part of Nivolumab, or encodes for Ipilimumab, an antigen-binding part of Ipilimumab, a variant of Ipilimumab or a variant of an antigen-binding part of Ipilimumab.

13. The nucleic acid according to any one of claims 9 to 12, wherein the nucleic acid consists of or comprises at least one of the nucleic acids according to SEQ ID No. 2 to 8 or variants thereof, preferably the variants having a sequence identity of at least 75 %, particularly of at least 90 %, more of at least particularly 95 % to the respective sequence.

14. The nucleic acid according to any one of claims 9 to 13, wherein the sequence encoding the recombinant NDV comprises a mutation in the F gene, wherein the mutated F gene encodes a fusion protein with an amino acid substitution at position 289 to an amino acid with an aliphatic side chain other than leucine (L), preferably where the encoded mutated F gene encodes F^{L289A}, and/or wherein the sequence encoding the recombinant NDV comprises at least one mutation in the L gene, wherein the encoded mutated L gene encodes a large protein with a amino acid substitution at position 1717 to an amino acid with aromatic side chain other than tyrosine (Y), preferably where the mutated L gene encodes L^{Y1717H}, and/or wherein the encoded mutated L gene encodes a large protein with a amino acid substitution at position 1910 to an amino acid with a basic side chain, preferably where the mutated L gene encodes L^{E1910K}.

15. The nucleic acid according to any one of claims 9 to 14, wherein the nucleic acid is derived from a recombinant NDV according to any one of claims 1 to 8.

16. A pharmaceutical formulation comprising particles of the recombinant NDV according to any one of claims 1 to 6 and/or a nucleic acid according to any one of claims 9 to 15, preferably for use in oncological treatment of a subject considered in need thereof, in particular for the treatment of one or more indications selected from the group consisting of brain tumors, bone tumors, like osteosarcoma and/or Ewing's sarcoma, soft tissue tumors, like rhabdomyosarcoma, gynecological tumors, like breast cancer, ovary cancer and/or cervix cancer, gastrointestinal tumors, like esophageal tumors, stomach tumors, colon tumors, pancreas tumors, prostate tumors, lung tumors, ear, nose, throat tumors, tongue tumors, and skin tumors, like melanoma.
